# EUROPEAN PATENT APPLICATION

(11) **EP 4 588 931 A2**
(43) Date of publication of application: **23.07.2025**
(21) Application number: 24222850.0
(22) Date of filing: 23.12.2024
(51) Int. Cl.: C07H 21/00, C07H 21/02, A61K 31/7084, A61K 31/7088

(54) **COMPOUND, PHARMACEUTICAL COMPOSITION, KIT FOR CAPPED RNA TRANSCRIPT, AND METHOD FOR IN VITRO**

(30) Priority: 29.12.2023 US 202363615882 P; 03.12.2024 TW 113146768
(71) Applicant: Industrial Technology Research Institute, 310401 Hsinchu (TW)
(72) Inventor: Fu, Chih-Wei, Taoyuan City (TW); Liu, Hao-Hsuan, Taoyuan City (TW); Yang, Ching-Wen, Kaohsiung City (TW); Wu, Ming-Hsi, Taipei City (TW); Li, Hsiu-Chuan, Hsinchu City (TW); Wu, Ssu-Yuan, Hsinchu County (TW)
(74) Representative: Fuchs Patentanwälte Partnerschaft mbB

(57) **Abstract**

A compound, pharmaceutical composition, kit for capped RNA transcript, and method for *in vitro* transcription are provided. The compound has a structure represented by Formula (I), Formula (II), or Formula (III) 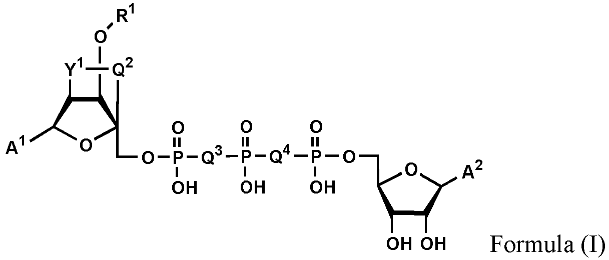 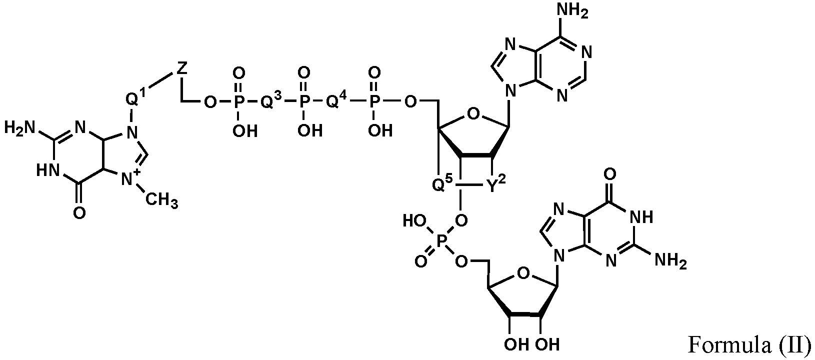 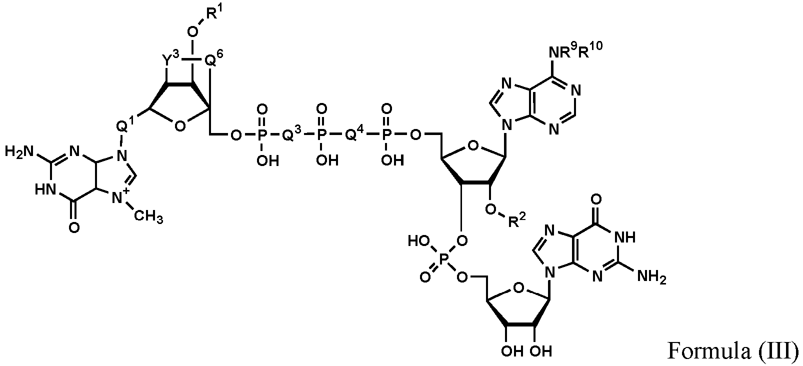 , wherein A¹ A², R¹, R², R⁹, R¹⁰, Q¹ Q², Q³, Q⁴, Q⁵, Q⁶, Y¹, Y², Y³, and Z are disclosed in the specification.

## Description

### TECHNICAL FIELD

The disclosure relates to a compound, pharmaceutical composition, kit for capped RNA transcript, and method for *in vitro.*

### BACKGROUND

The synthesis of messenger RNA (mRNA) through *in vitro* transcription has become a crucial tool for introducing exogenous genes and expressing genetic information. It is widely used in the treatment and prevention of diseases. Industrial-scale preparation of mRNA using a one-pot *in vitro* transcription (IVT) reaction has the advantages of simple process and low cost.

Capping analogs can enhance the translation efficiency of messenger RNA (mRNA) andare the critical key raw material in the development of one-pot *in vitro* transcription processes.

### SUMMARY

According to embodiments of the disclosure, the disclosure provides a compound such as a cap analog. The compound of the disclosure may have a structure represented by Formula (I), Formula (II), or Formula (III) 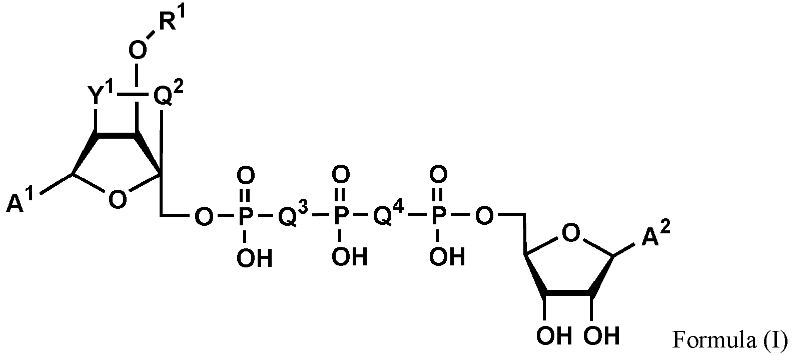 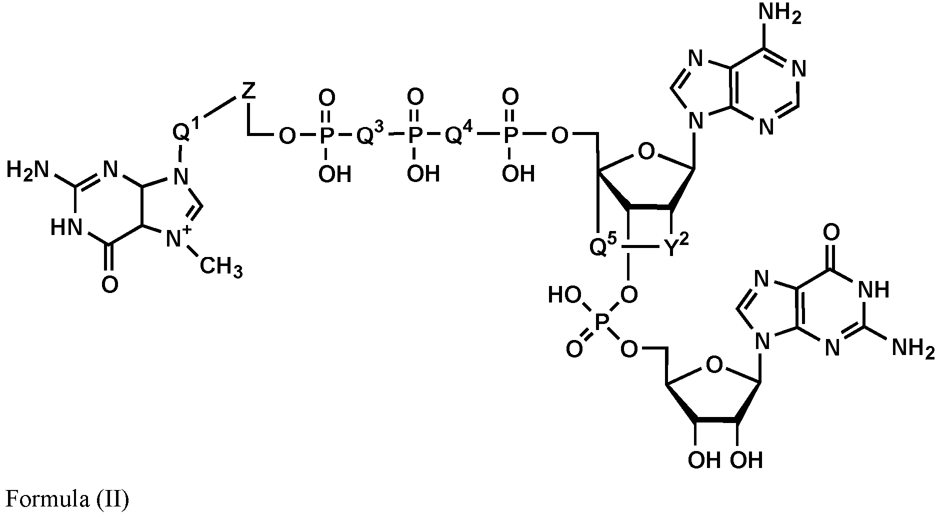 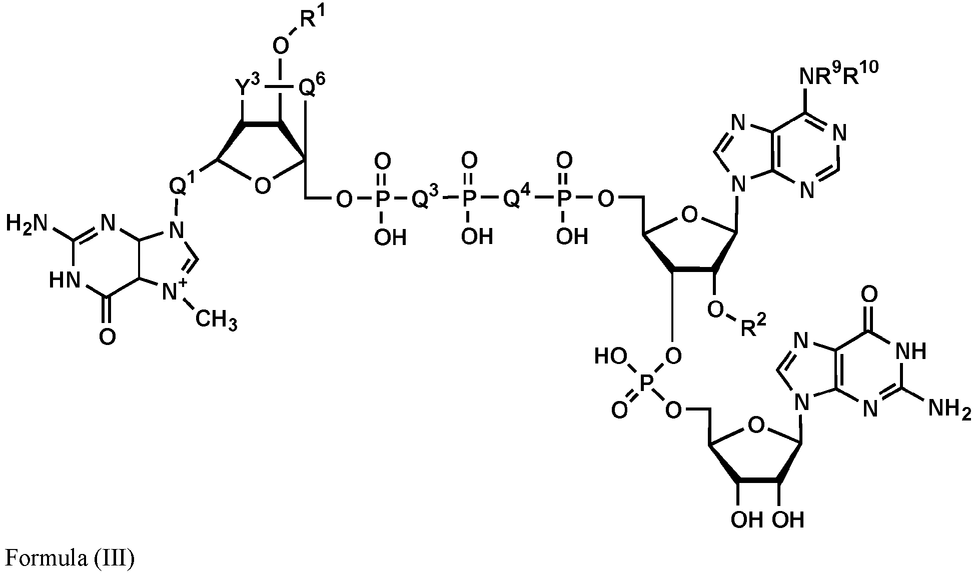 , wherein A¹ and A² are independently 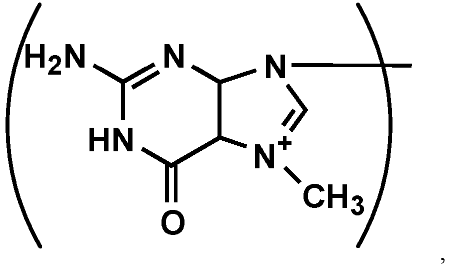 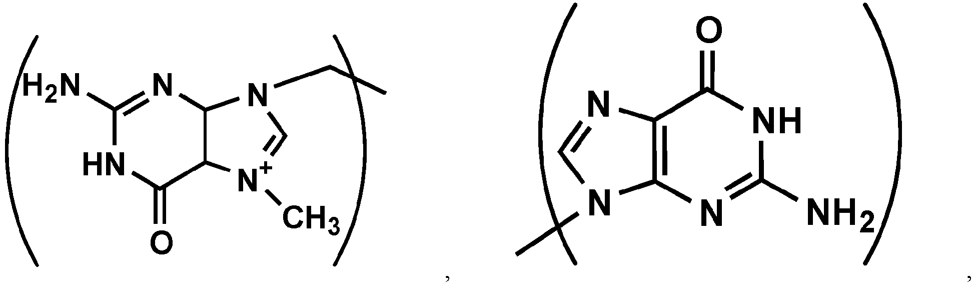 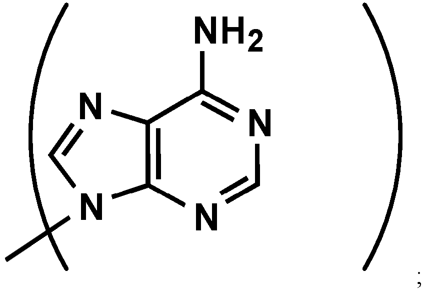 R¹ and R² may be hydrogen, methyl or phenyl group; Q¹ may be single bond or -CH₂-; Y¹ and Y³ may be independently -O-, 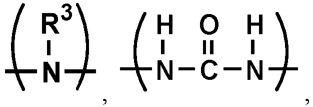 or 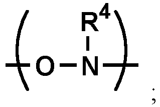 Z may be 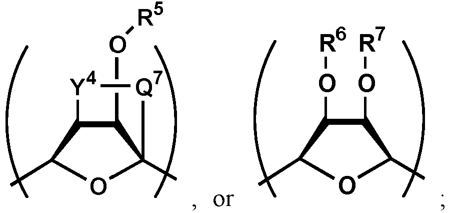 Y² and Y⁴ may be independently -O-, 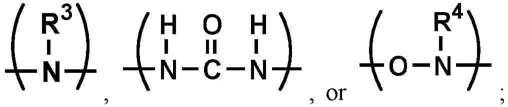 Q², Q⁵, Q⁶ and Q⁷ may be independently -CH₂-, or 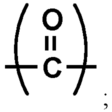 Q³ and Q⁴ may be independently -O-, -CH₂-, or -CCl₂-; R³ may be C1-C6 alkyl group, or 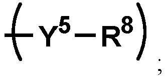 R⁴ may be hydrogen or methyl; R⁵, R⁶, and R⁷ may be independently hydrogen, methyl or phenyl group; Y⁵ may be 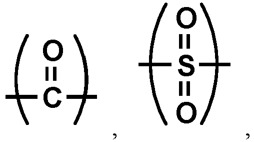 or 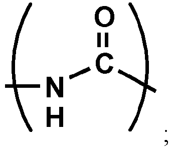 R⁸ may be C1-C6 alkyl group, C4-C8 cycloalkyl group, phenyl group, benzyl group or C3-C5 heterocyclic group; and R⁹ and R¹⁰ are independently hydrogen, C1-C6 alkyl group, or benzyl group.

According to embodiments of the disclosure, the disclosure also provides a pharmaceutical composition. The pharmaceutical composition can include a compound having the structure represented by Formula (I), Formula (II), or Formula (III); and an RNA molecule.

According to embodiments of the disclosure, the disclosure provides a kit for a capped RNA transcript. The kit for a capped RNA transcript includes a compound and an RNA polymerase. According to embodiments of the disclosure, the compound has a structure represented by Formula (I), Formula (II), or Formula (III).

According to embodiments of the disclosure, the disclosure also provides a method for *in vitro* transcription. The method for *in vitro* transcription includes the following steps. A composition is provided, wherein the composition includes an RNA polymerase, nucleoside triphosphate, and the compound of the disclosure; and a DNA template is contacted with the composition to transcribe the DNA template into RNA *in vitro.*

A detailed description is given in the following embodiments.

### DETAILED DESCRIPTION

The compound, pharmaceutical composition, kit for capped RNA transcript, and method for *in vitro* for forming the electrode material and method for preparing the same are described in detail in the following description. In the following detailed description, for purposes of explanation, numerous specific details and embodiments are set forth in order to provide a thorough understanding of the present disclosure. The specific elements and configurations described in the following detailed description are set forth in order to clearly describe the present disclosure. It will be apparent, however, that the exemplary embodiments set forth herein are used merely for the purpose of illustration, and the inventive concept may be embodied in various forms without being limited to those exemplary embodiments.

The disclosure provides a compound, a pharmaceutical composition, a kit for capping RNA transcripts and a method of *in vitro* transcription. According to embodiments of the disclosure, due to the specific structures of the disclosed compounds, they can serve as cap analogs, enabling the capping reaction of messenger RNA (mRNA) to be completed simultaneously during transcription. As a result, the disclosed compounds may be applied to one-pot *in vitro* transcription (IVT) processes, achieving the purposes of increasing yield, reducing process costs, enhancing product stability, and improving protein expression capability.

According to embodiments of the disclosure, the compound of the disclosure may have a structure represented by Formula (I), Formula (II), or Formula (III) 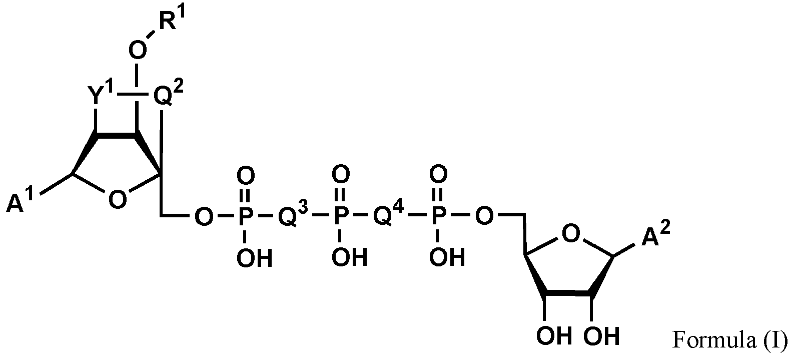 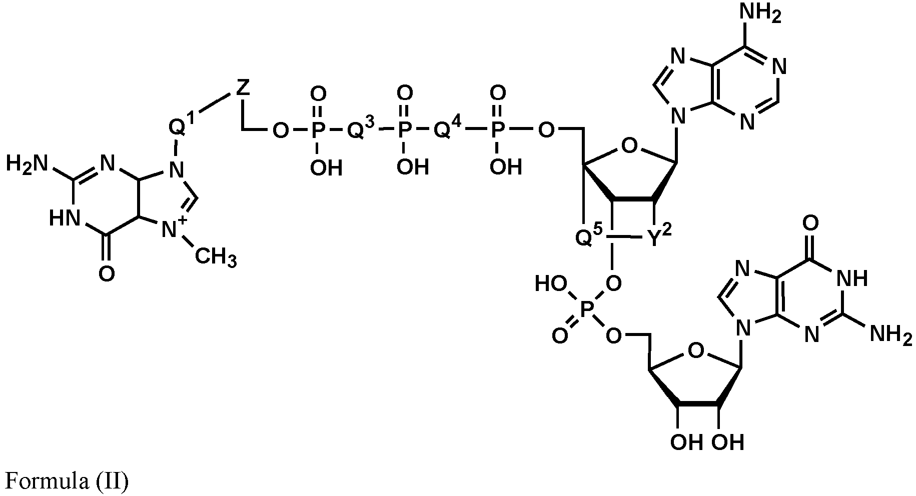 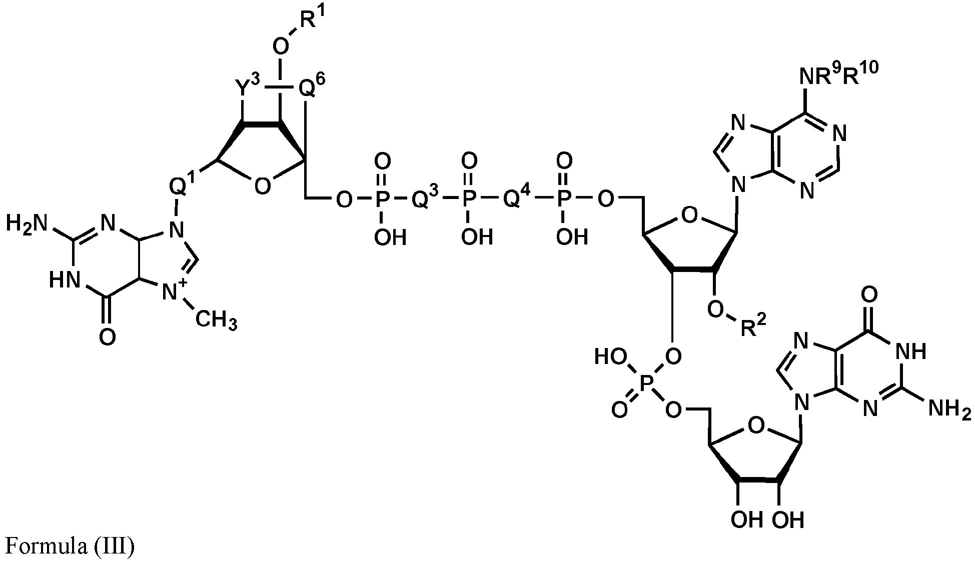 , wherein A¹ and A² are independently 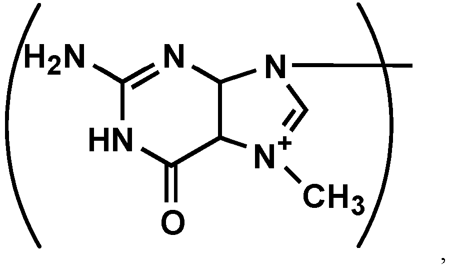 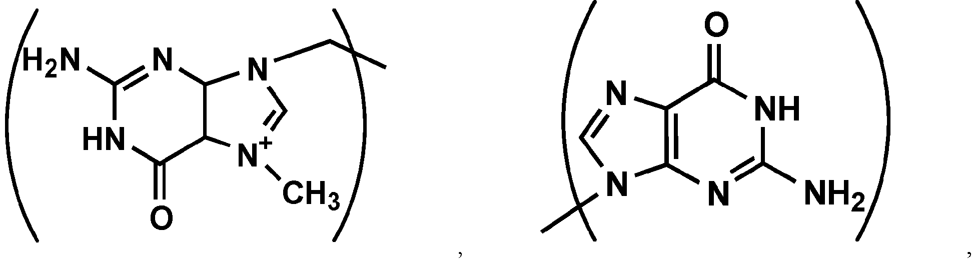 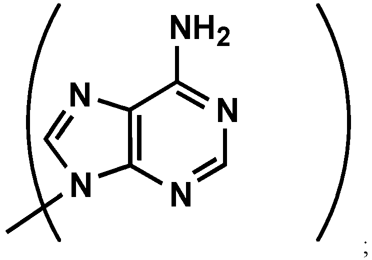 R¹ and R² may be hydrogen, methyl or phenyl group; Q¹ may be single bond or -CH₂-; Y¹ and Y³ may be independently -O-, 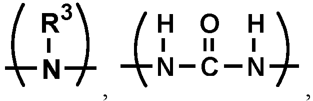 or 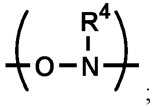 Z may be 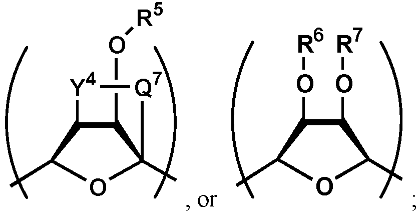 Y² and Y⁴ may be independently -O-, 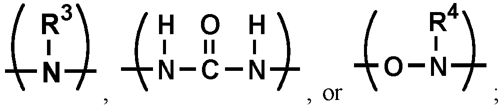 Q², Q⁵, Q⁶ and Q⁷ may be independently - CH₂-, or 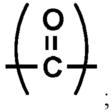 Q³ and Q⁴ may be independently -O-, -CH₂-, or -CCl₂-; R³ may be C1-C6 alkyl group, or 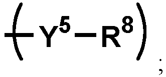 R⁴ may be hydrogen or methyl; R⁵, R⁶ and R⁷ may be independently hydrogen, methyl or phenyl group; Y⁵ may be 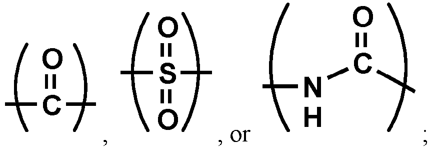 R⁸ may be C1-C6 alkyl group, C4-C8 cycloalkyl group, phenyl group, benzyl group or C3-C5 heterocyclic group; and R⁹ and R¹⁰ are independently hydrogen, C1-C6 alkyl group, or benzyl group.

According to embodiments of the disclosure, the term "single bond" may mean a case where no separate atom is present at the relevant site. For example, in the structures of Formula (I) to (III), when Q¹ is single bond, there is no separate atom at the site represented by Q².

According to embodiments of the disclosure, the alkyl group of the disclosure may be linear or branched alkyl group. According to embodiments of the disclosure, C1-C6 alkyl group of the disclosure may be methyl, ethyl, propyl, butyl, pentyl, hexyl, or an isomer thereof. For example, C1-C6 alkyl group may be methyl, ethyl, n-propyl, isopropyl, n-butyl, sec-butyl, iso-butyl, or tert-butyl.

According to embodiments of the disclosure, C4-C8 cycloalkyl group of the disclosure may be cyclopentyl, cyclohexyl, cycloheptyl, or cyclooctyl.

According to embodiments of the disclosure, C3-C5 heterocyclic group of the disclosure may be pyrrolyl, imidazolyl, pyrazolyl, pyridinyl, pyrimidinyl, tetrahydrofuranyl, or piperidinyl.

According to embodiments of the disclosure, the compound of the disclosure having a structure represented by Formula (I). According to embodiments of the disclosure, when Q² is -CH₂-, Y¹ may be 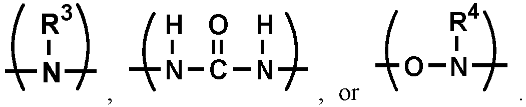 In addition, according to some embodiments of the disclosure, when Q² is 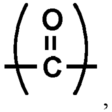 Y¹ may be

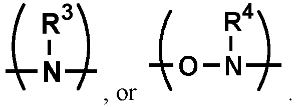

According to embodiments of the disclosure, the compound of the disclosure having a structure represented by Formula (II). According to embodiments of the disclosure, when Q⁵ is -CH₂-, Y² may be -O-, 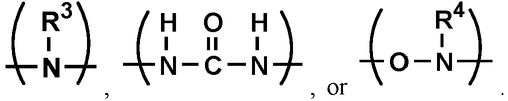 In addition, according to some embodiments of the disclosure, when Q⁵ is 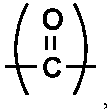 Y² may be

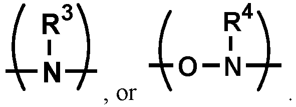

According to embodiments of the disclosure, the compound of the disclosure has a structure represented by Formula (II). According to embodiments of the disclosure, when Y² is -O-, Z is 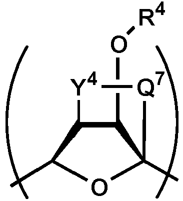 and Y⁴ is

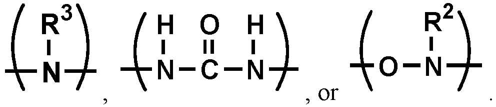

According to embodiments of the disclosure, the compound of the disclosure has a structure represented by Formula (II), and Z may be 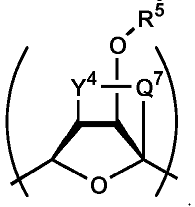 According to embodiments of the disclosure, when Q⁷ is -CH₂-, Y⁴ may be -O-, 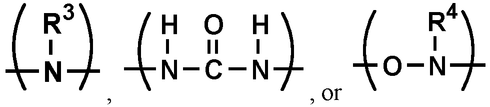 . According to some embodiments of the disclosure, when Q⁷ is 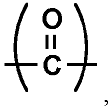 Y⁴ may be 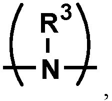 or

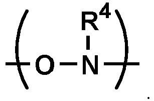

According to embodiments of the disclosure, the compound of the disclosure having a structure represented by Formula (III). According to embodiments of the disclosure, when Q⁶ is - CH₂-, Y³ may be 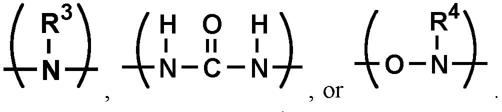 In addition, According to some embodiments of the disclosure, when Q⁶ is 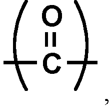 Y³ may be

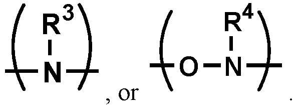

According to embodiments of the disclosure, the compound may be 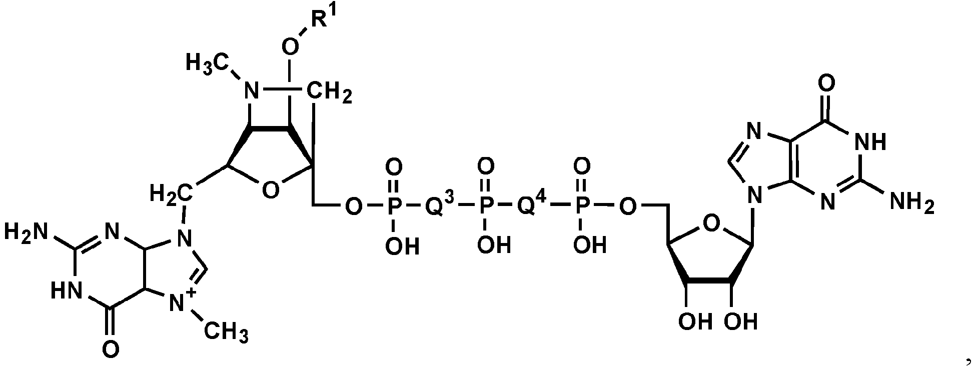 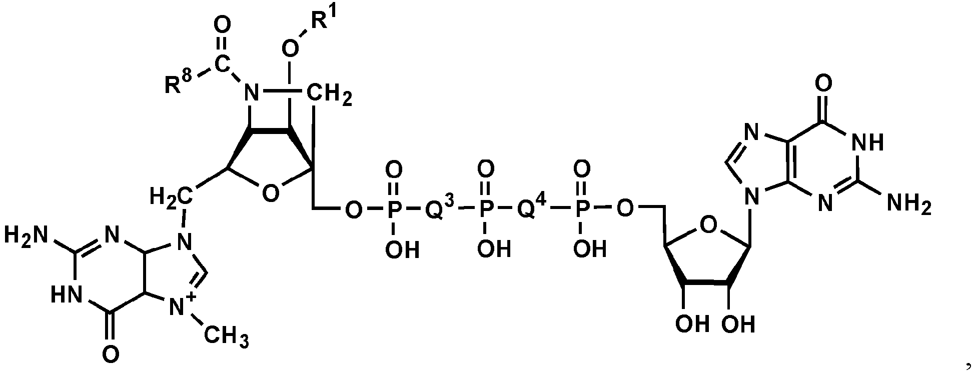 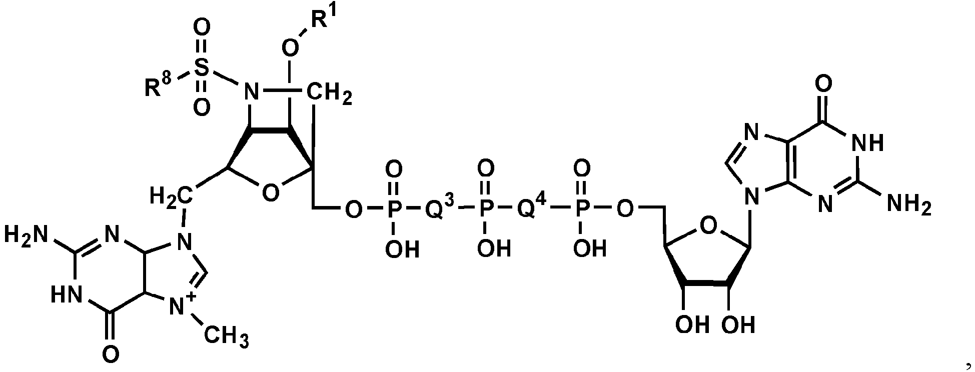 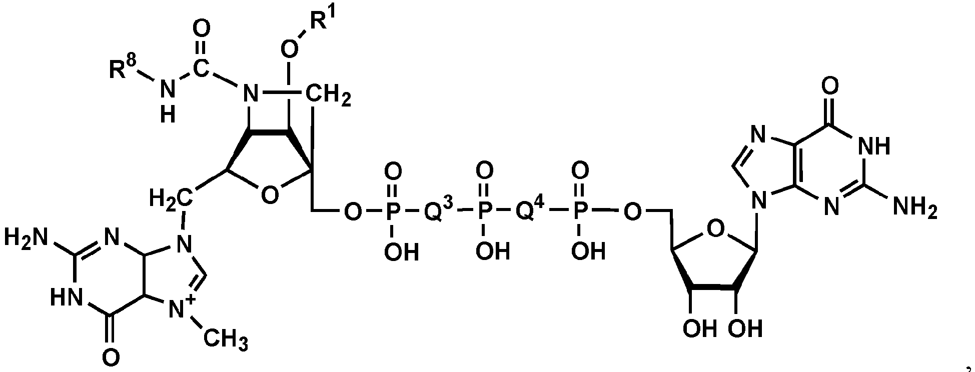 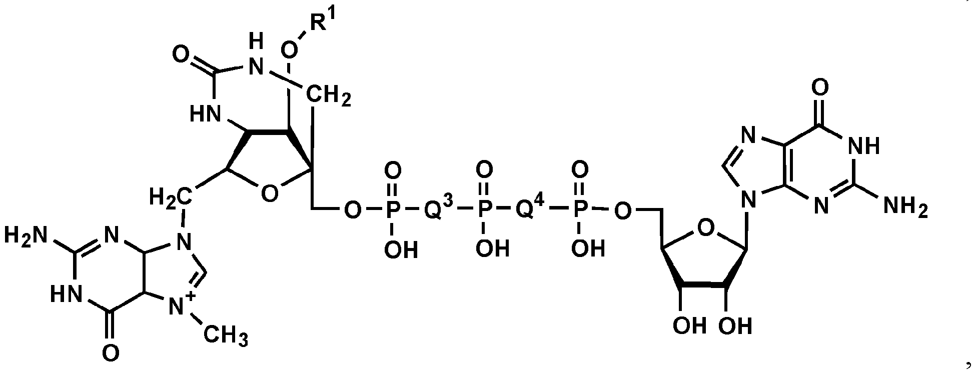 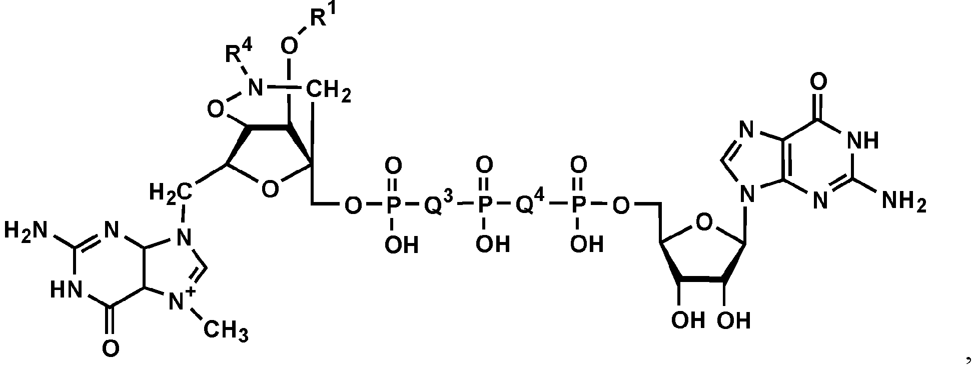 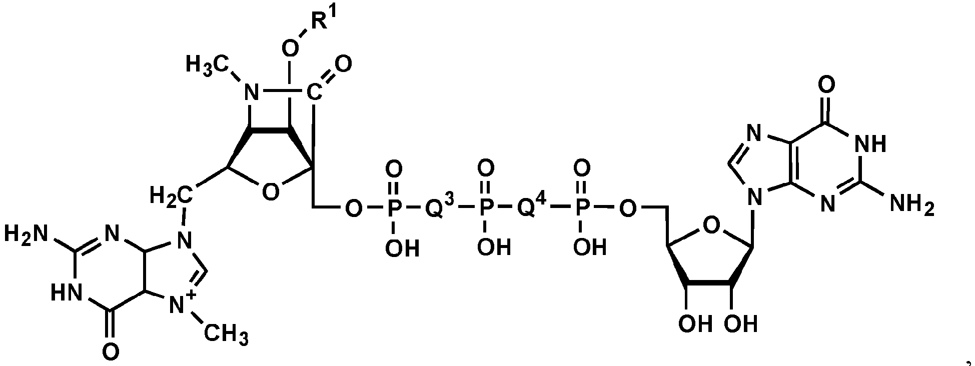 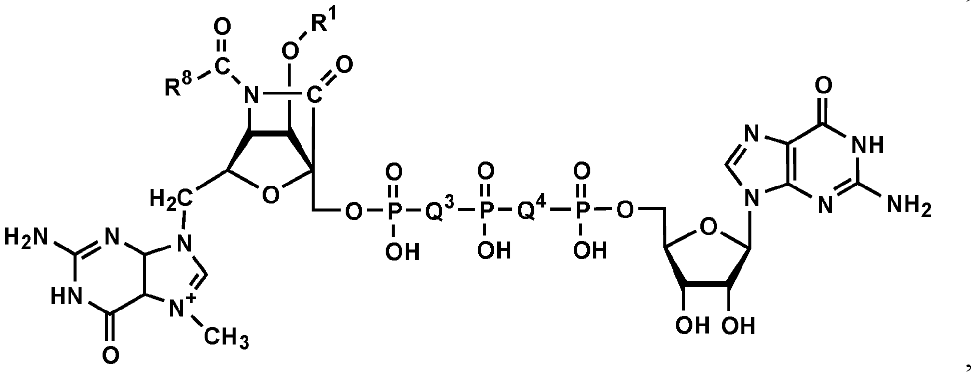 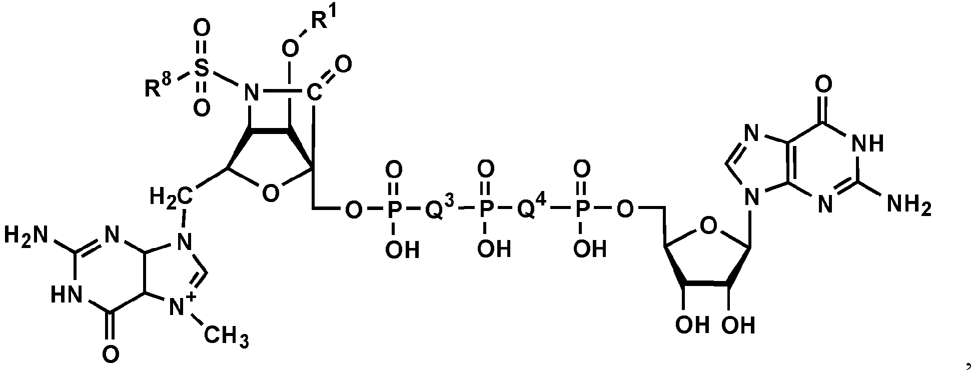 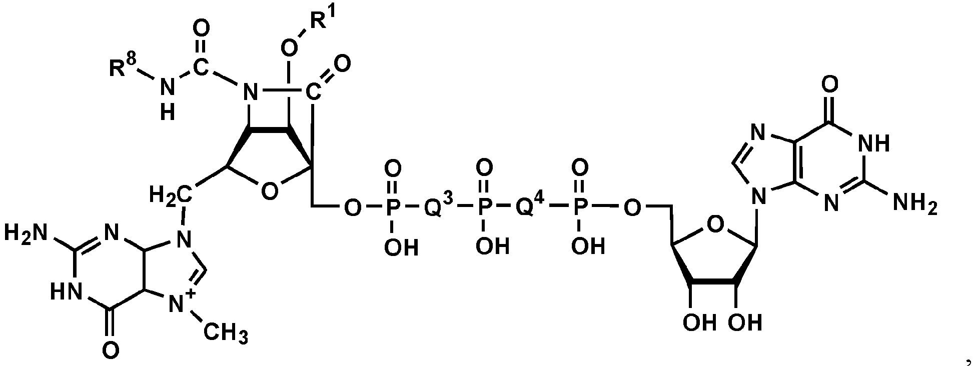 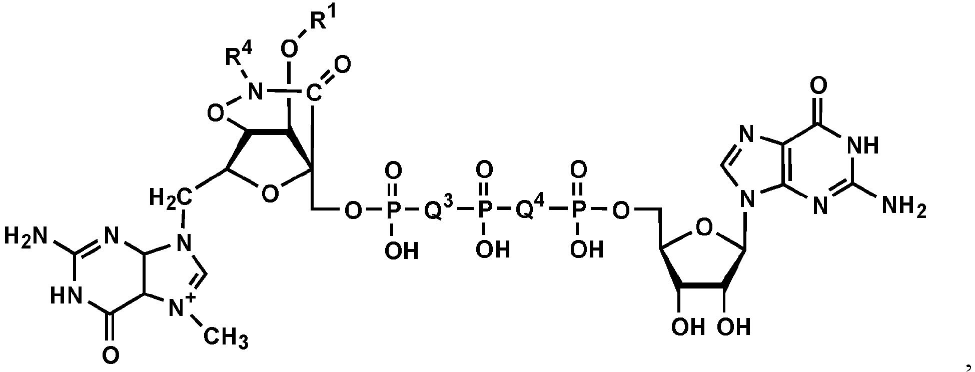 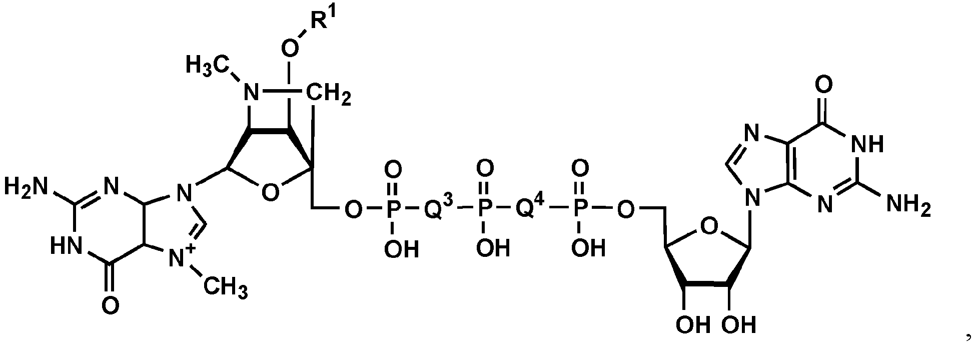 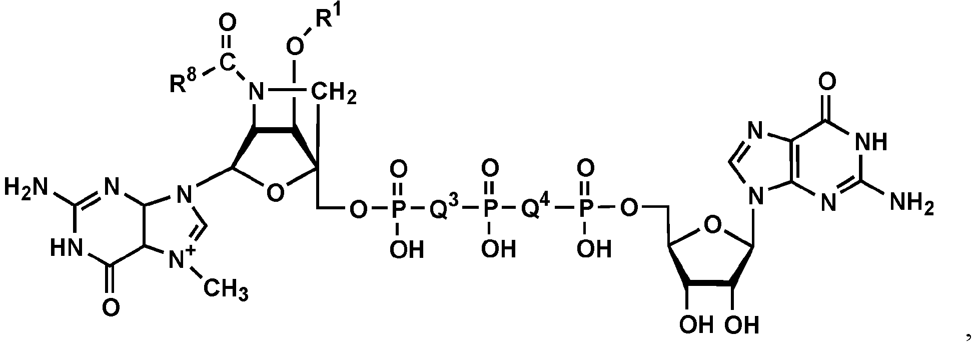 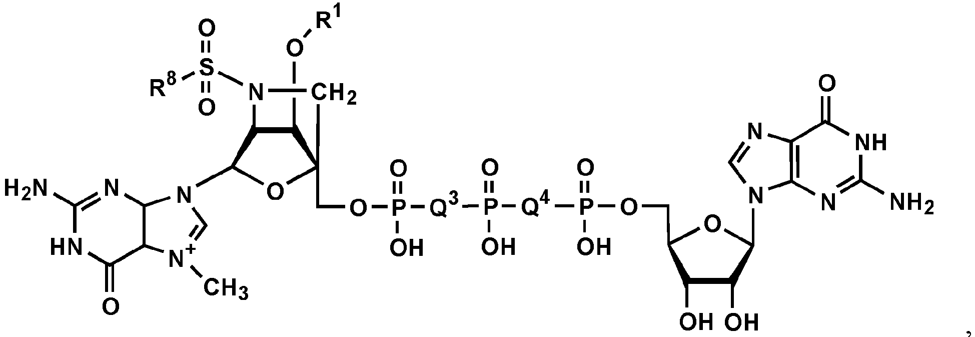 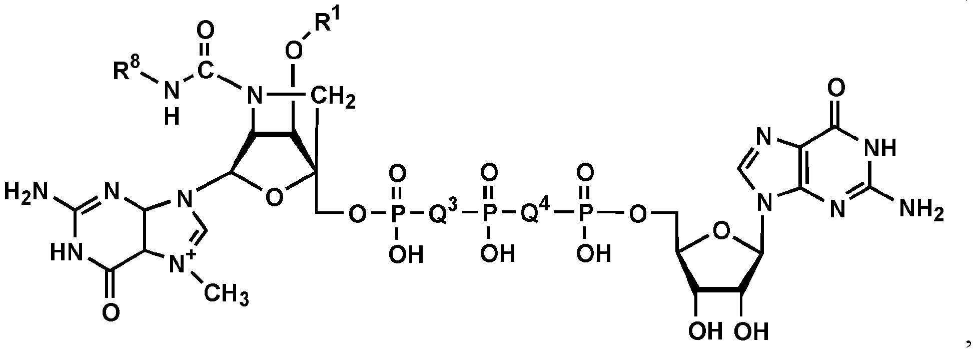 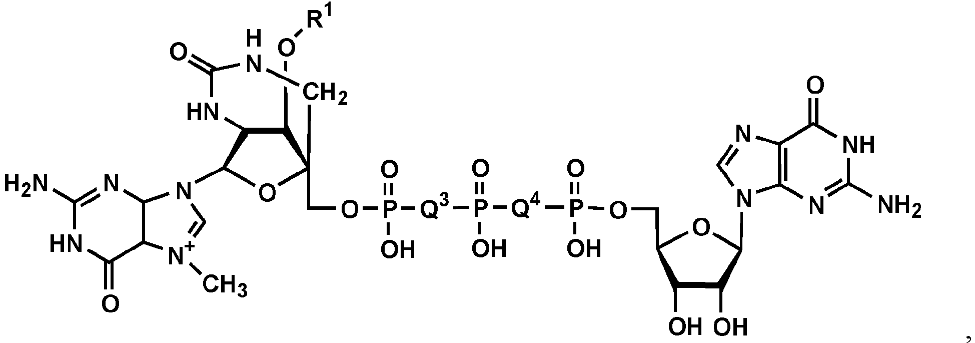 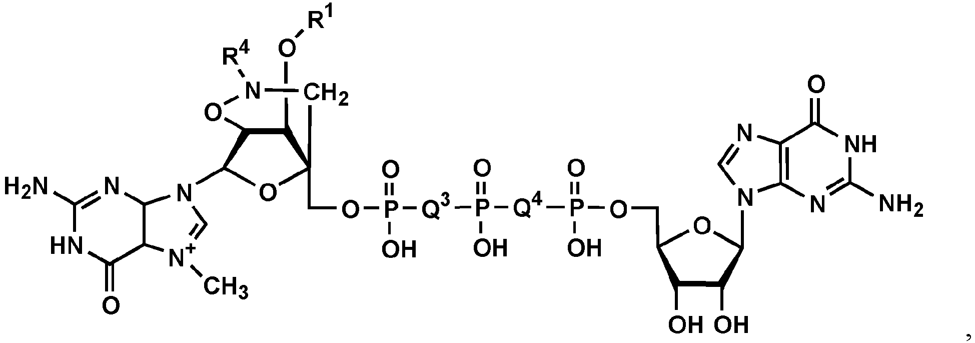 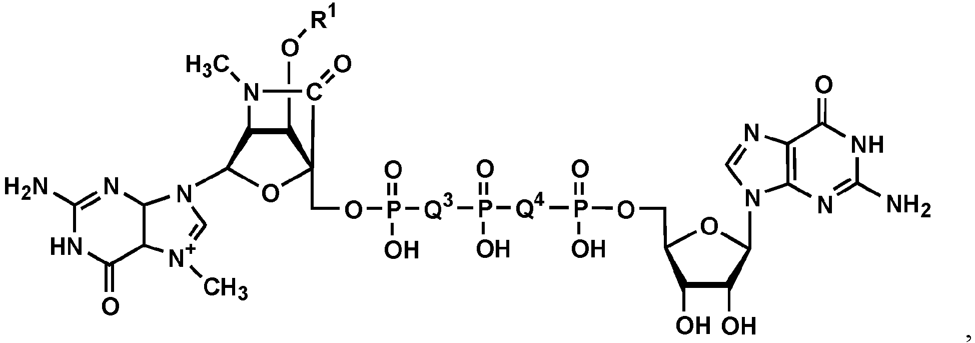 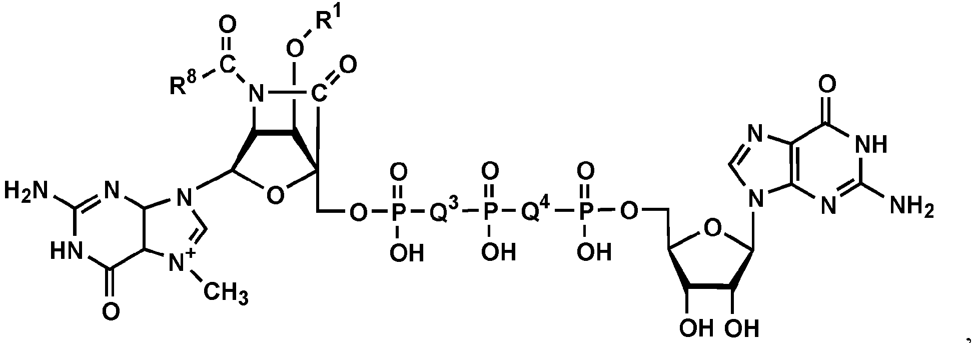 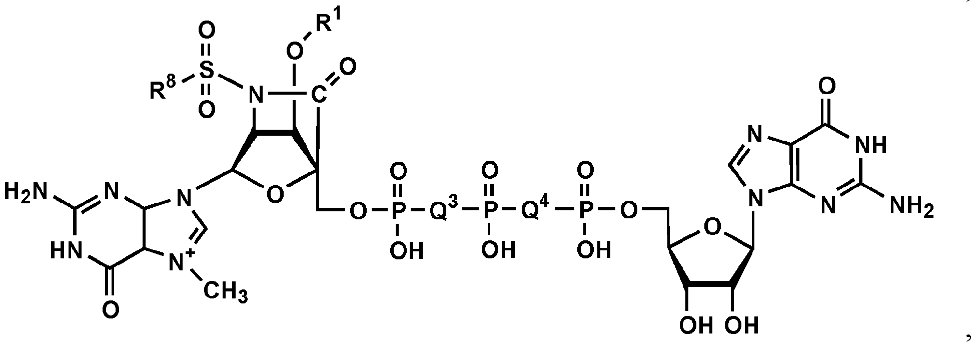 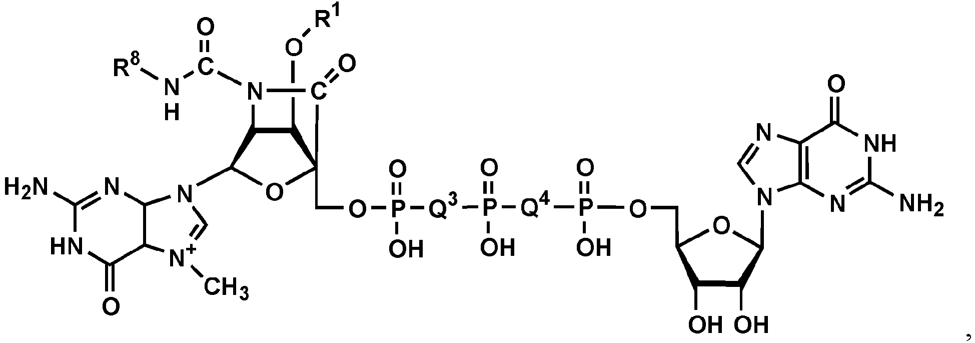 or 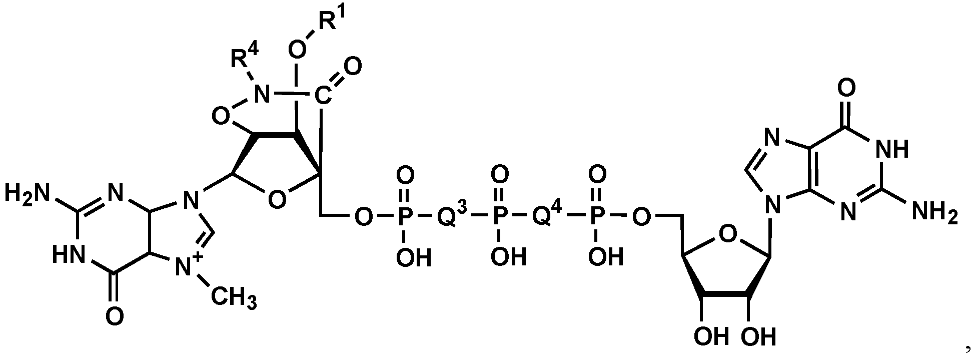 wherein R¹ may be hydrogen, methyl or phenyl group; Q³ and Q⁴ may be independently -O-, -CH₂-, or -CCl₂-; R⁴ may be hydrogen or methyl; and R⁸ may be C1-C6 alkyl group, C4-C8 cycloalkyl group, phenyl group, benzyl group or C3-C5 heterocyclic group.

According to embodiments of the disclosure, the compound may be 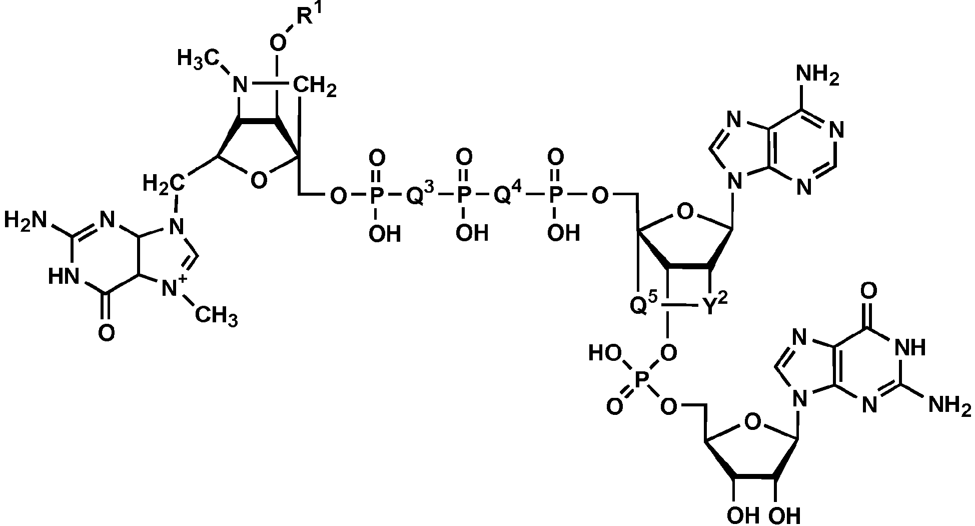 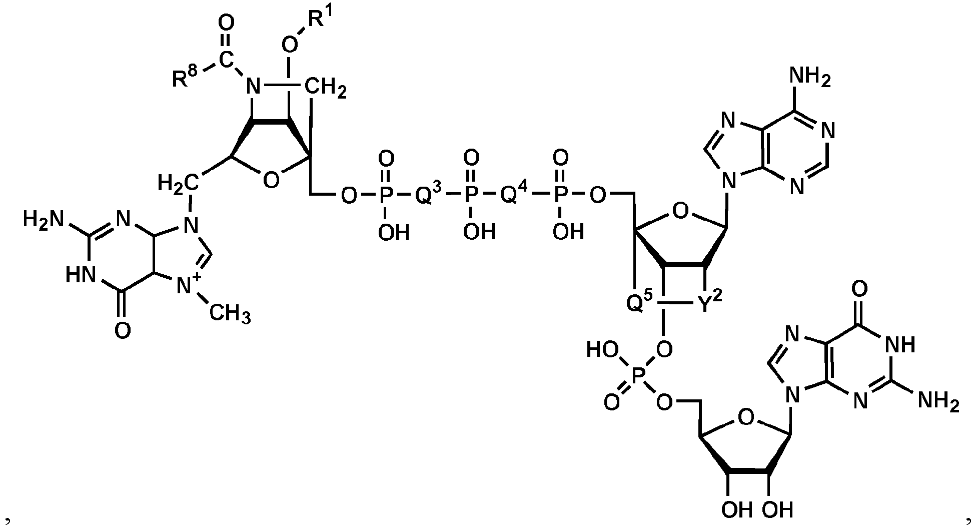 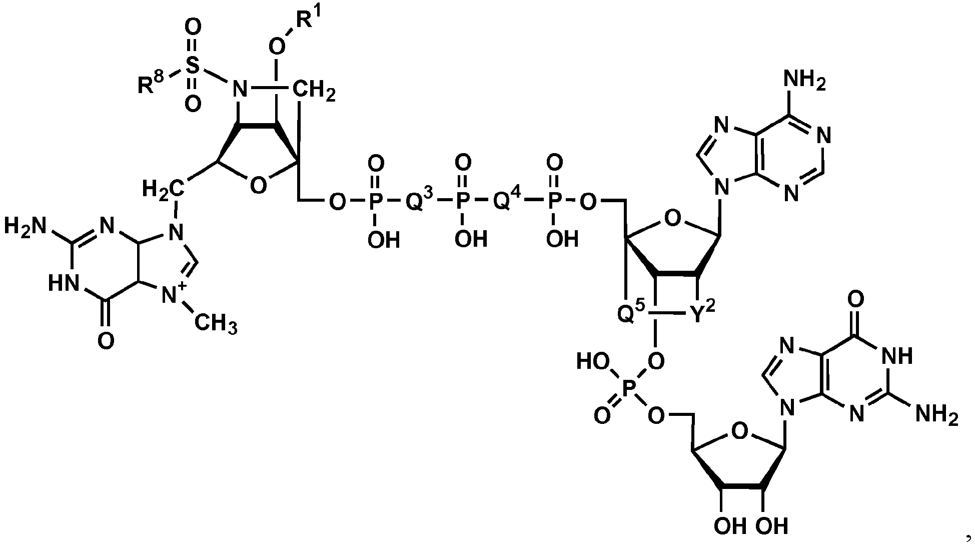 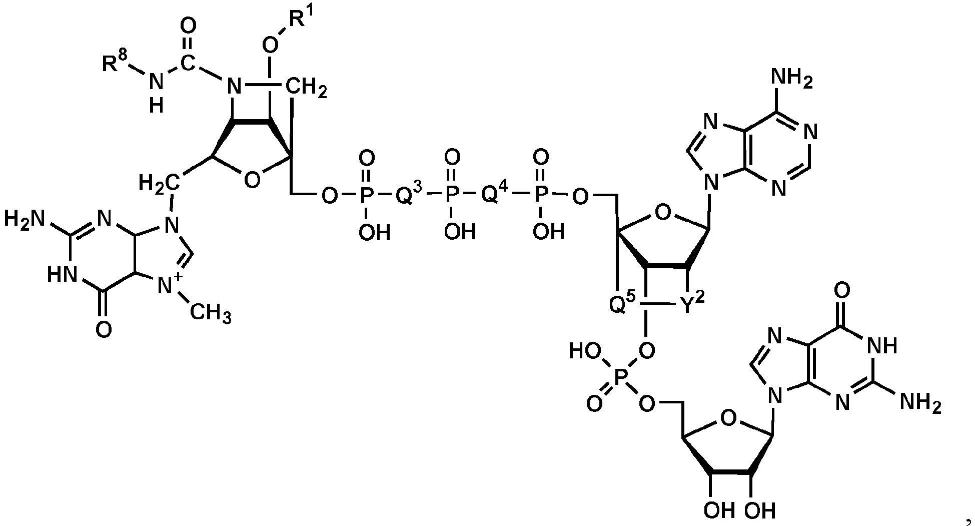 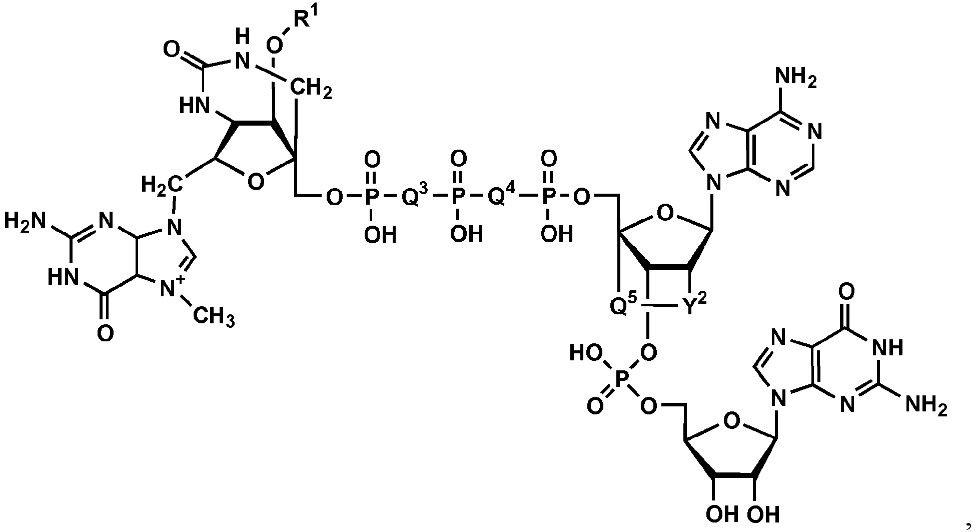 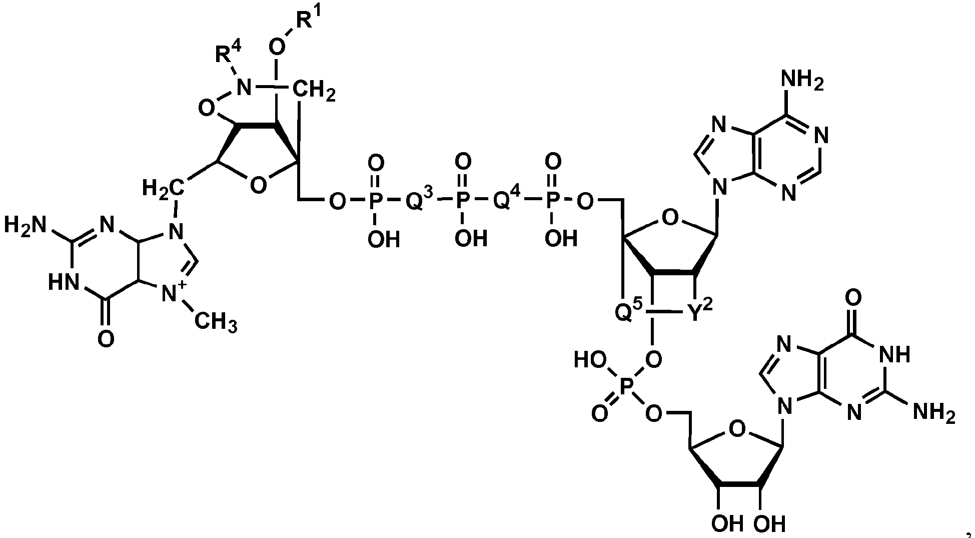 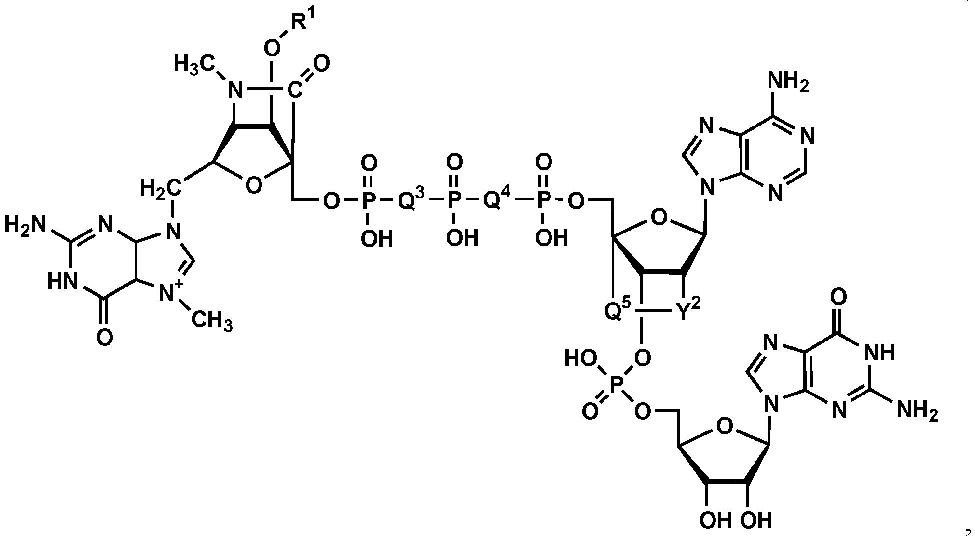 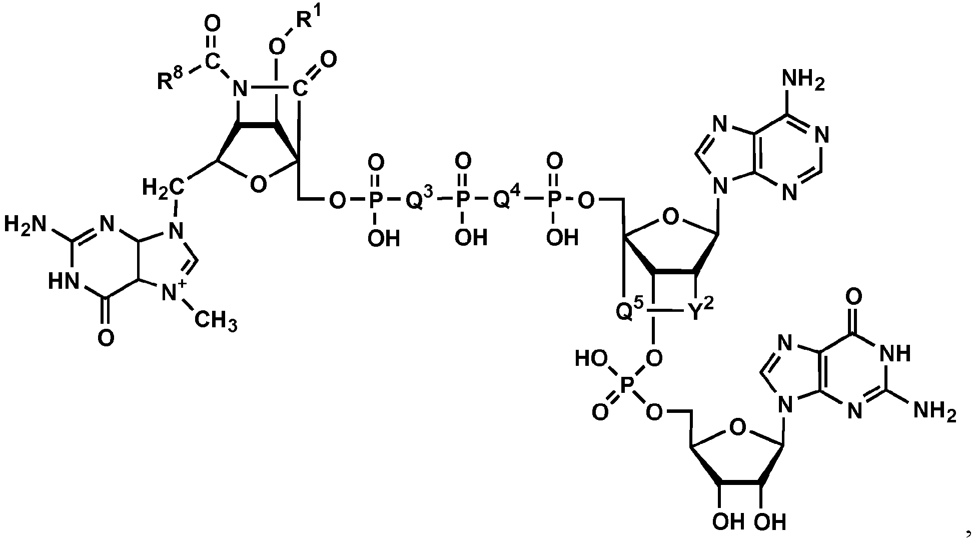 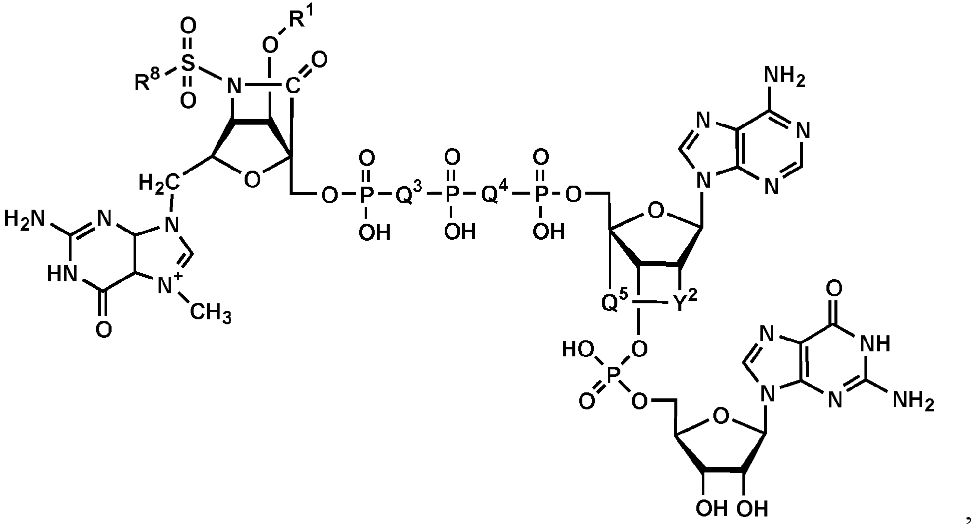 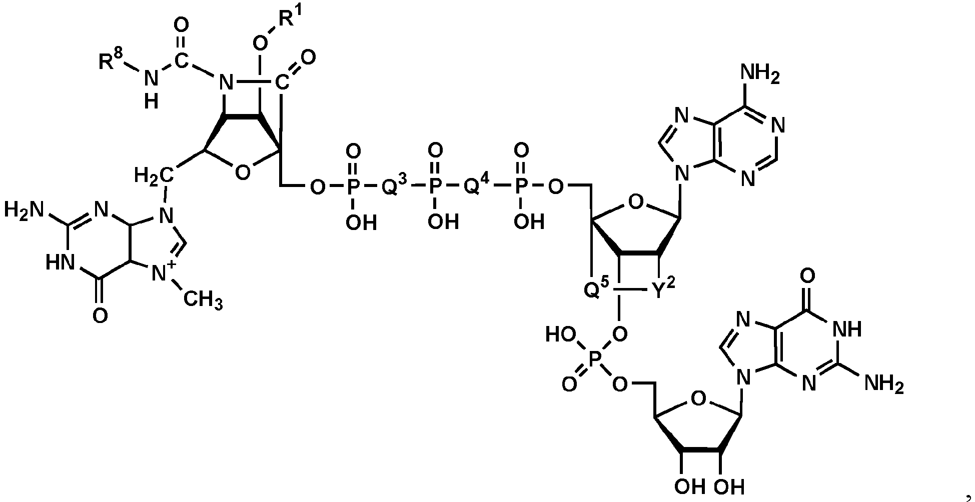 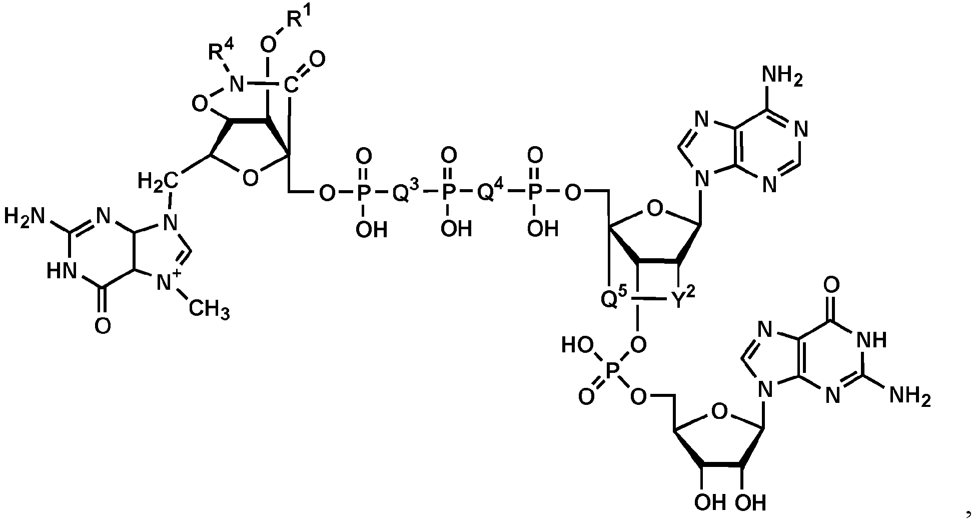 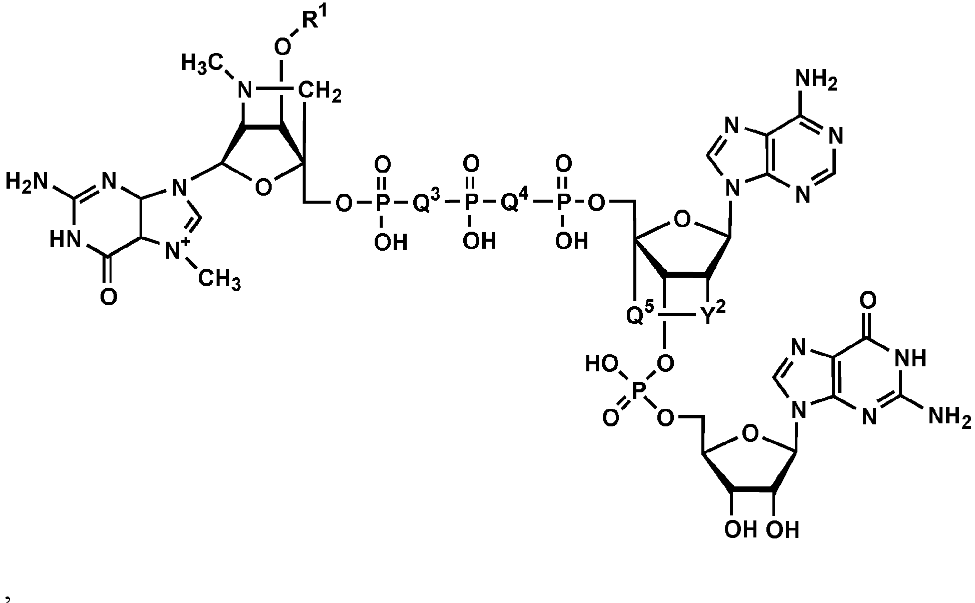 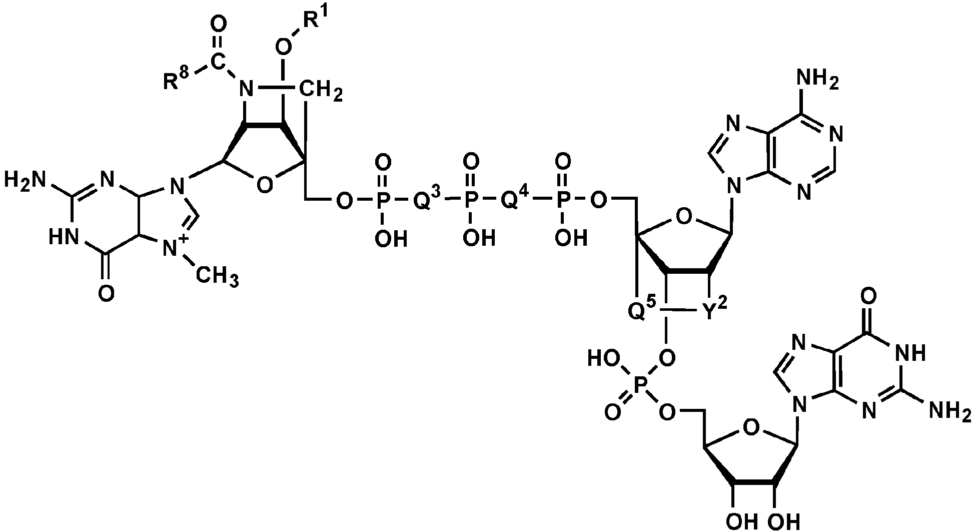 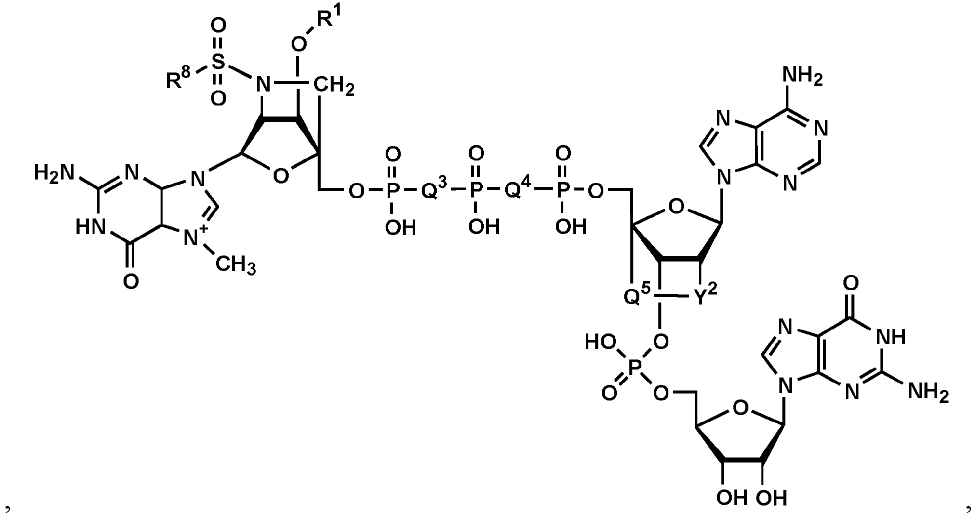 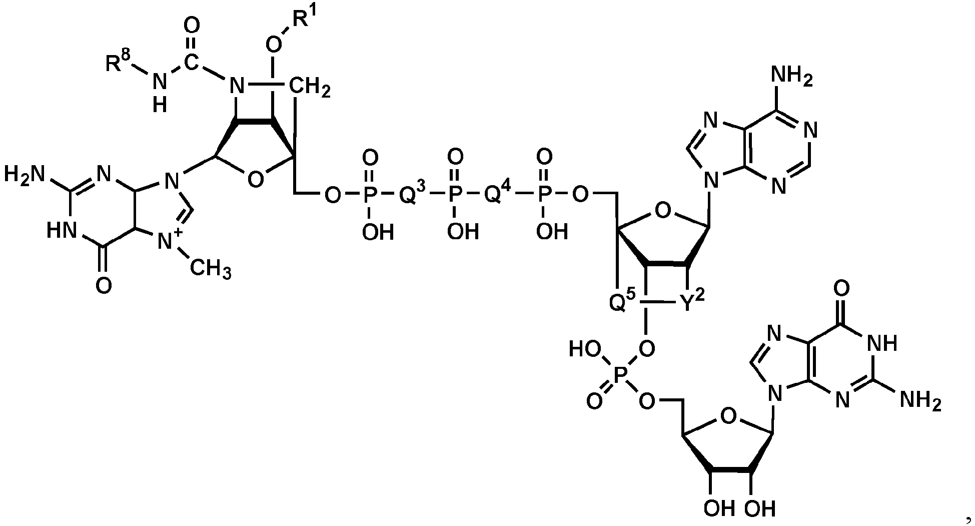 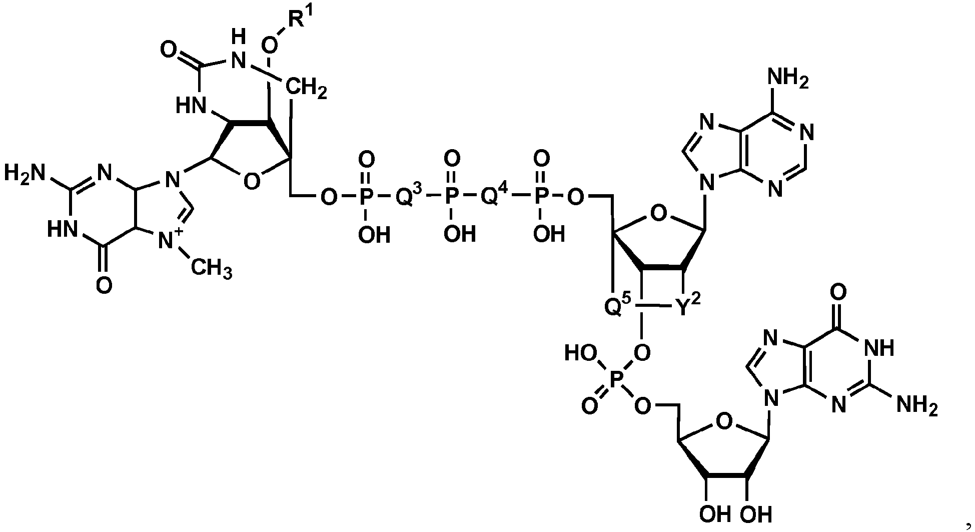 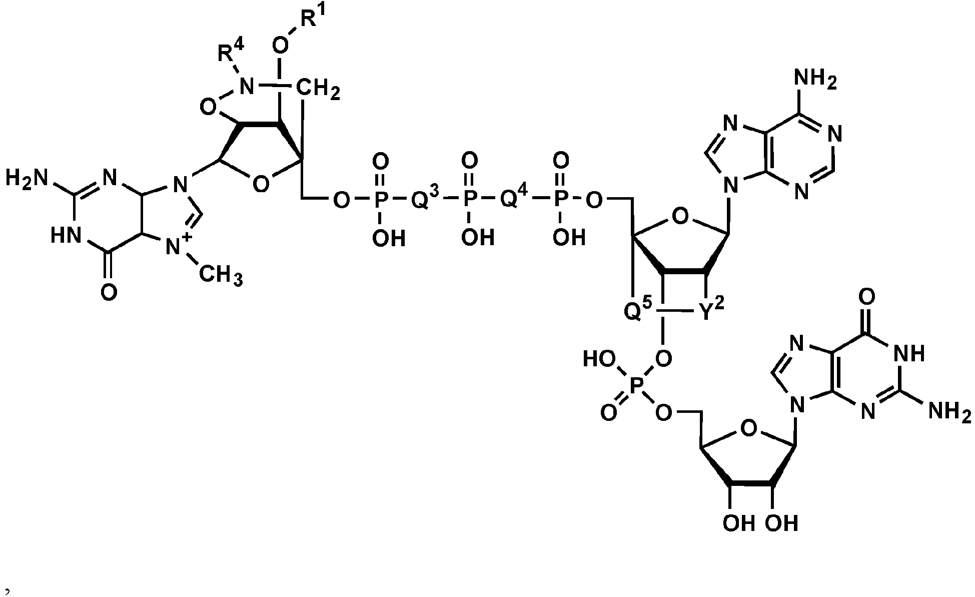 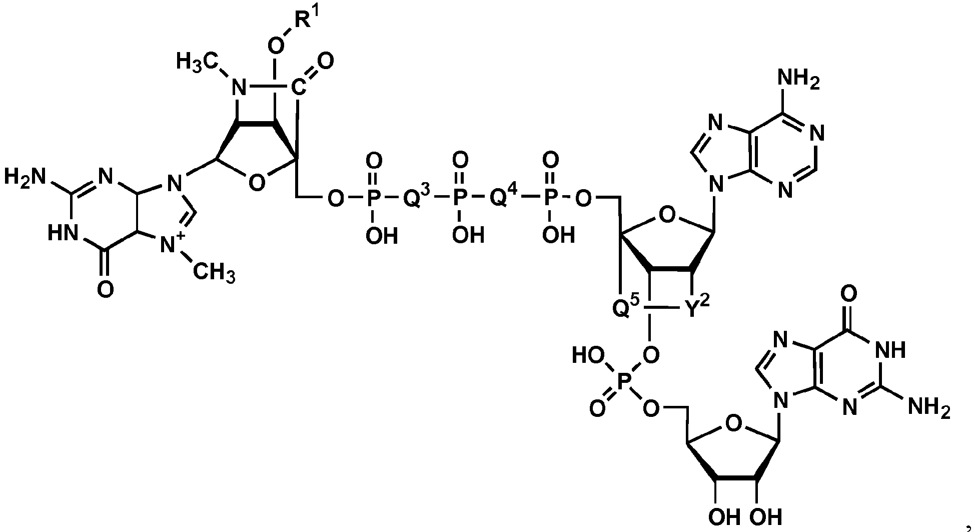 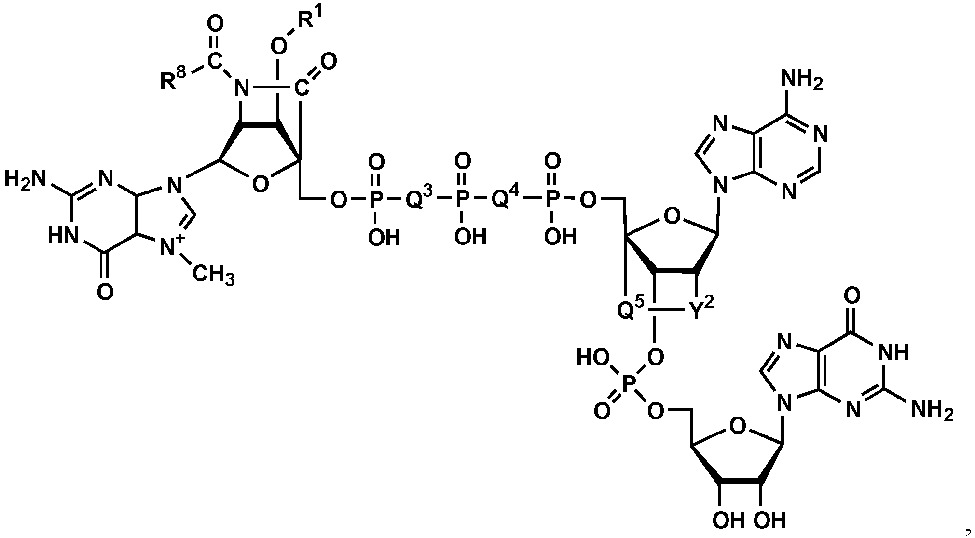 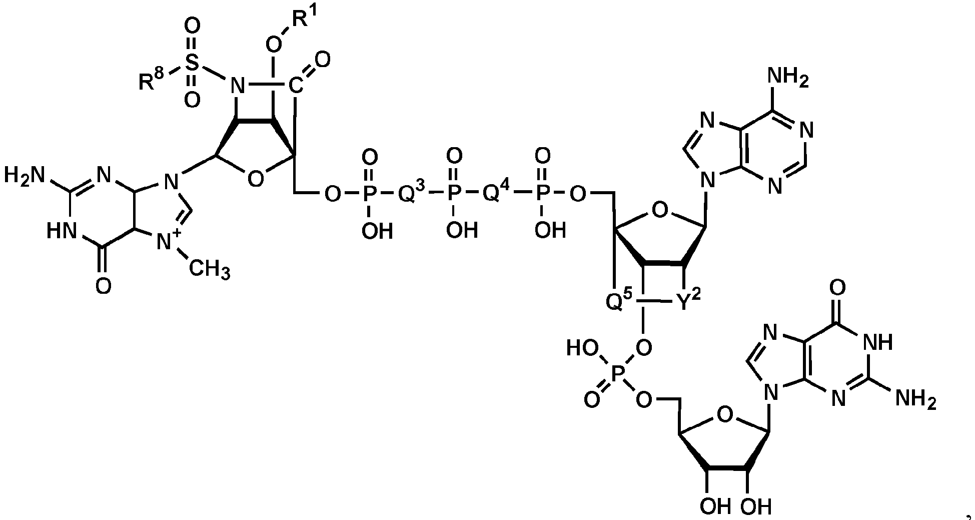 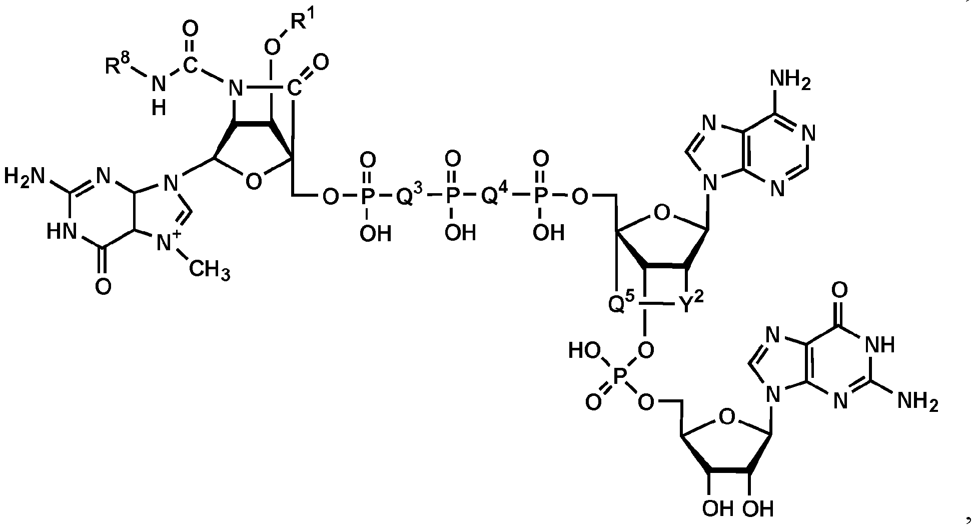 or 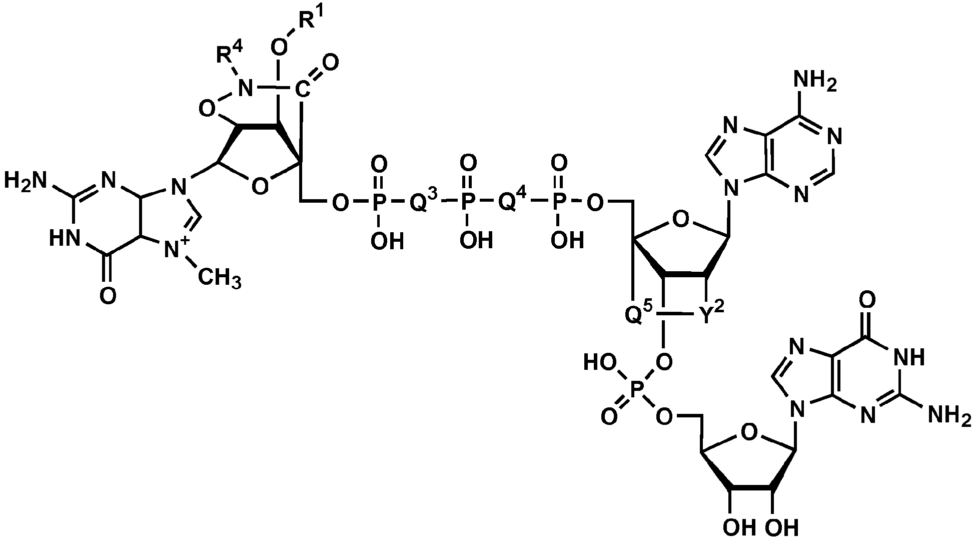 , wherein R¹ may be hydrogen, methyl or phenyl group; Q³ and Q⁴ may be independently -O-, -CH₂-, or -CCl₂-; Q⁵ is -CH₂-, or 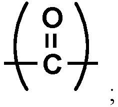 Y² may be -O-, 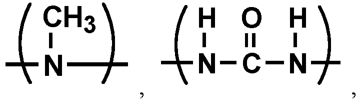 or 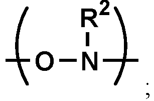 R⁴ may be hydrogen or methyl; and R⁸ may be C1-C6 alkyl group, C4-C8 cycloalkyl group, phenyl group, benzyl group or C3-C5 heterocyclic group.

According to embodiments of the disclosure, the compound may be 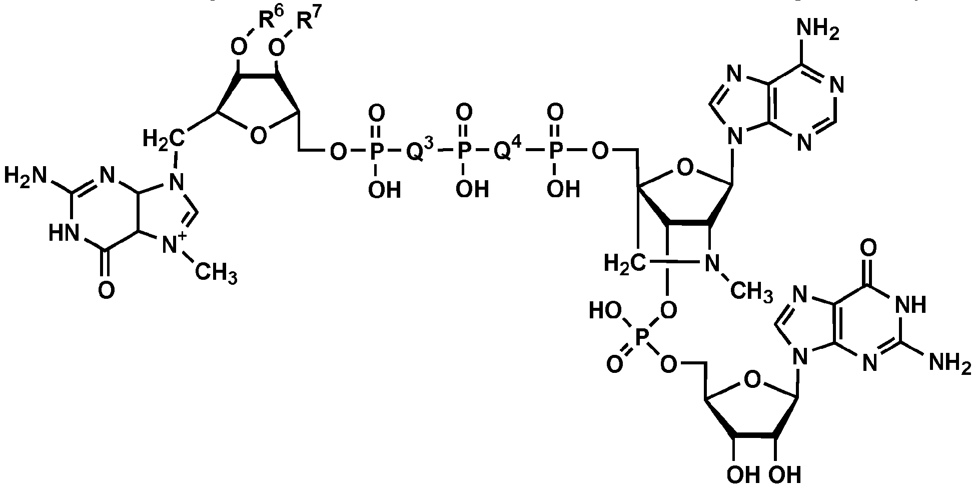 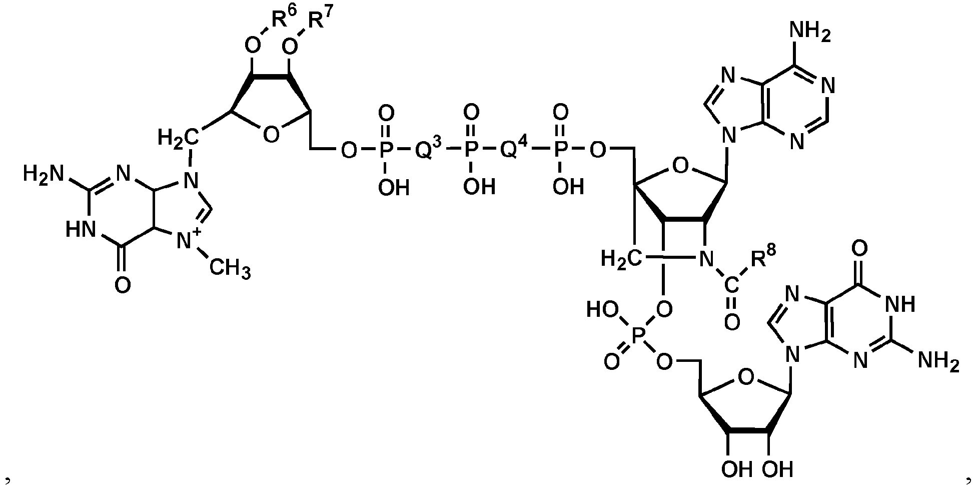 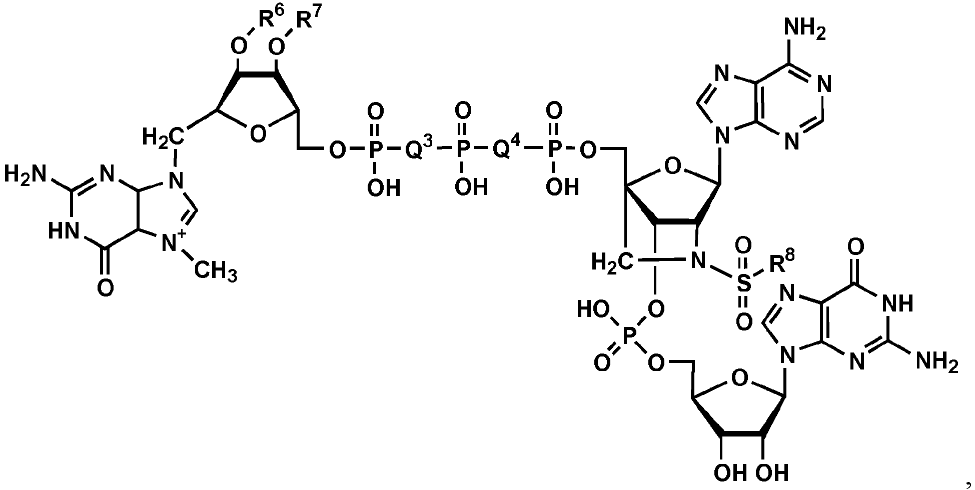 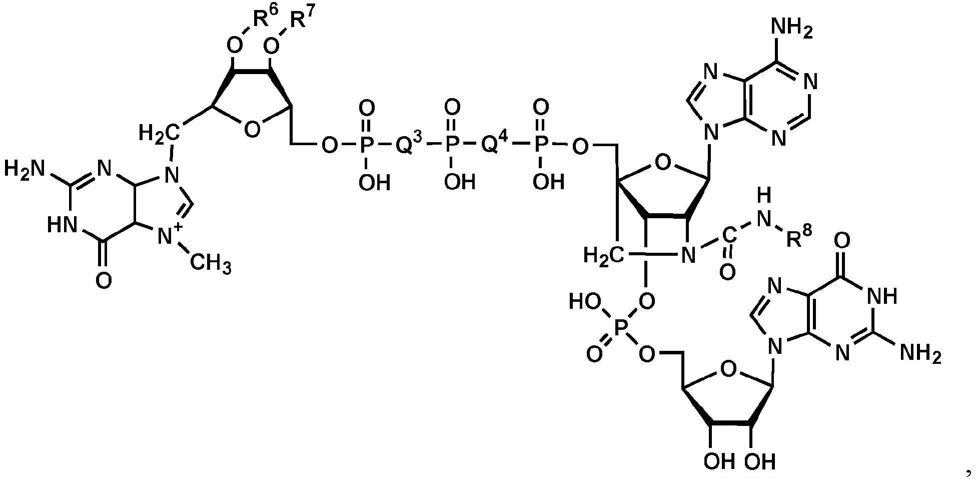 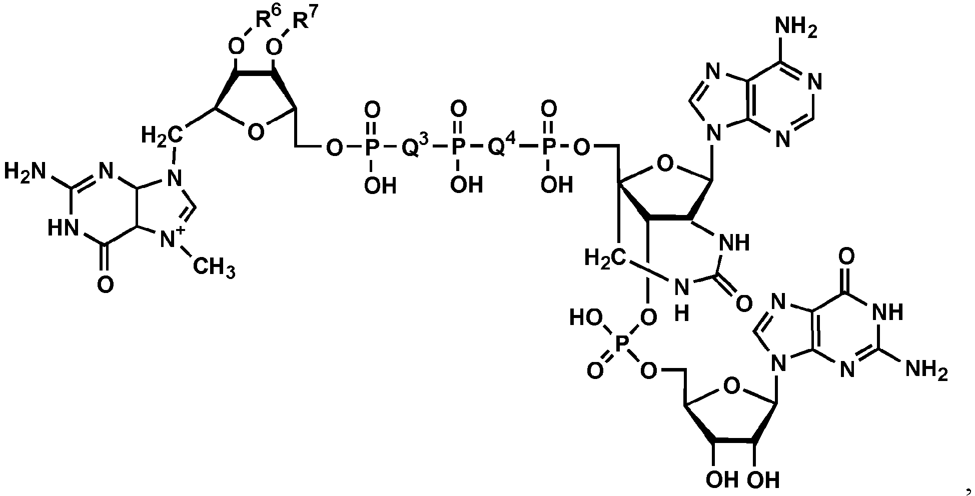 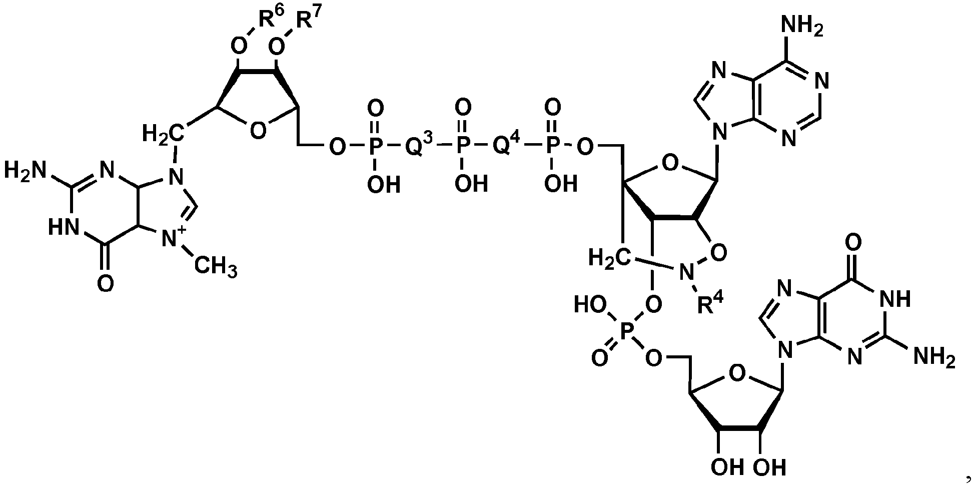 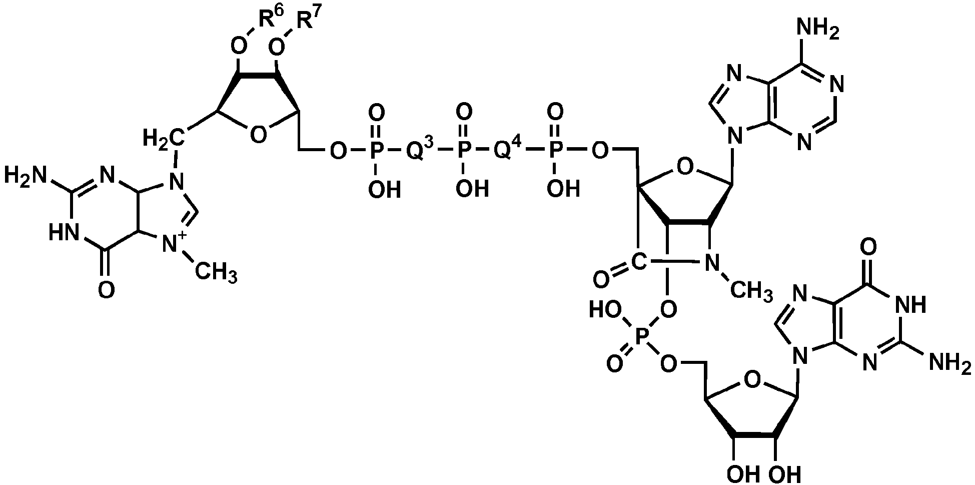 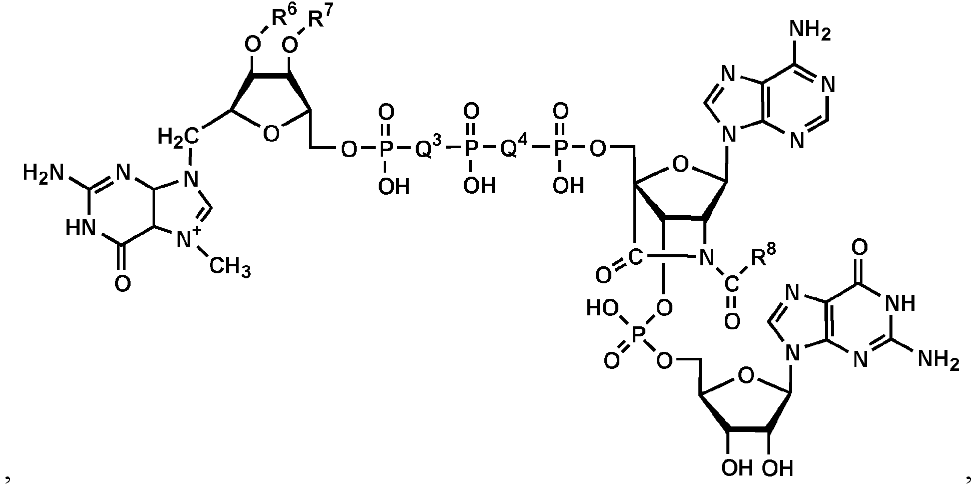 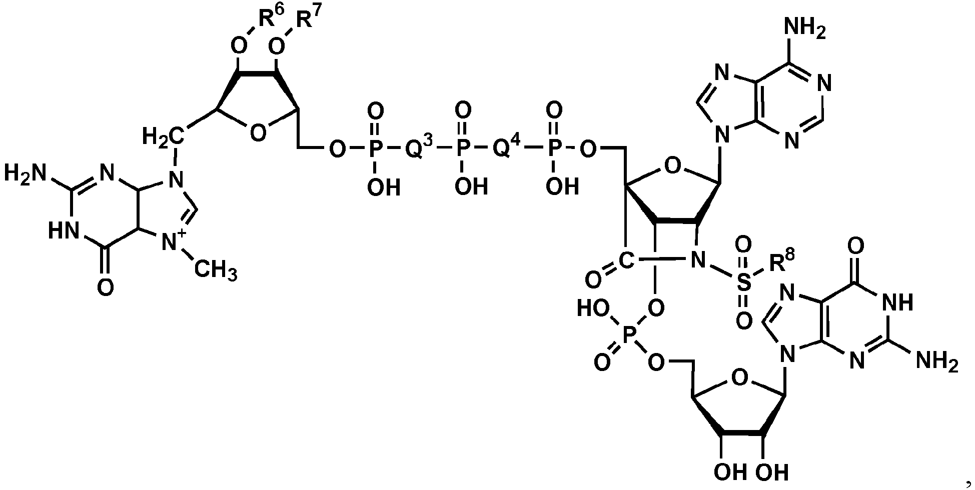 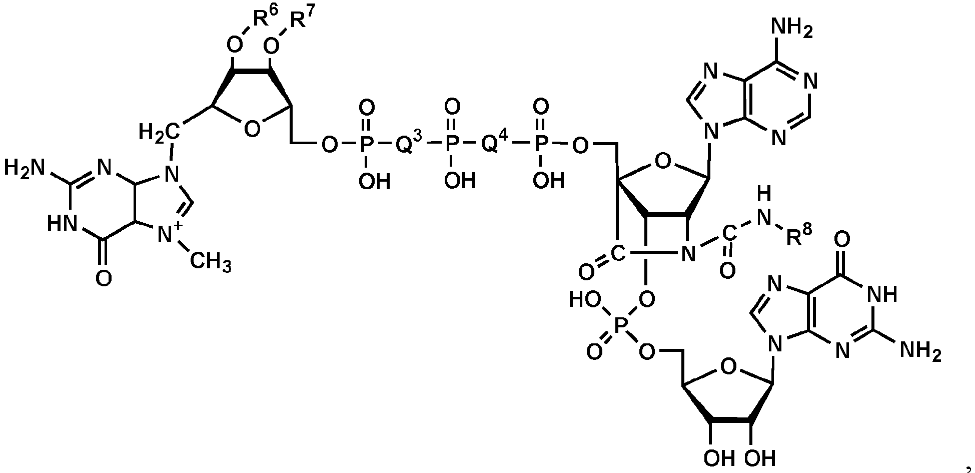 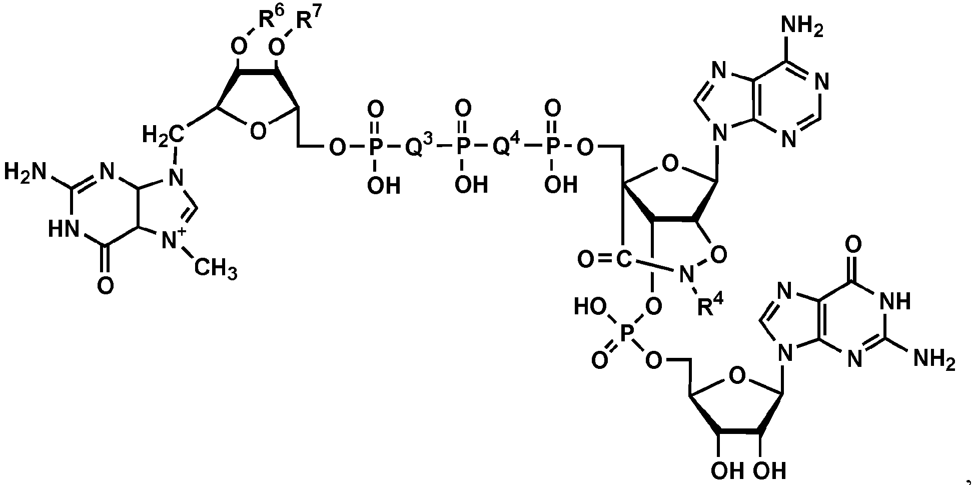 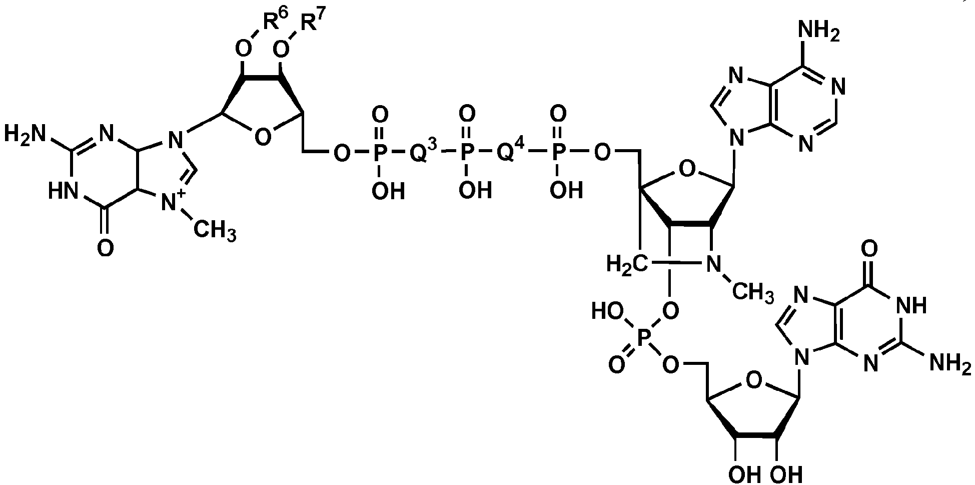 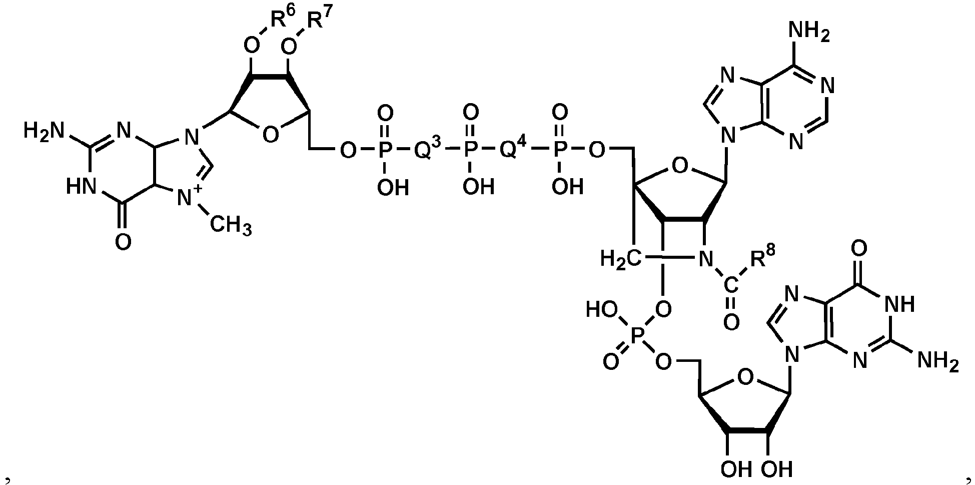 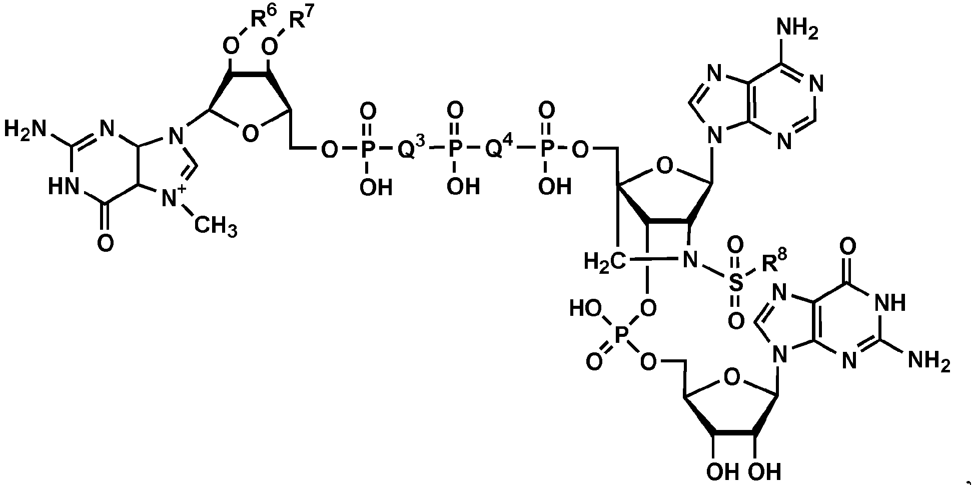 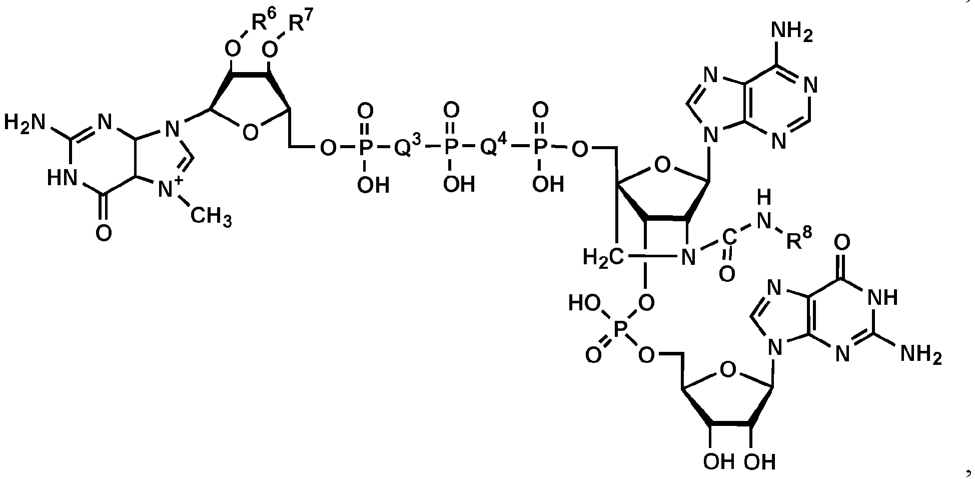 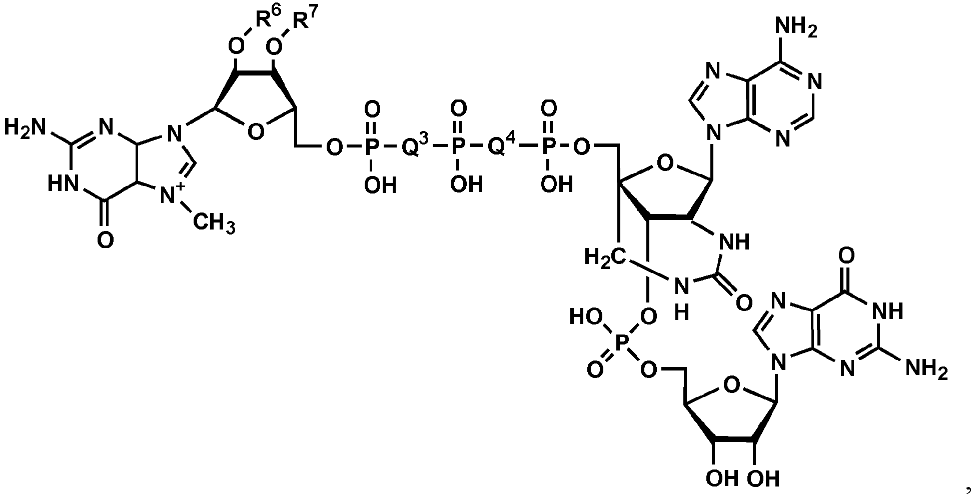 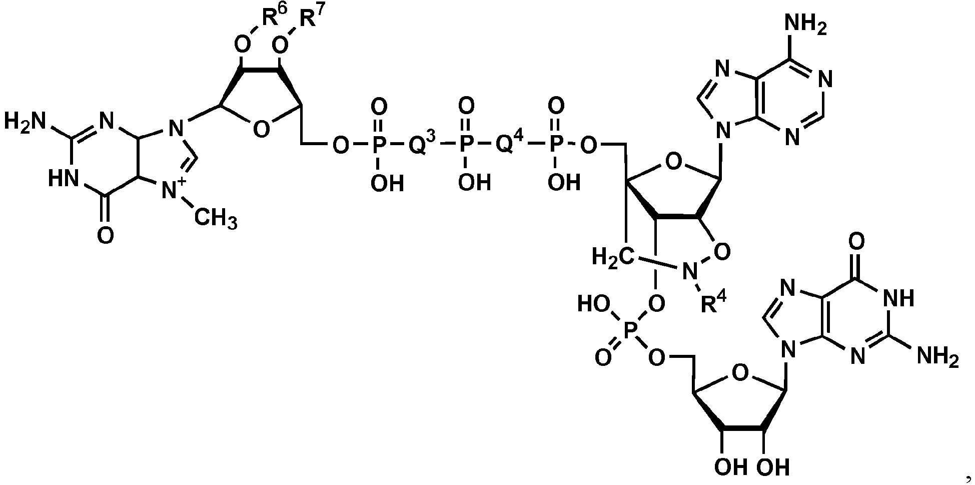 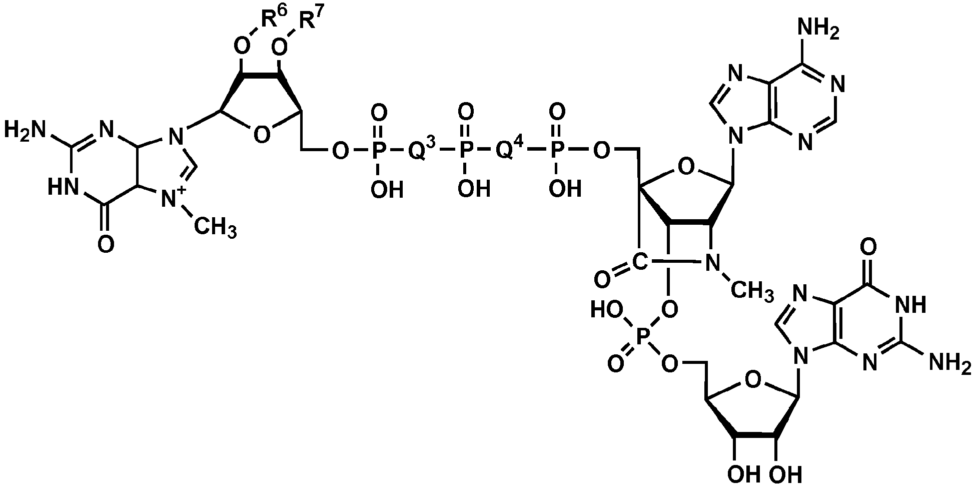 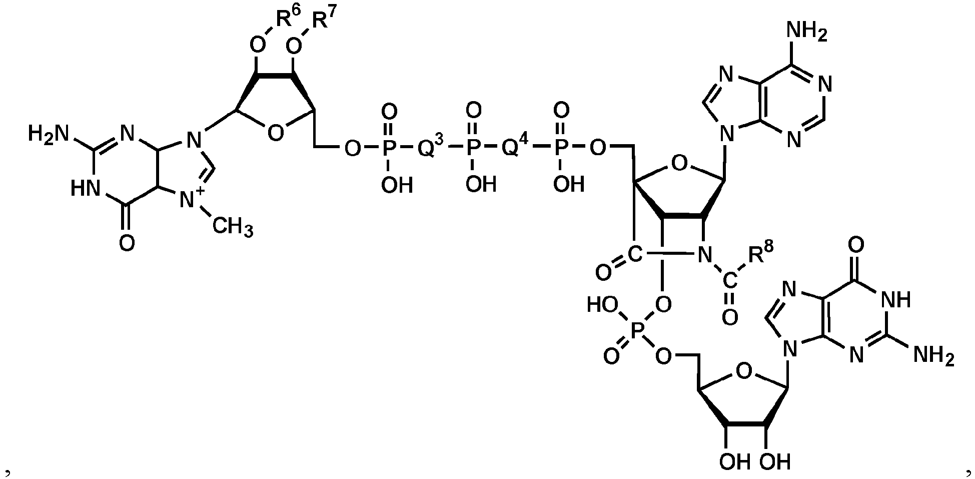 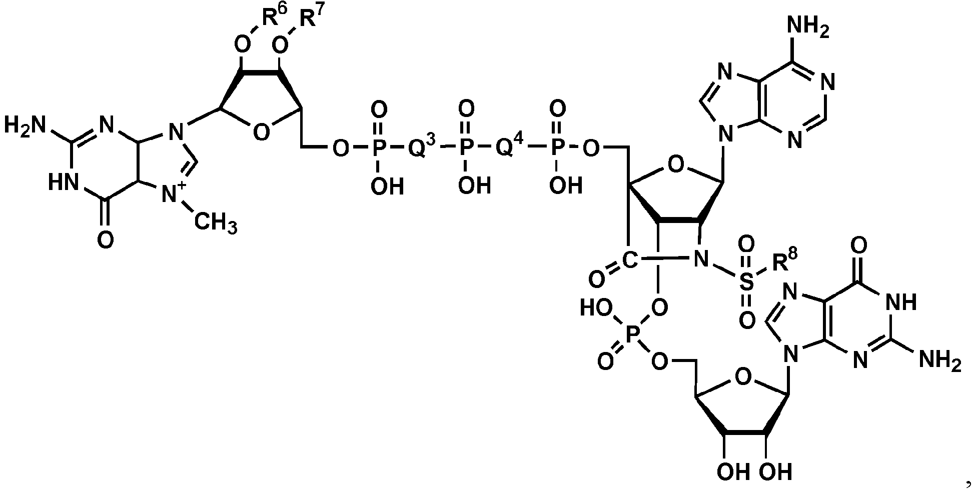 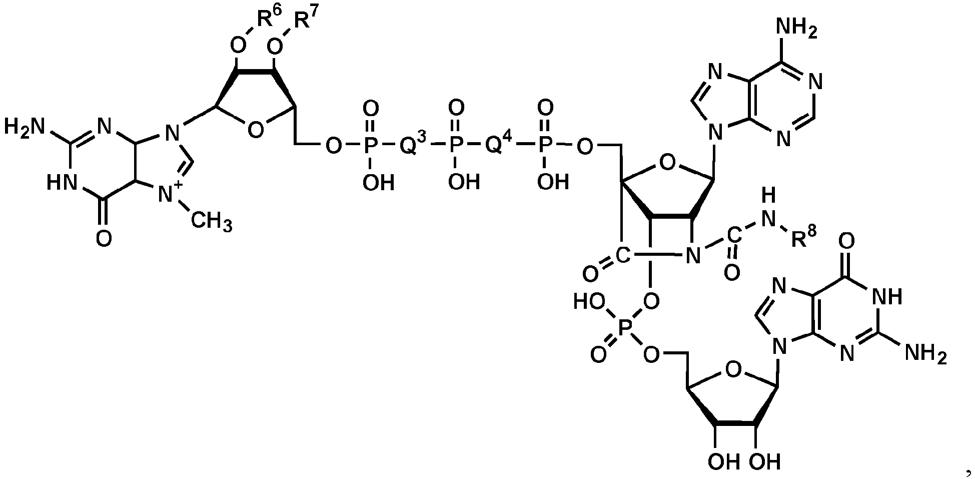 or 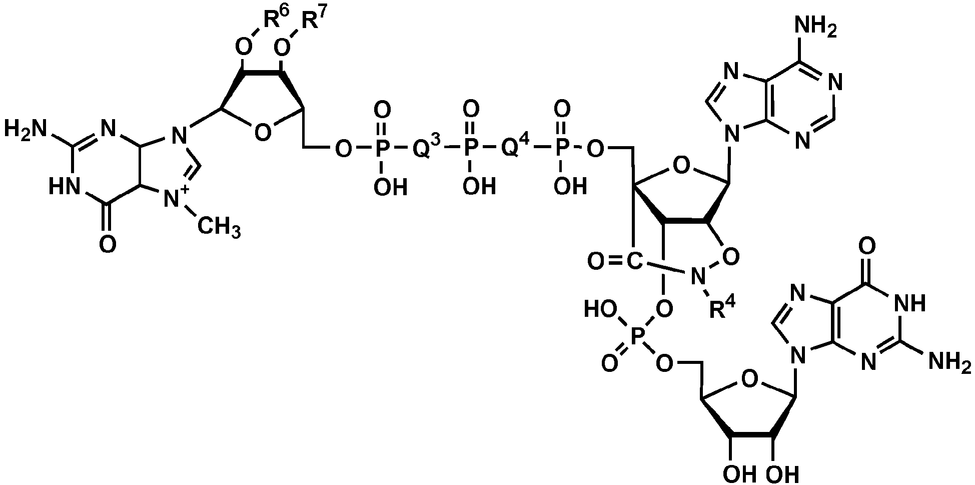 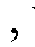 wherein Q³ and Q⁴ are independently -O-, -CH₂-, or -CCl₂-; R⁴ is hydrogen or methyl; R⁶ and R⁷ are hydrogen, methyl or phenyl group; and R⁸ is C1-C6 alkyl group, C4-C8 cycloalkyl group, phenyl group, benzyl group or C3-C5 heterocyclic group.

According to embodiments of the disclosure, the compound may be 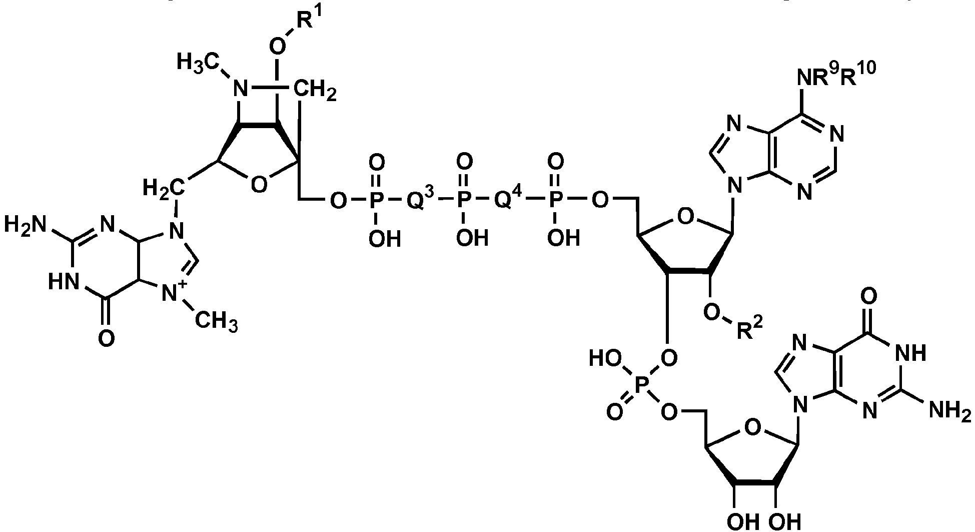 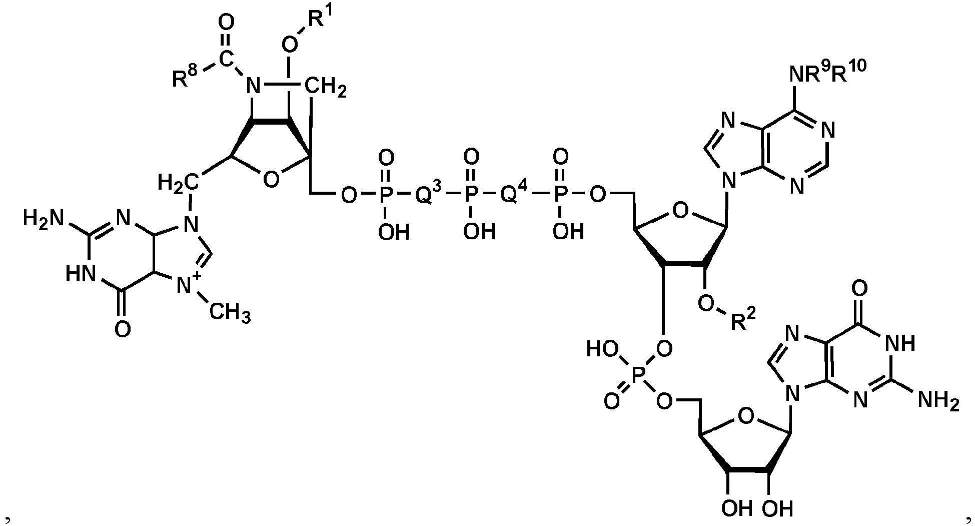 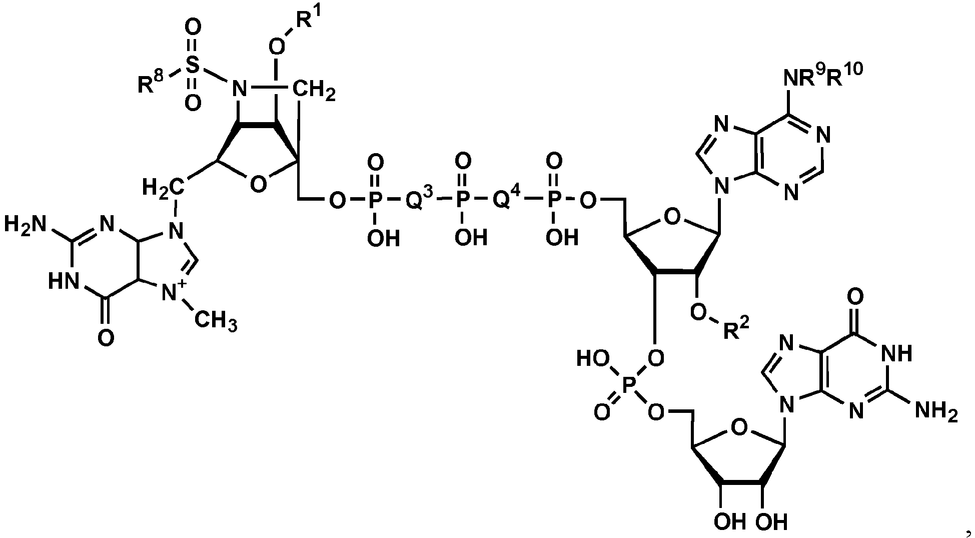 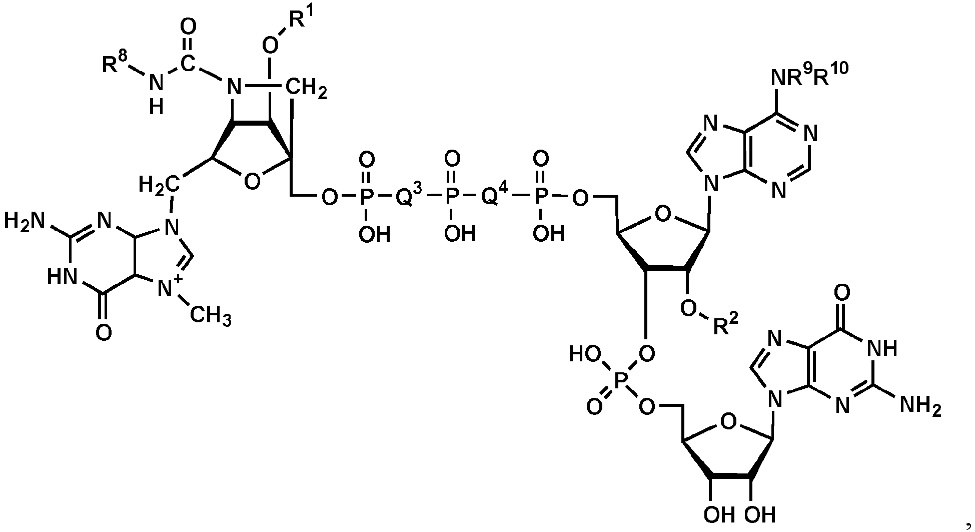 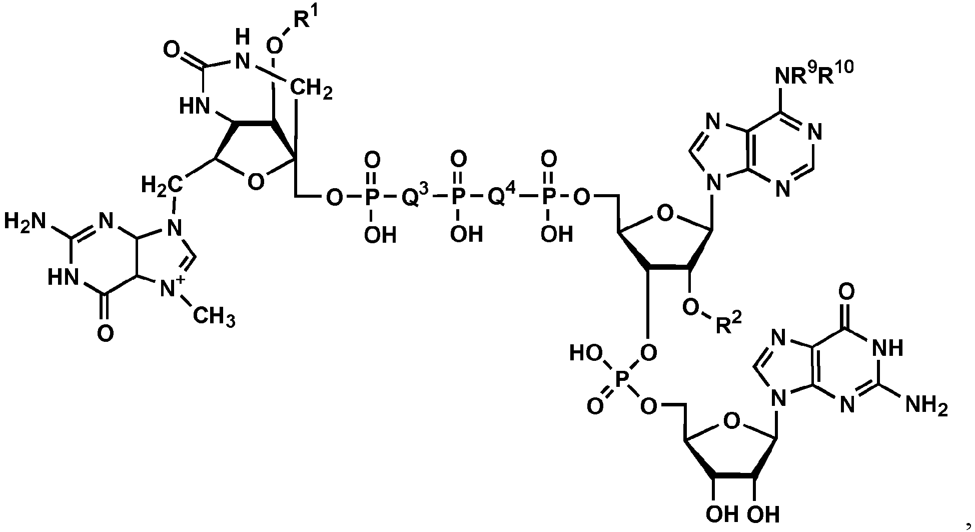 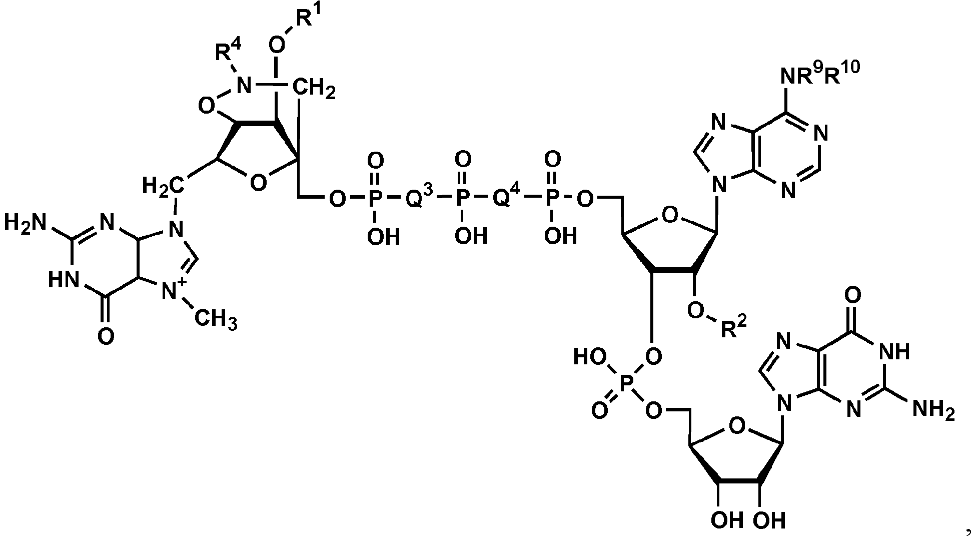 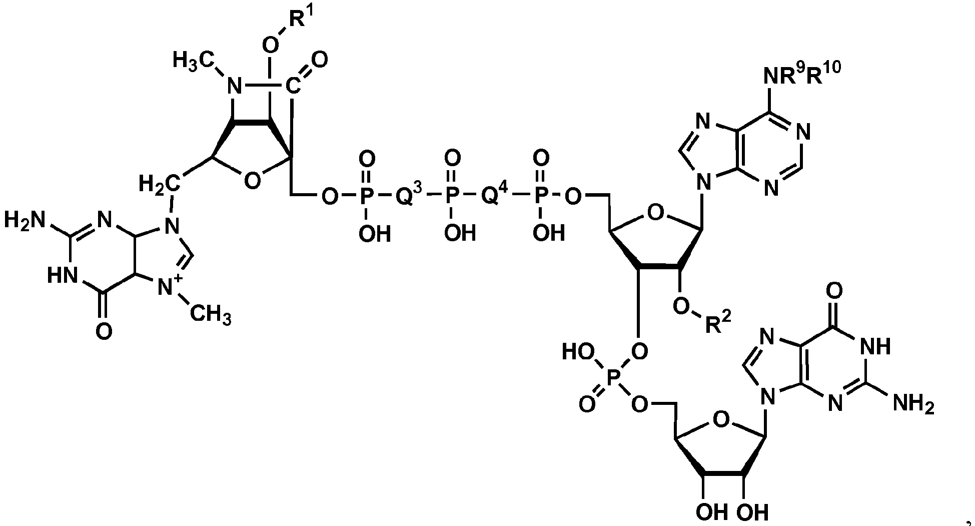 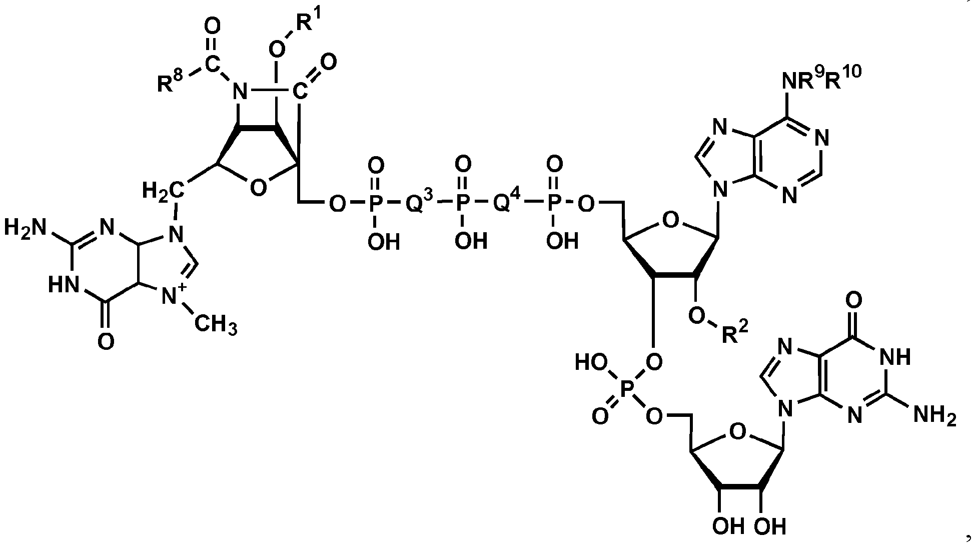 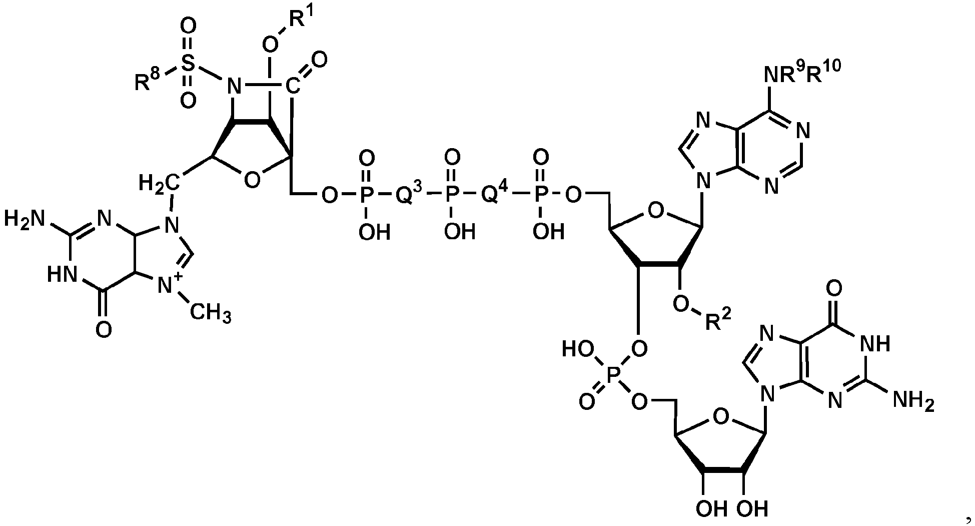 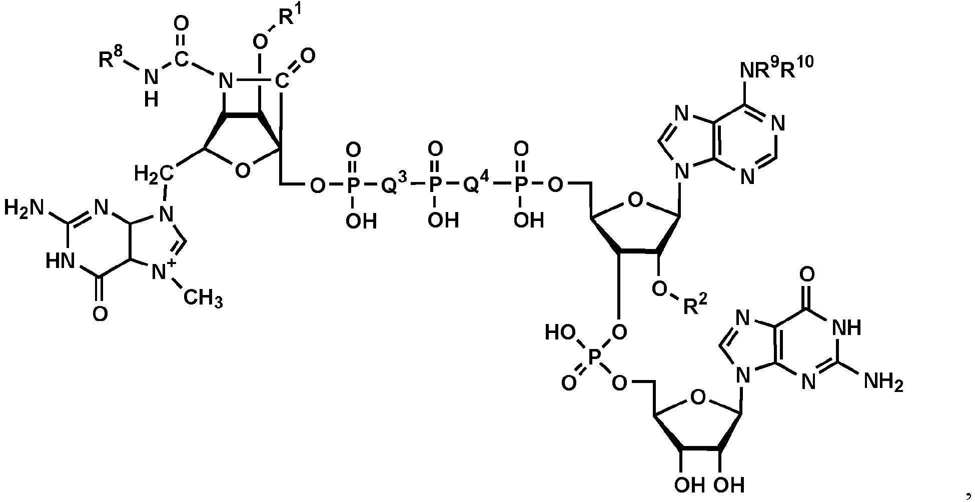 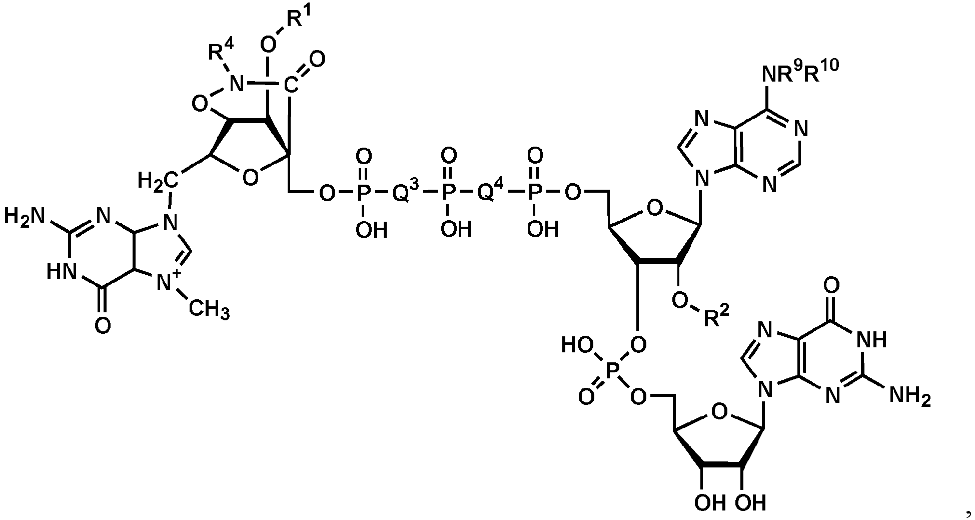 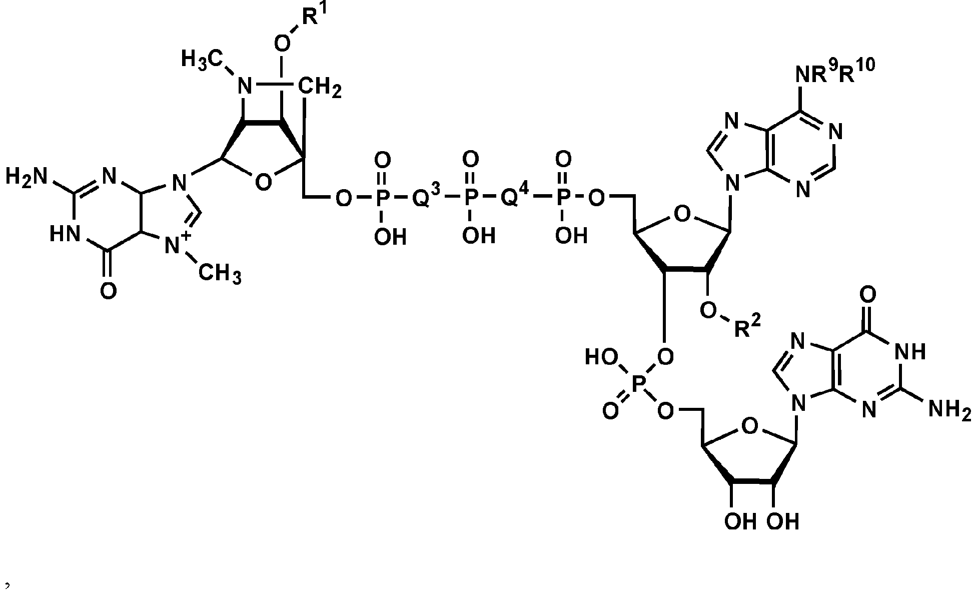 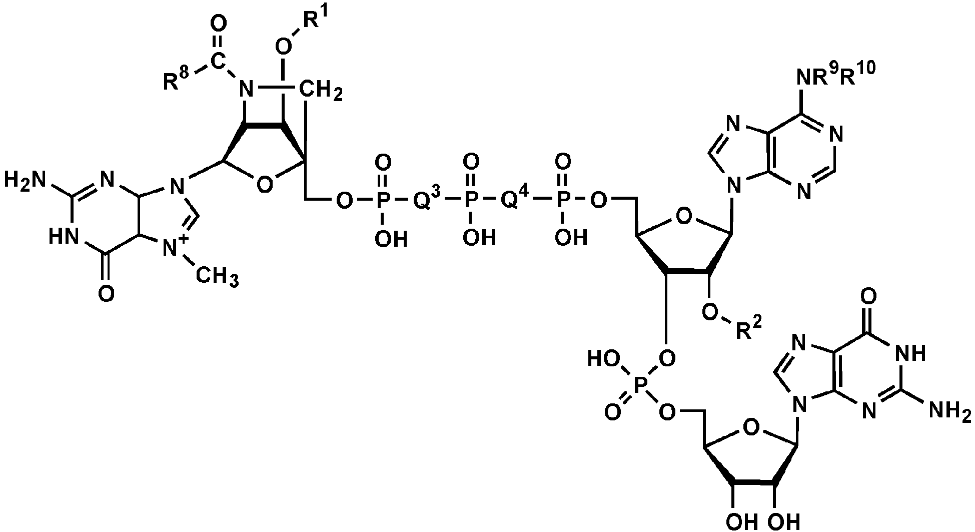 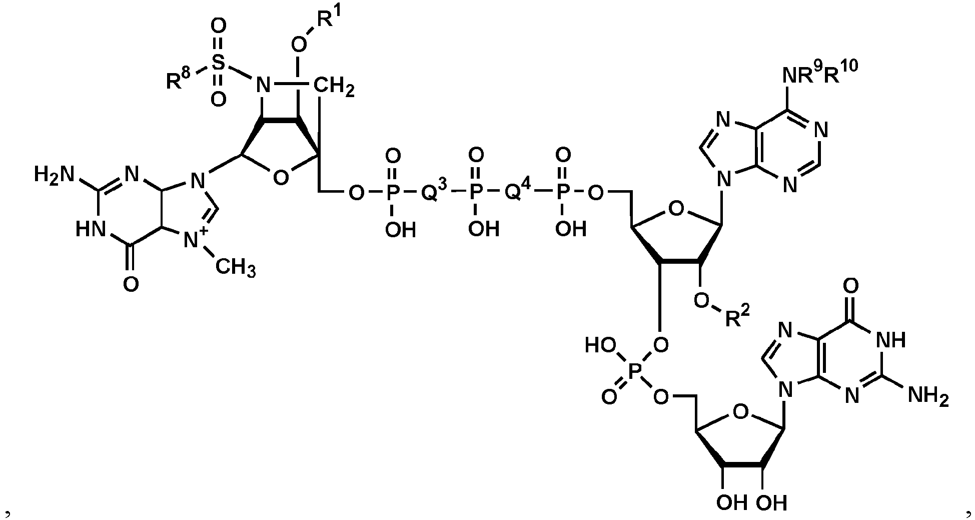 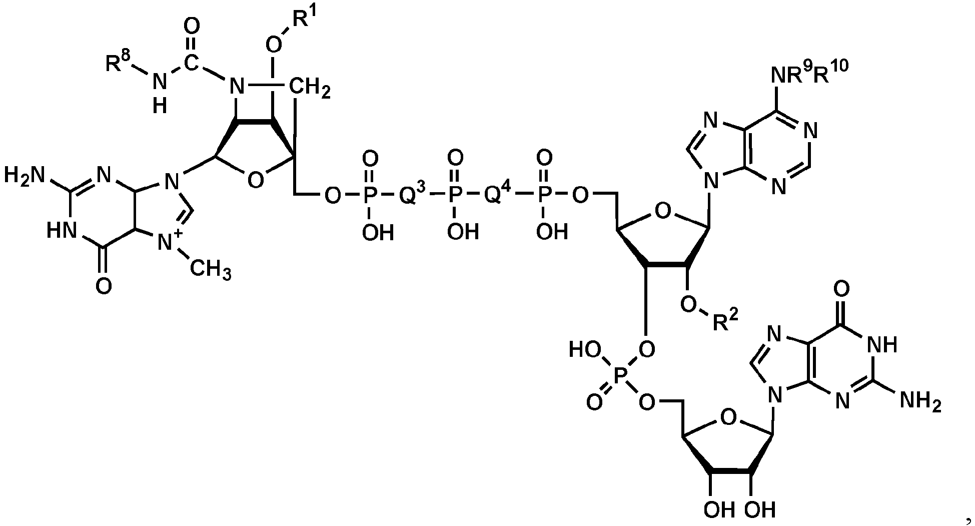 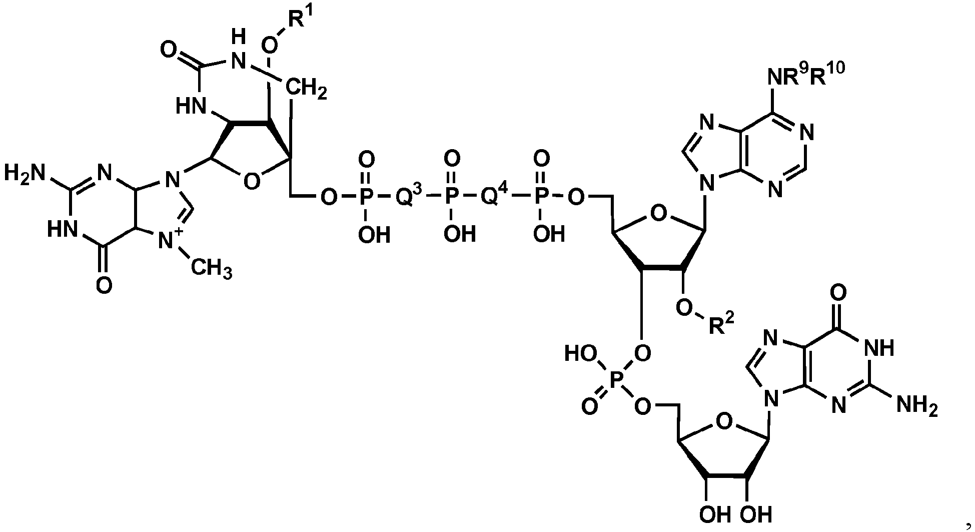 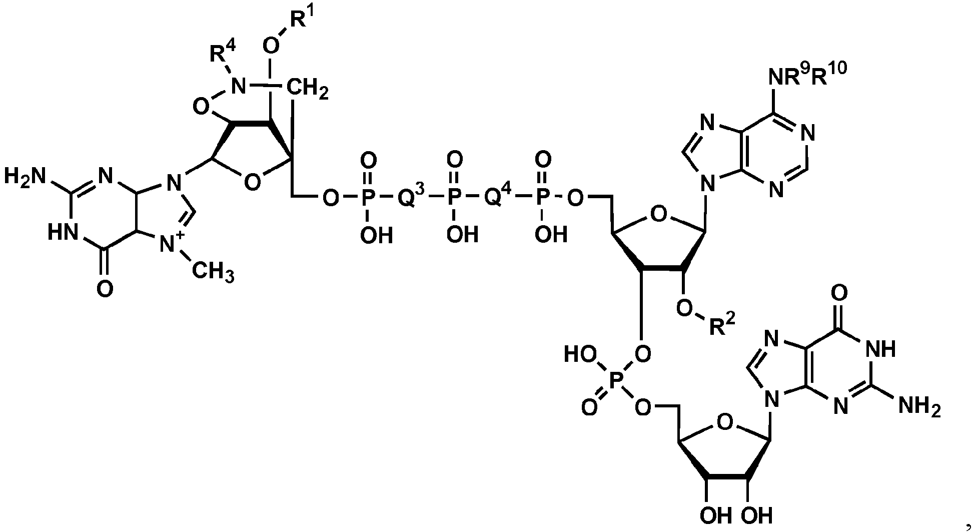 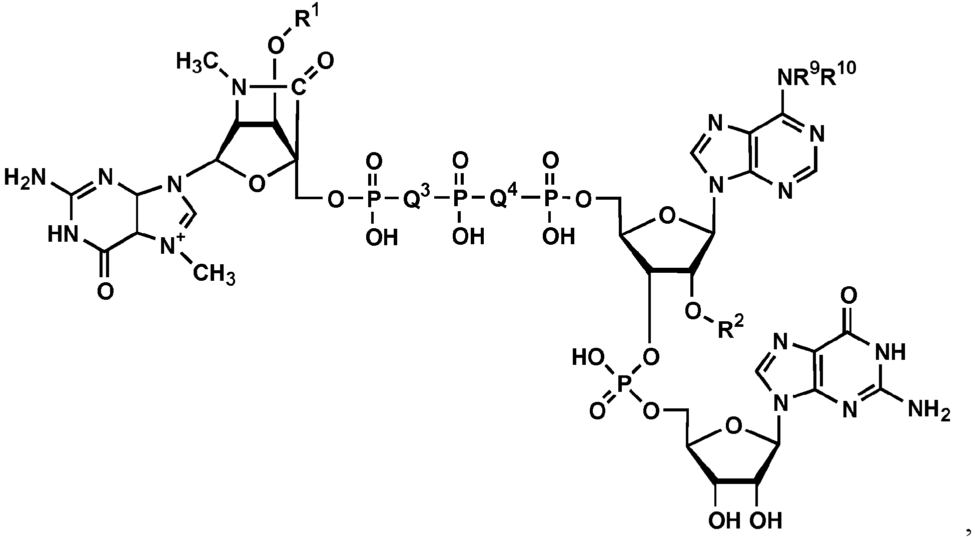 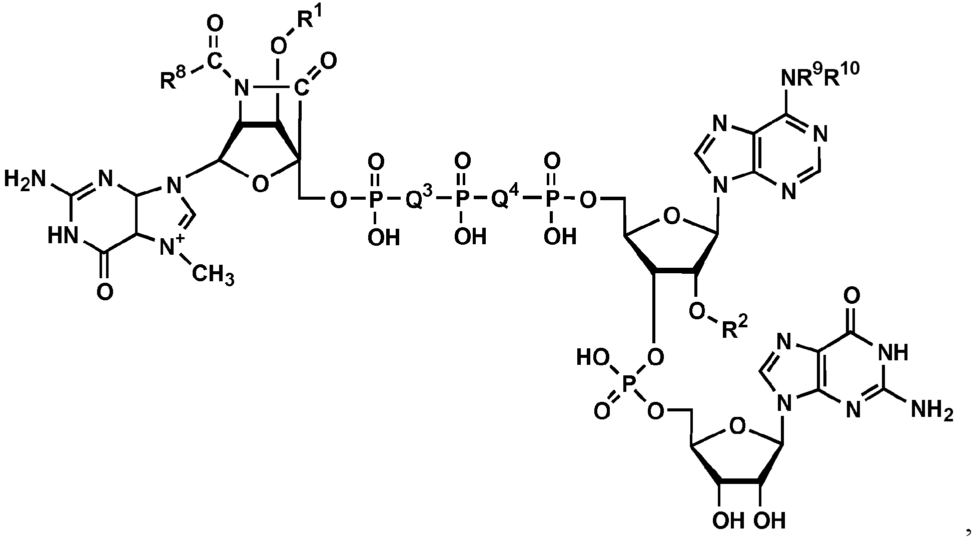 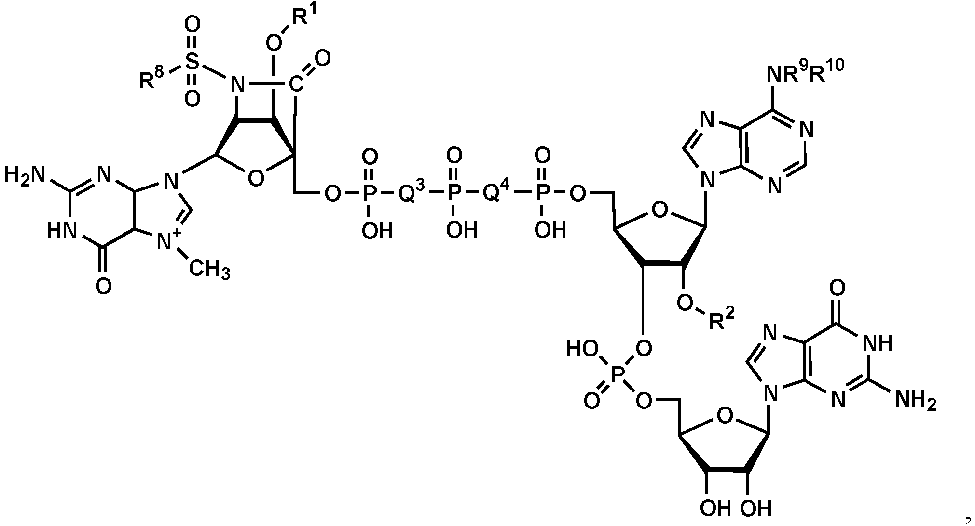 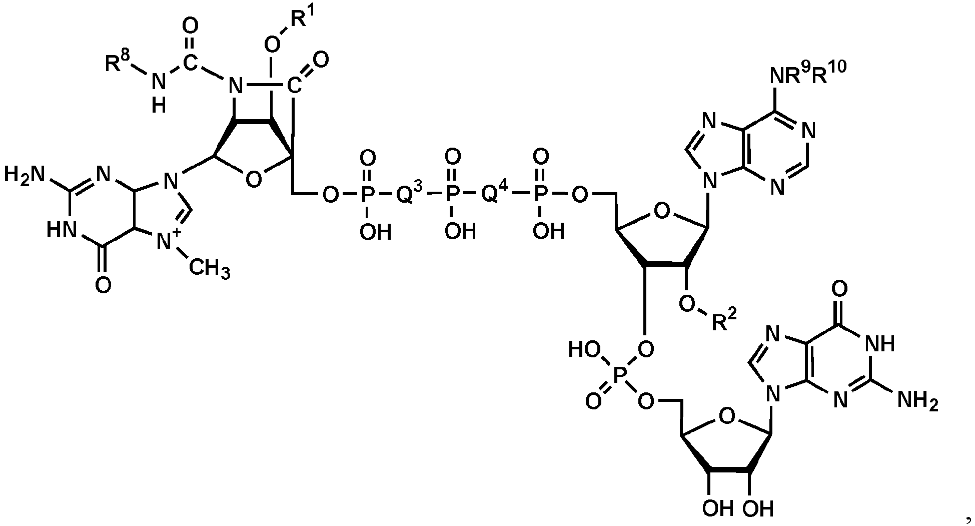 or 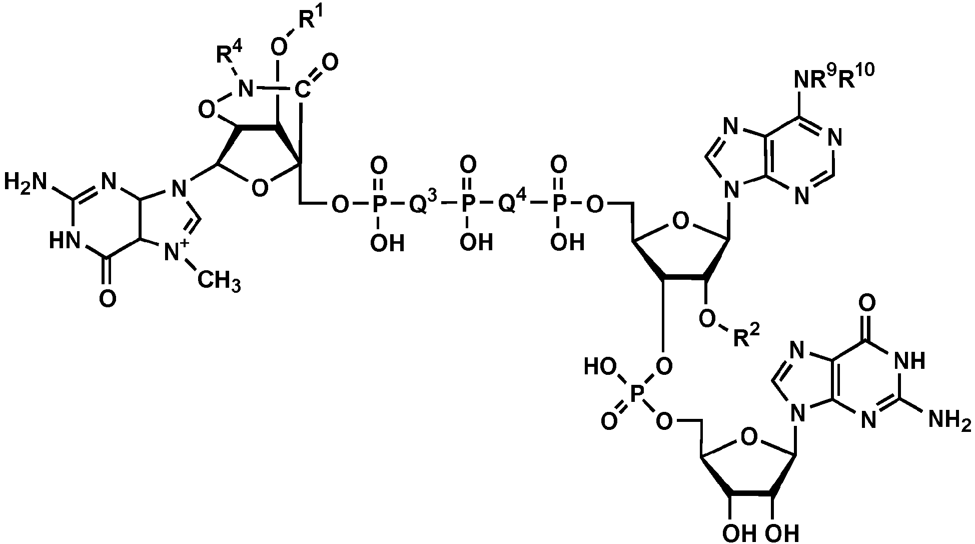 , wherein R¹ is hydrogen, methyl or phenyl group; R² is hydrogen or methyl; Q³ and Q⁴ are independently -O-, -CH₂-, or -CCl₂-; Q⁵ is -CH₂-, or 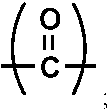 R⁴ is hydrogen or methyl; R⁸ is C1-C6 alkyl group, C4-C8 cycloalkyl group, phenyl group, benzyl group or C3-C5 heterocyclic group; and R⁹ and R¹⁰ are independently hydrogen, C1-C6 alkyl group, or benzyl group.

According to embodiments of the disclosure, the method for preparing the compound having a structure represented by Formula (I) of the disclosure is not limited, and the preparation may be carried out using the following reaction equation:

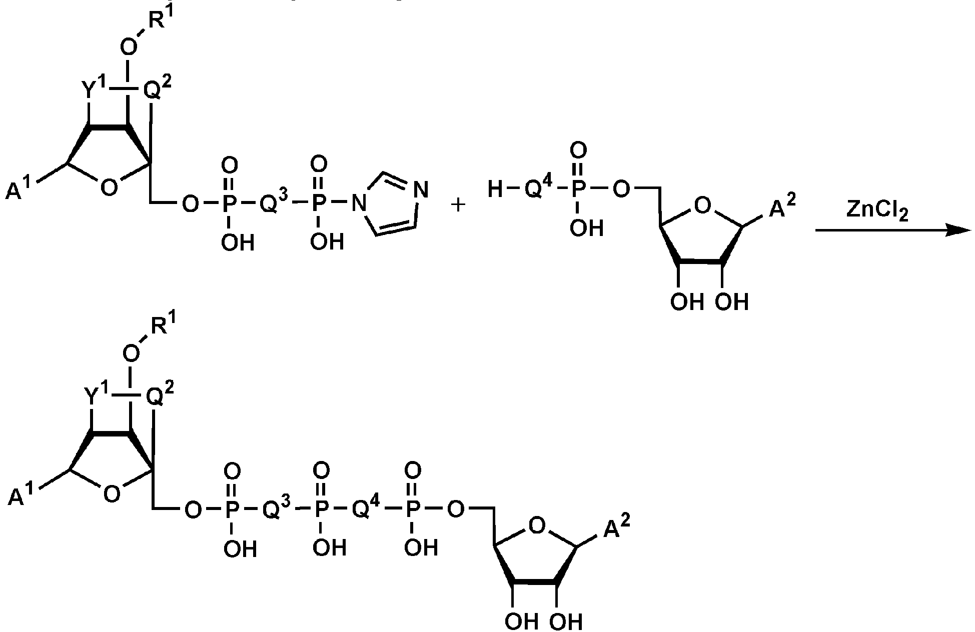

According to embodiments of the disclosure, the method for preparing the compound having a structure represented by Formula (II) of the disclosure is not limited, and the preparation may be carried out using the following reaction equation:

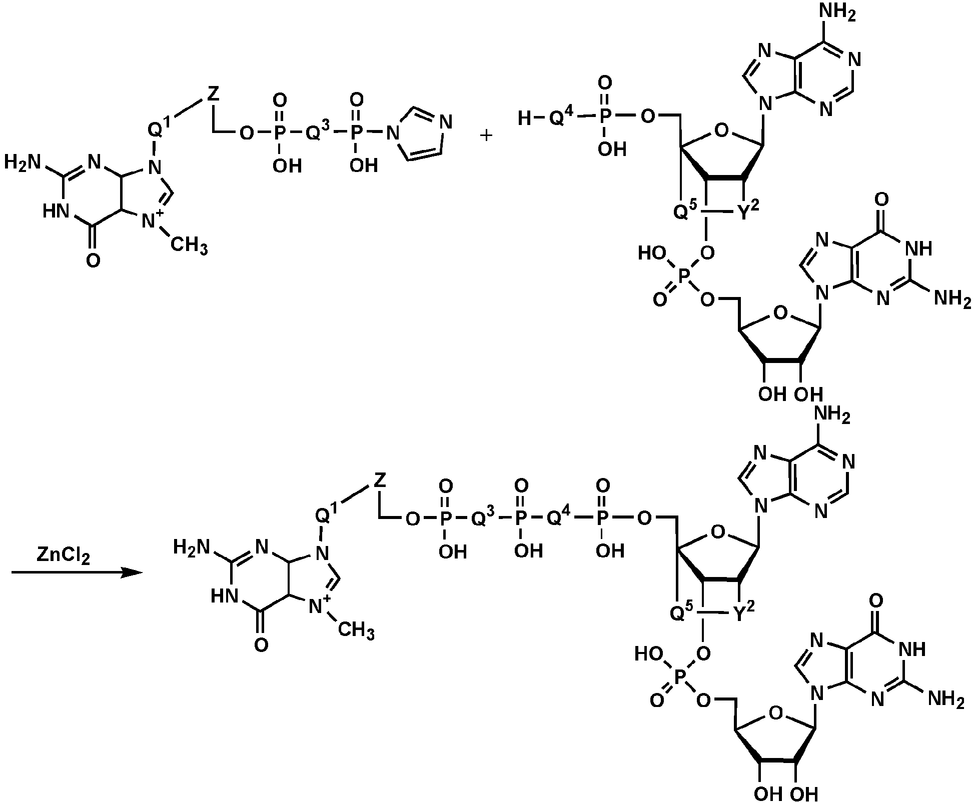

According to embodiments of the disclosure, the method for preparing the compound having a structure represented by Formula (III) of the disclosure is not limited, and the preparation may be carried out using the following reaction equation:

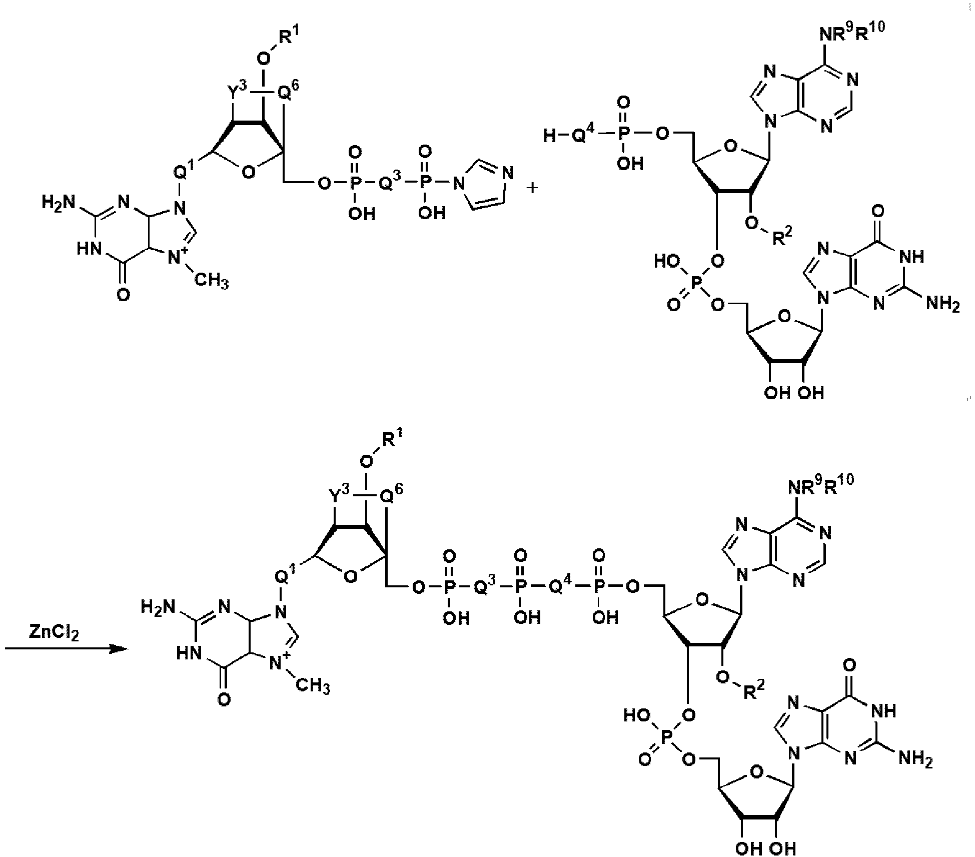

According to embodiments of the disclosure, the disclosure provides a pharmaceutical composition, which includes a compound and an RNA molecule. According to embodiments of the disclosure, the compound has a structure represented by Formula (I), Formula (II), or Formula (III), and the compound may covalently bond with the RNA molecule (i.e., it may react with the RNA molecule to form a covalent bond). According to embodiments of the disclosure, the RNA molecule is an mRNA molecule.

According to embodiments of the disclosure, the disclosure provides a kit for capping an RNA transcript, including a compound and an RNA polymerase. According to embodiments of the disclosure, the compound has a structure represented by Formula (I), Formula (II), or Formula (III).

According to embodiments of the disclosure, the kit for capping an RNA transcript of the disclosure may further include an RNA molecule, wherein the RNA molecule is an mRNA molecule.

According to embodiments of the disclosure, the disclosure provides a method for *in vitro* transcription. The method for *in vitro* transcription includes the following steps. A composition is provided, wherein the composition includes an RNA polymerase, nucleoside triphosphate, and the compound of the disclosure. And, a DNA template is performed to contact with the composition to transcribe the DNA template into RNA *in vitro.*

Below, exemplary embodiments will be described in detail with reference to the accompanying drawings so as to be easily realized by a person having ordinary knowledge in the art. The inventive concept may be embodied in various forms without being limited to the exemplary embodiments set forth herein. Descriptions of well-known parts are omitted for clarity, and like reference numerals refer to like elements throughout.

The compound having the structure represented by Formula (I)

Table 1 lists compounds having the structure represented by Formula (I).

**Table 1**

| | structure |
|---|---|
| Example 1 | 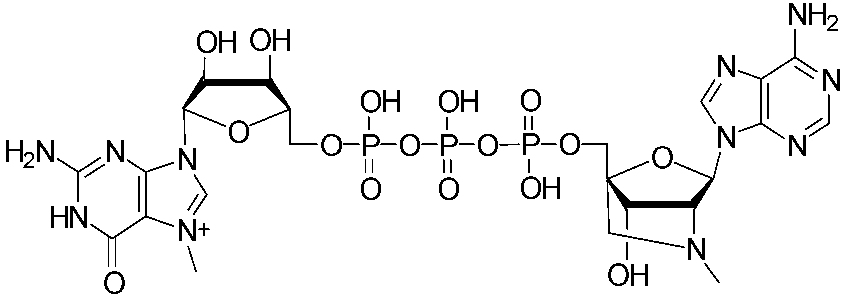 |
| Example 2 | 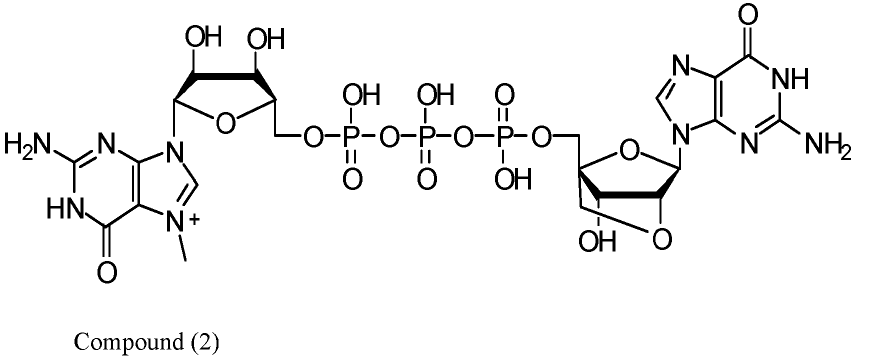 |
| Example 3 | 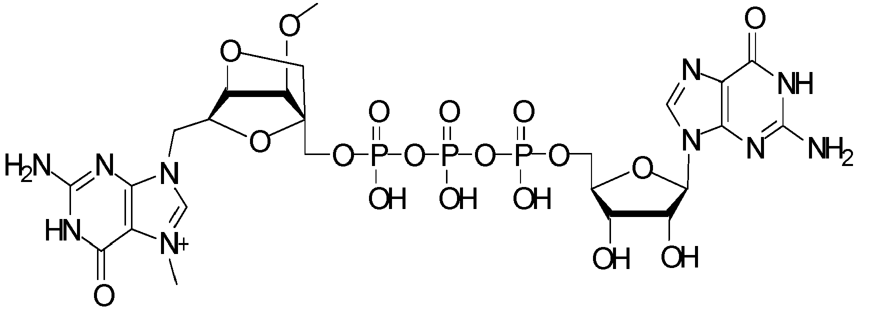 |
| Example 4 | 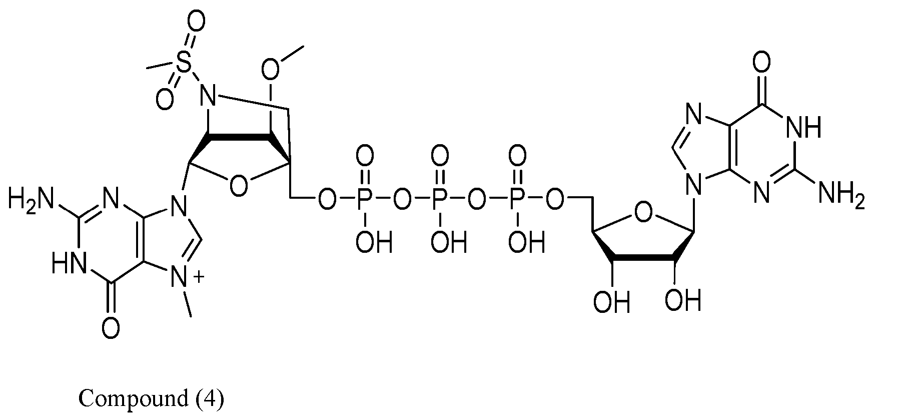 |
| Example 5 | 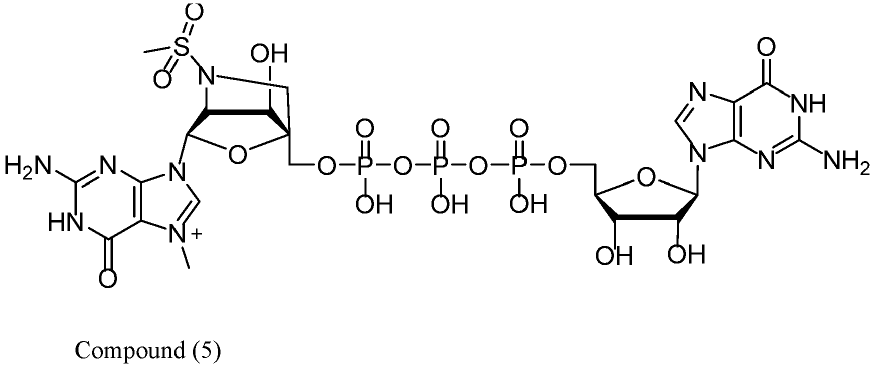 |
| Example 6 | 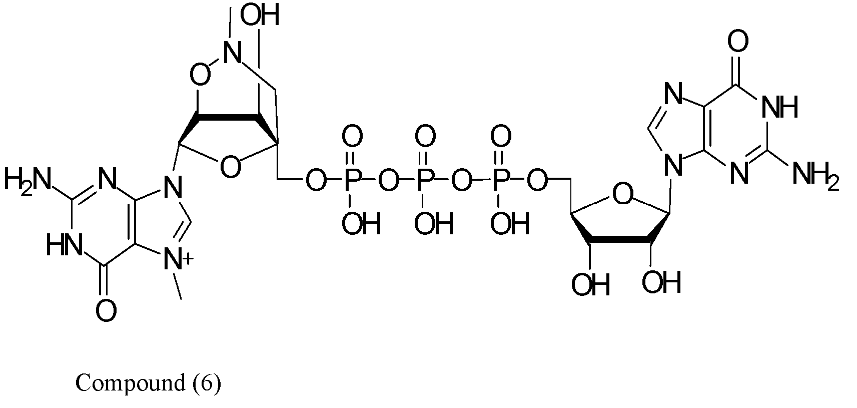 |
| Example 7 | 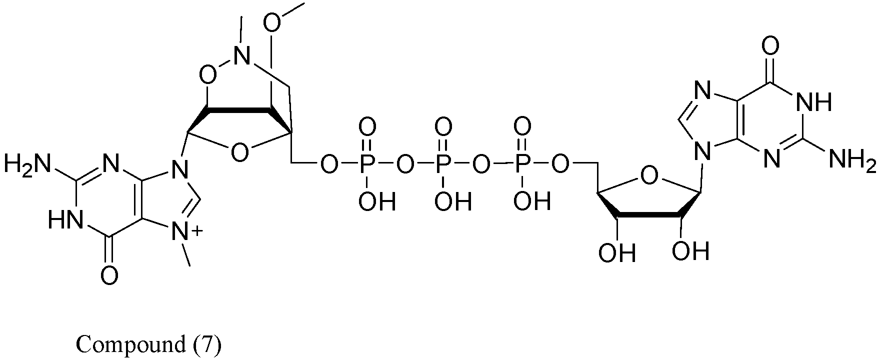 |
| Example 8 | 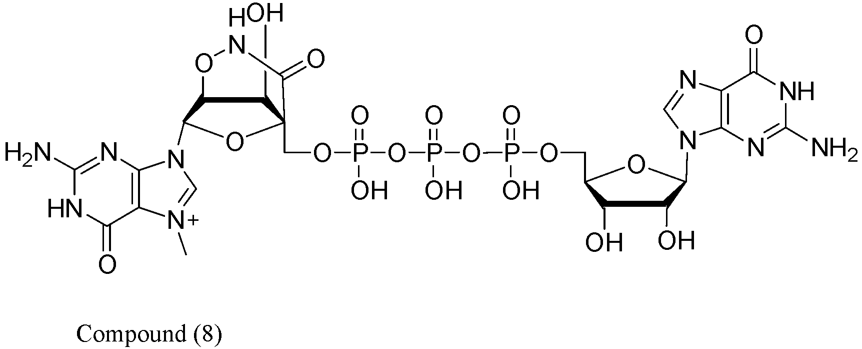 |
| Example 9 | 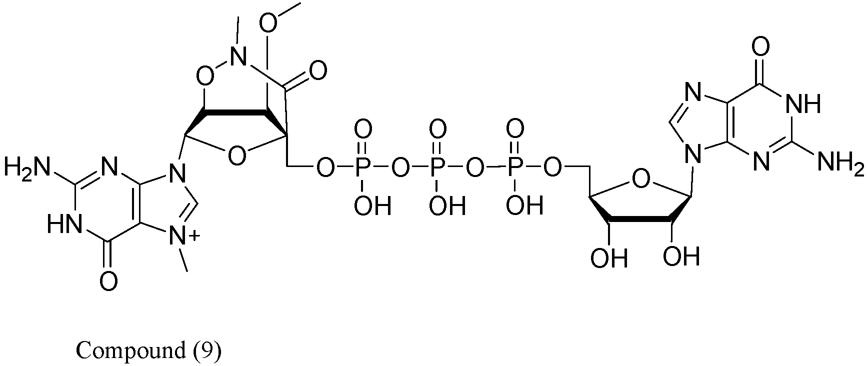 |
| Example 10 | 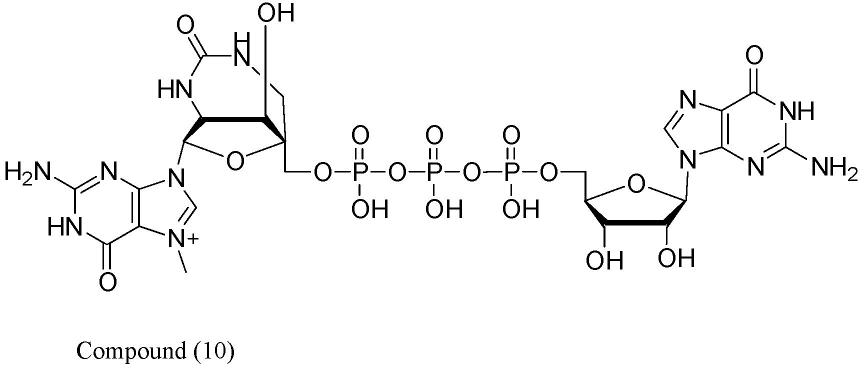 |
| Example 11 | 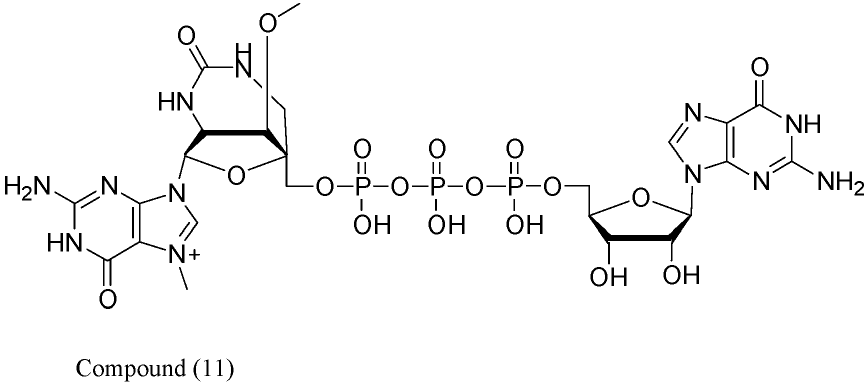 |
| Example 12 | 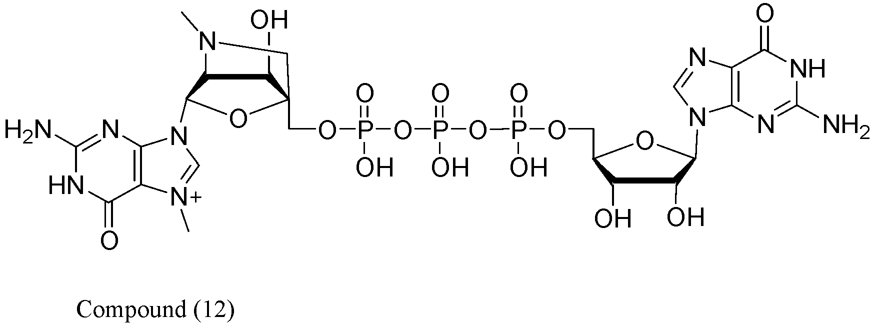 |
| Example 13 | 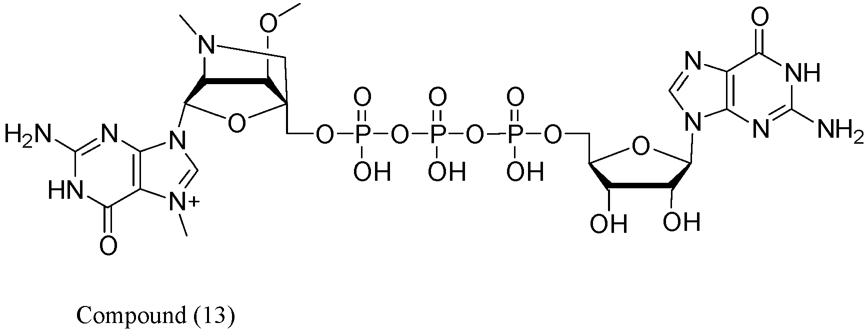 |
| Example 14 | 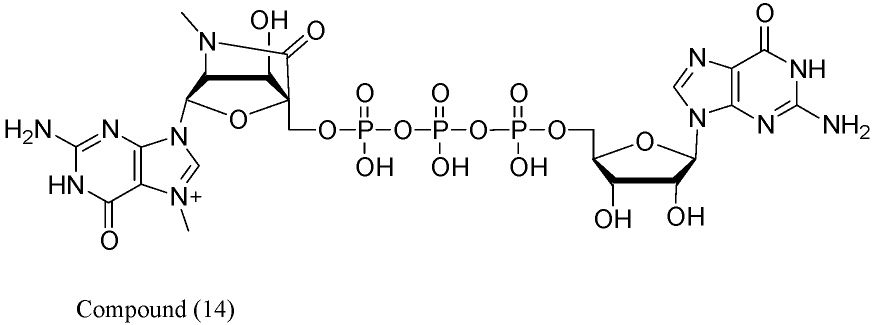 |
| Example 15 | 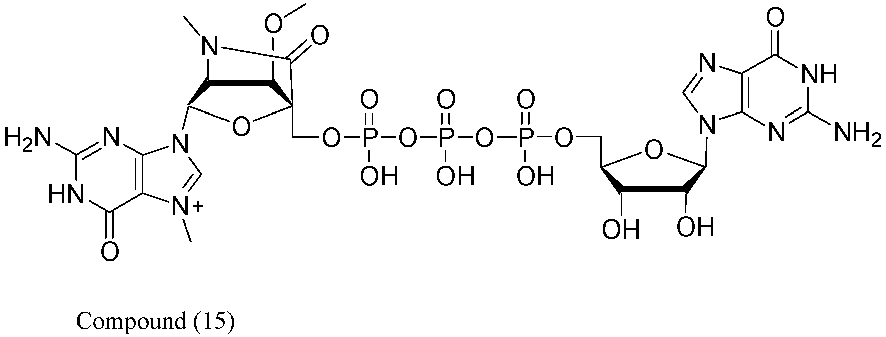 |
| Example 16 | 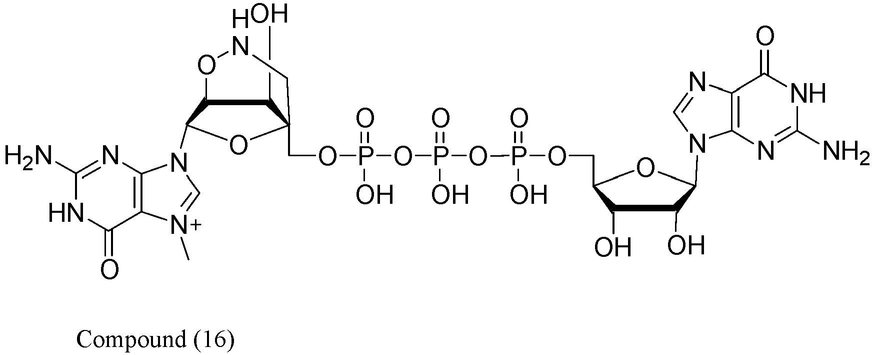 |
| Example 17 | 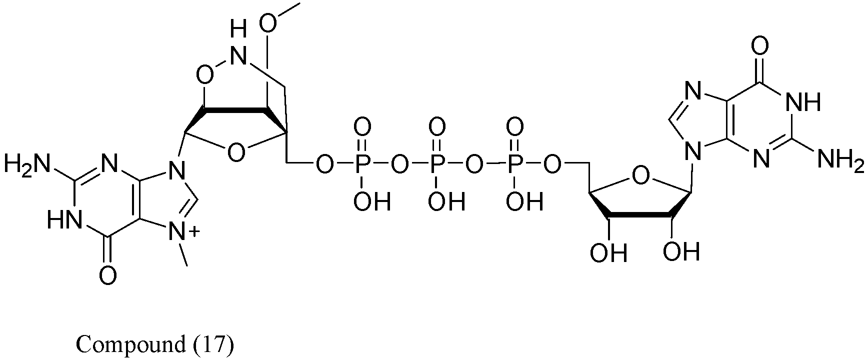 |
| Example 18 | 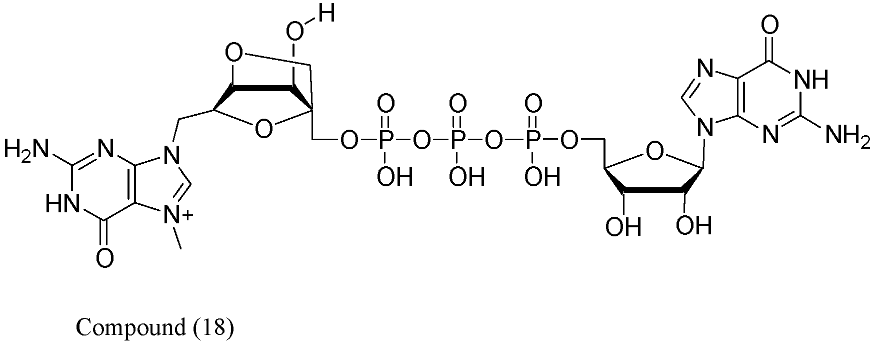 |
| Example 19 | 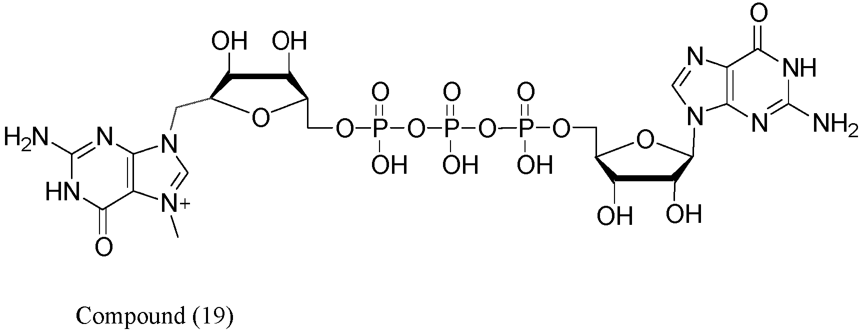 |
| Example 20 | 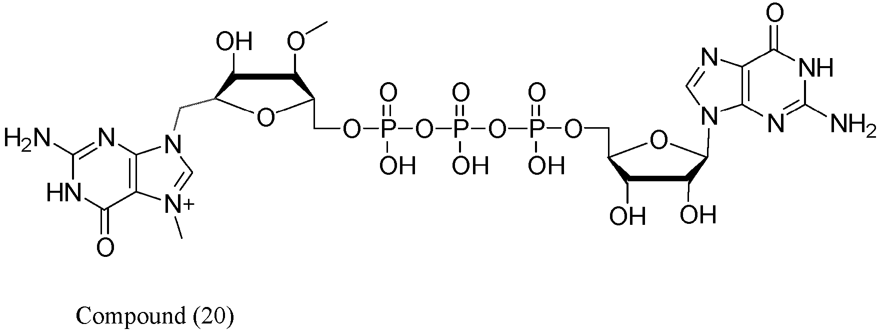 |
| Example 21 | 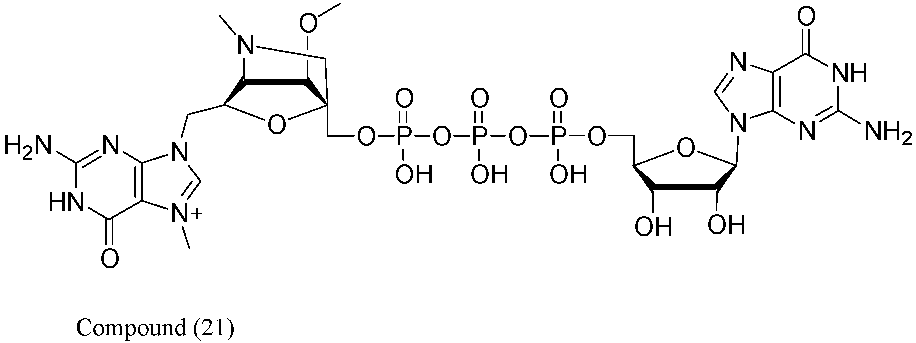 |
| Example 22 | 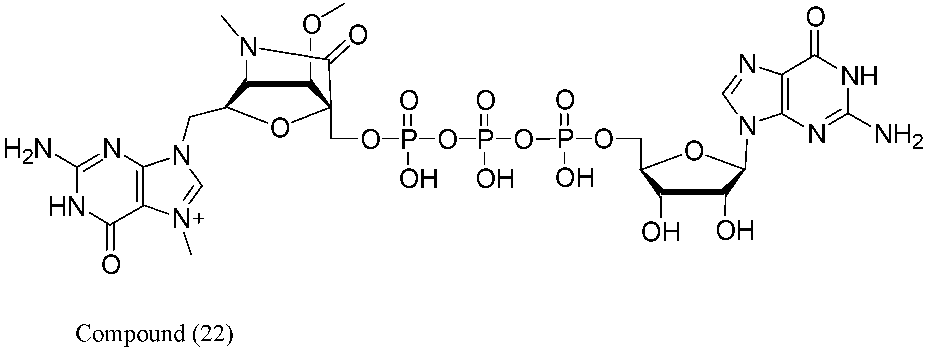 |
| Example 23 | 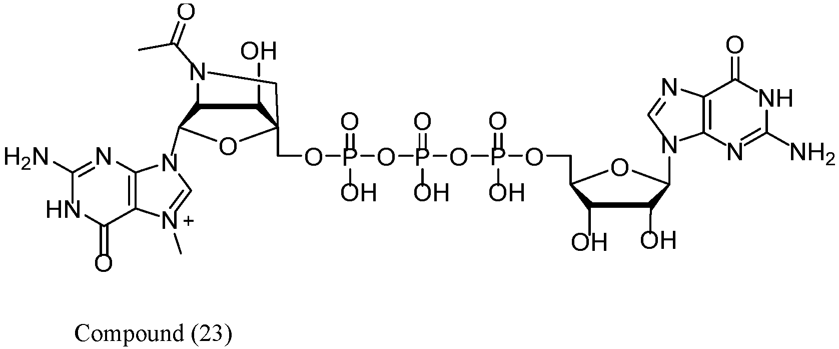 |
| Example 24 | 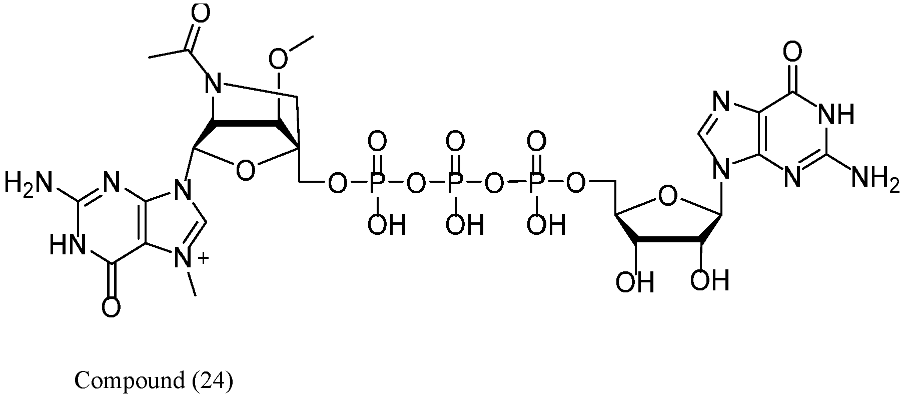 |

The compound having the structure represented by Formula (II)

Table 2 lists the compounds having the structure represented by Formula (II) of the disclosure.

**Table 2**

| | structure |
|---|---|
| Example 25 | 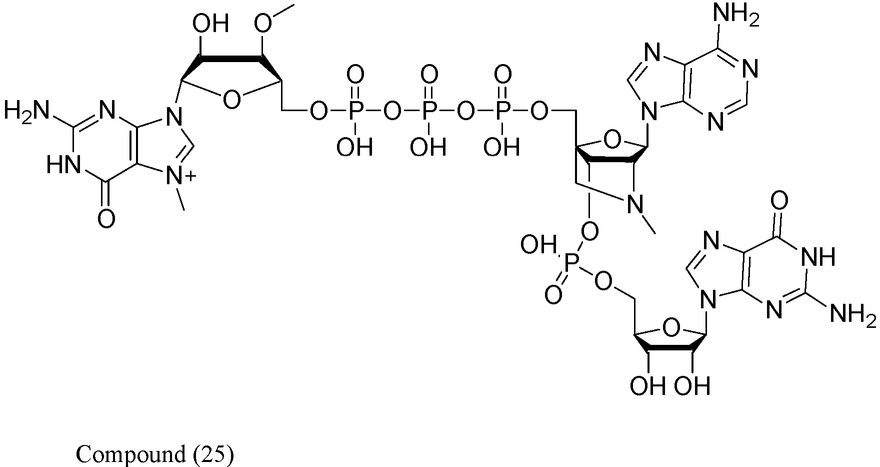 |
| Example 26 | 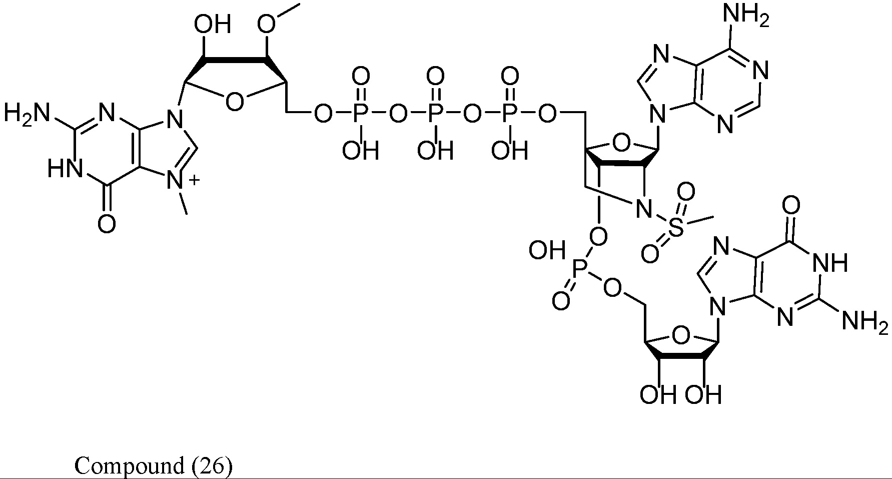 |
| Example 27 | 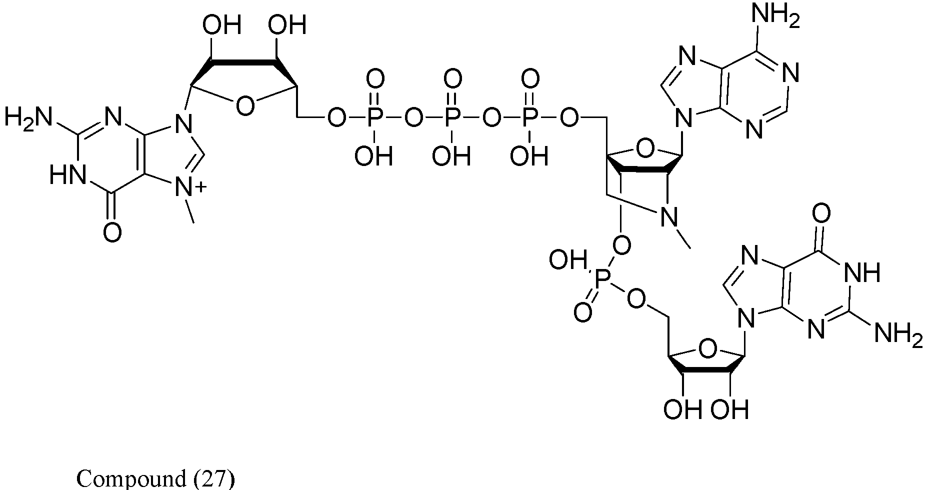 |
| Example 28 | 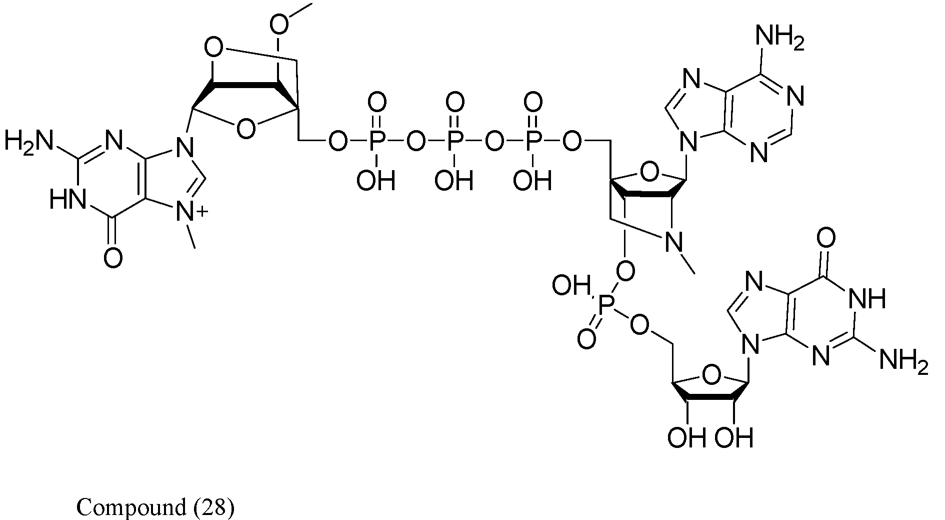 |
| Example 29 | 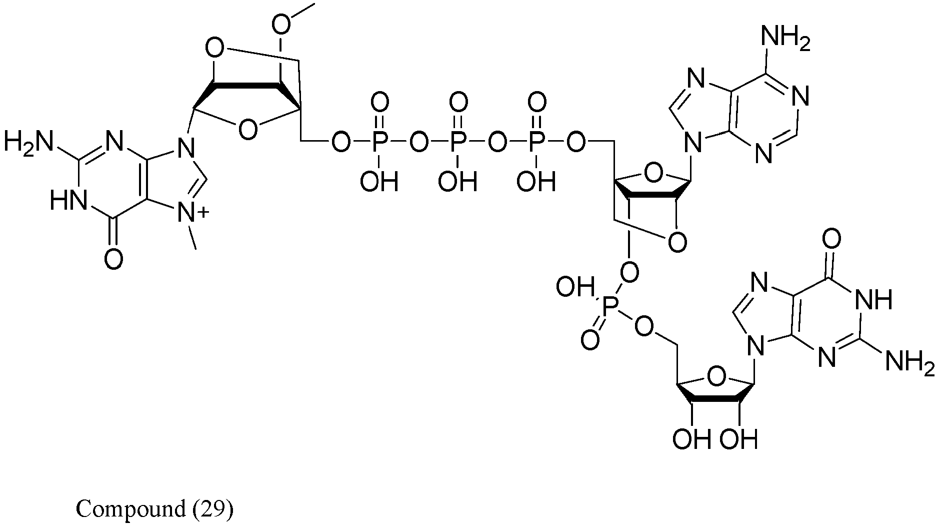 |
| Example 30 | 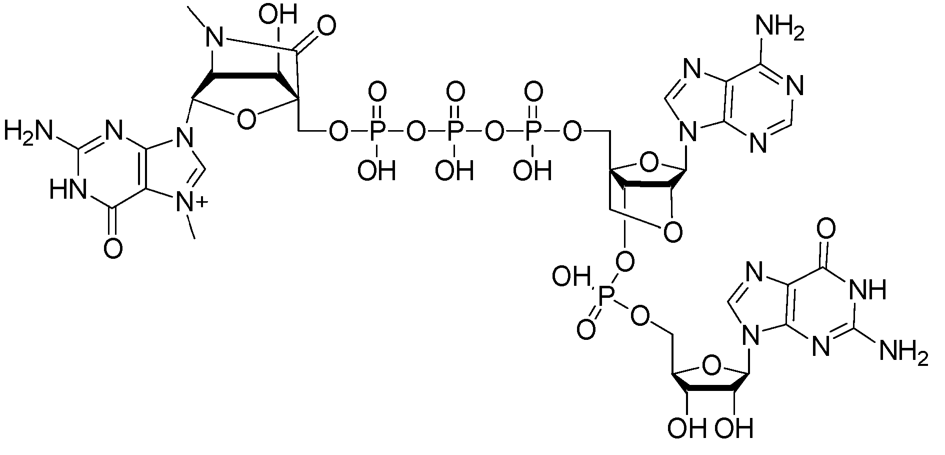 |
| | 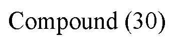 |
| Example 31 | 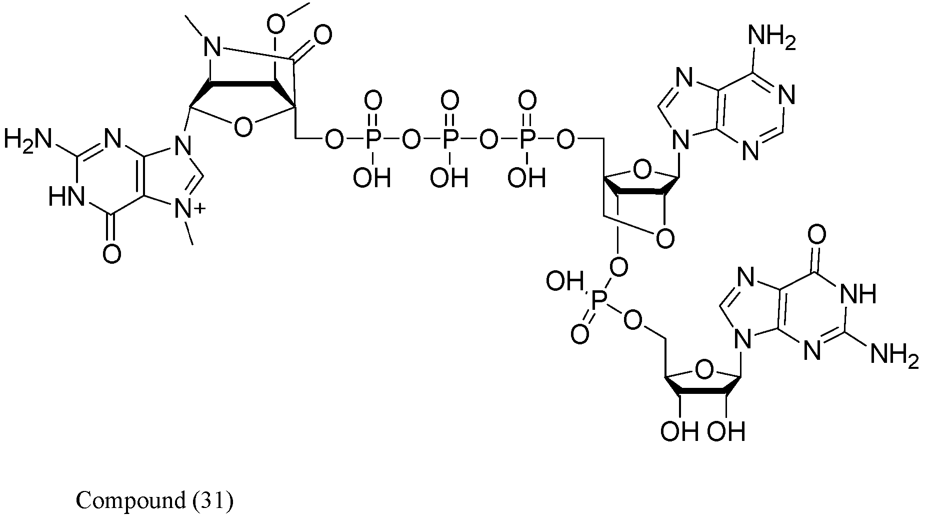 |
| Example 32 | 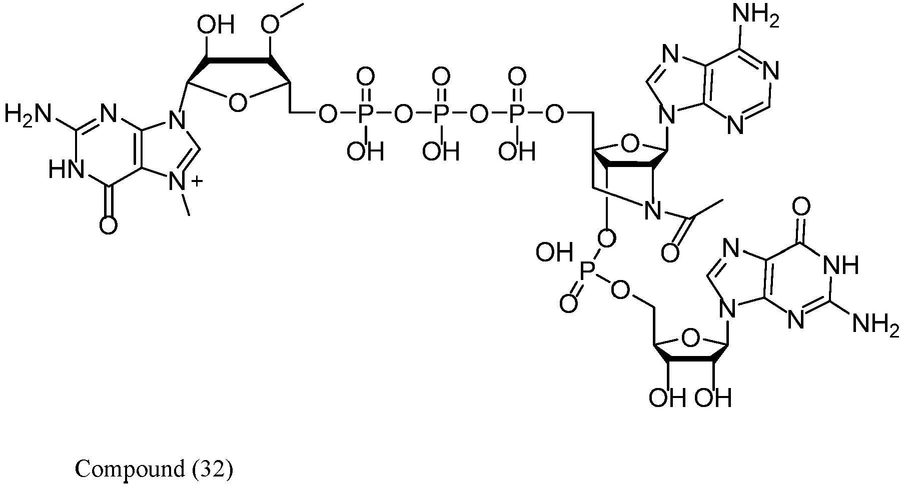 |

The compound having the structure represented by Formula (III)

Table 3 lists the compound having the structure represented by Formula (III) of the disclosure.

**Table 3**

| | structure |
|---|---|
| Example 33 | 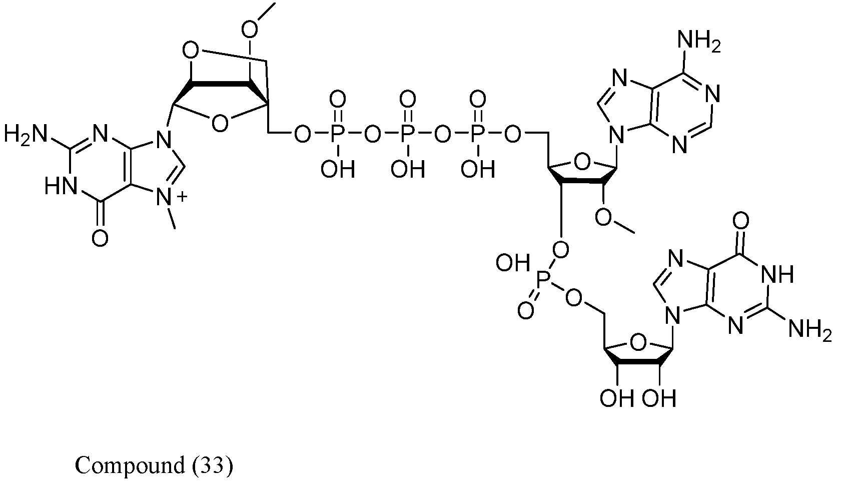 |
| Example 34 | 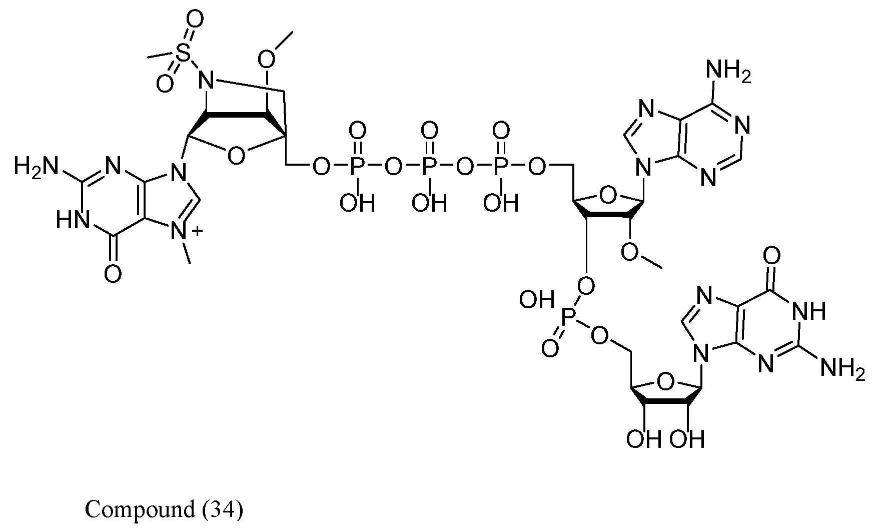 |
| Example 35 | 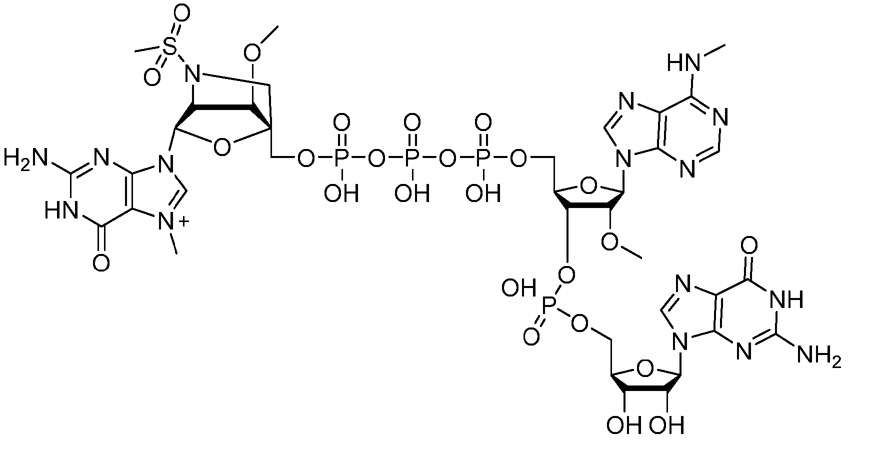 |
| | 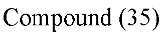 |
| Example 36 | 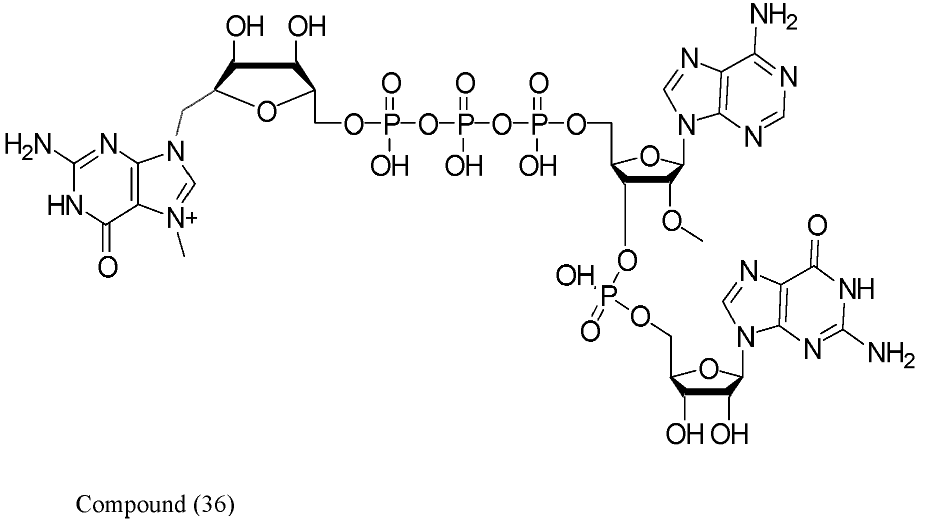 |
| Example 37 | 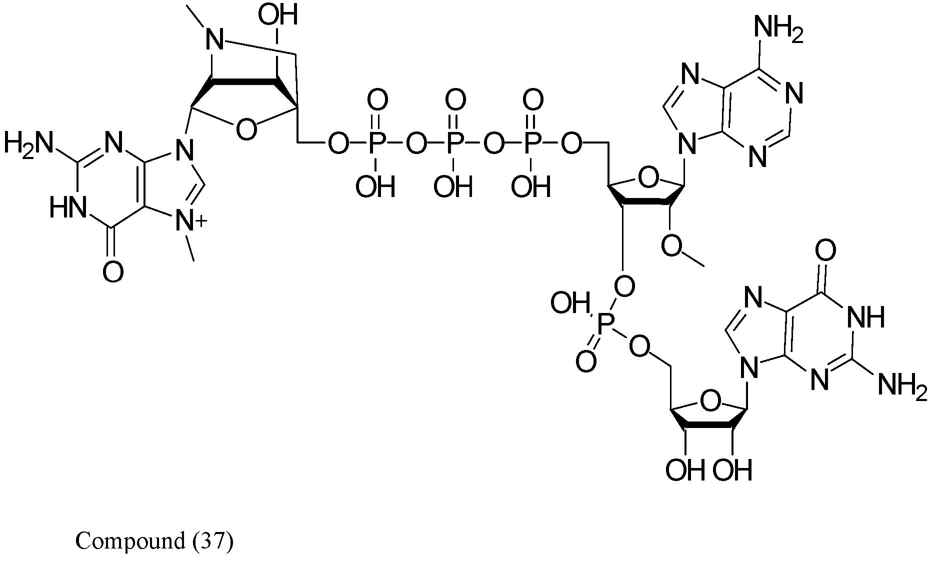 |
| Example 38 | 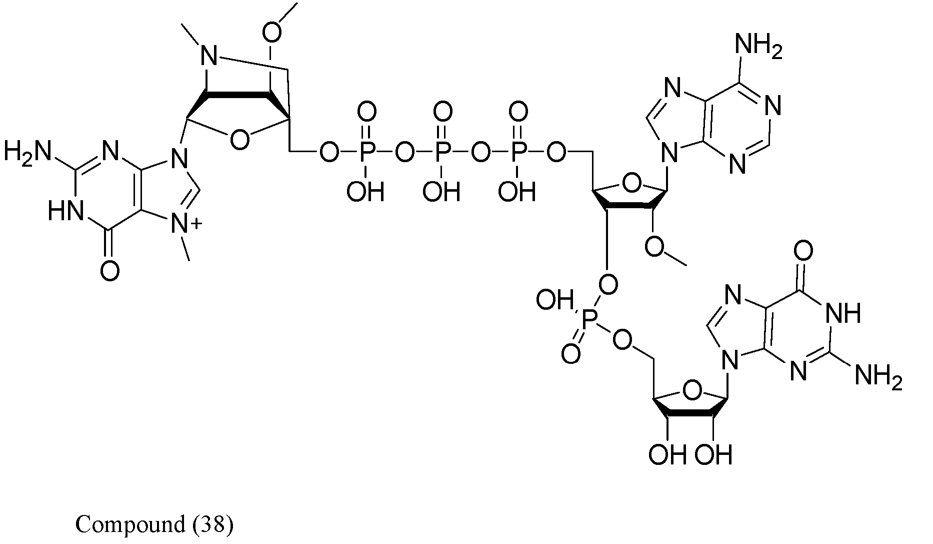 |
| Example 39 | 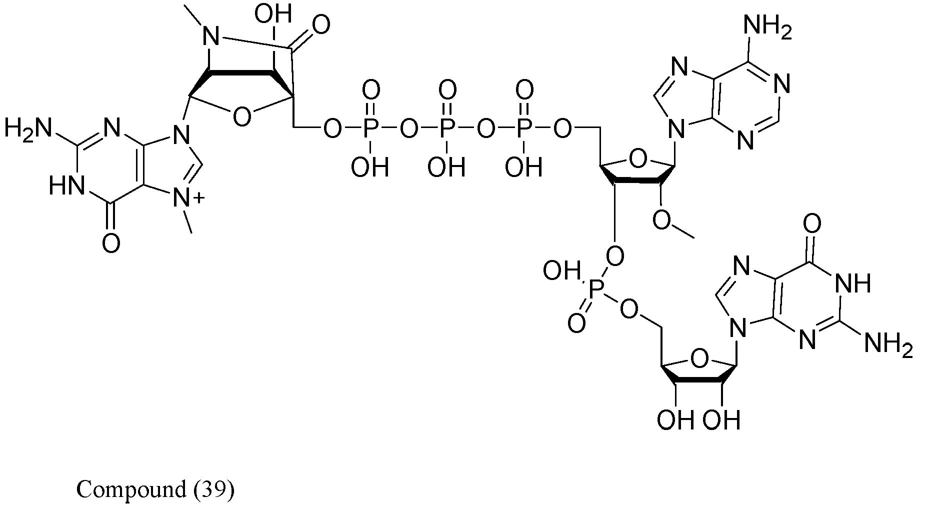 |
| Example 40 | 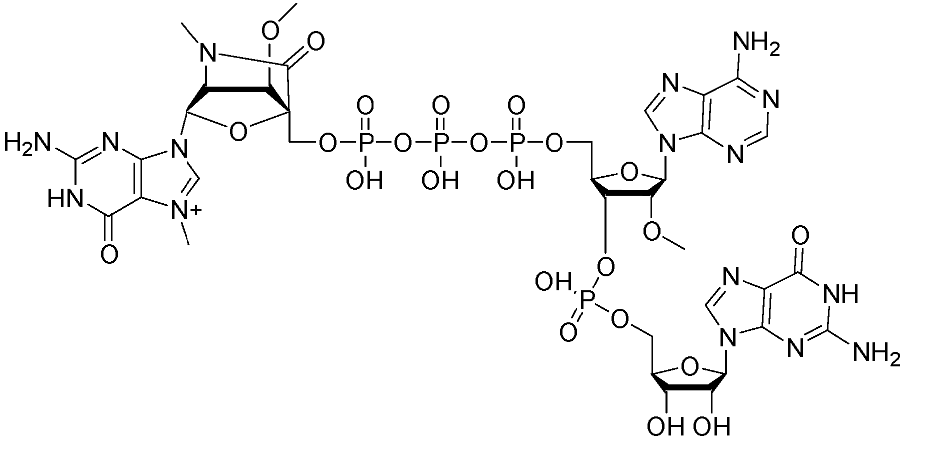 |
| | |
| Example 41 | 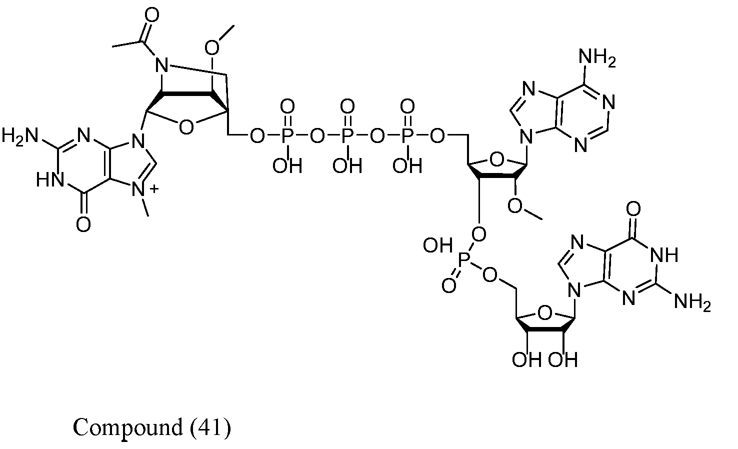 |
| Example 42 | 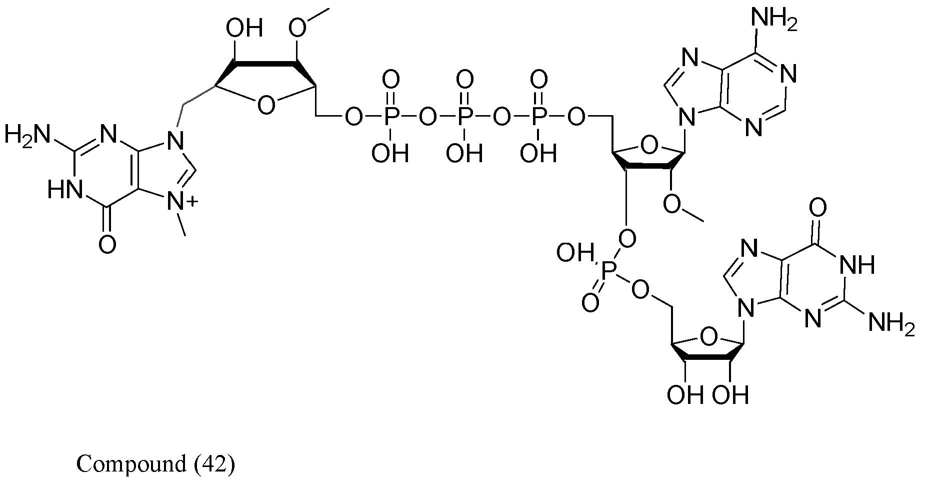 |
| Example 43 | 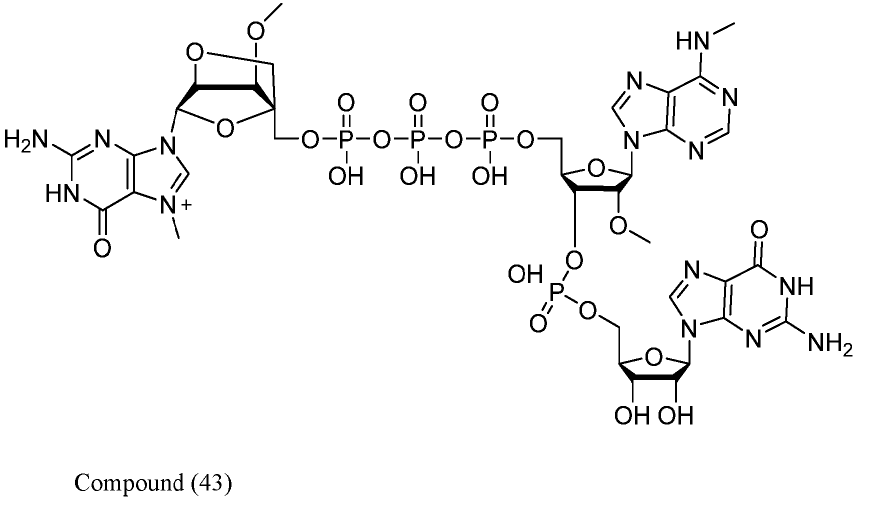 |

To further illustrate the preparation method of the compounds described in the disclosure, the preparation processes for the compounds described in Examples 1-4, 25, 26, and 33-35 are outlined below.

The structure and the measurement result of nuclear magnetic resonance spectrometry of the compounds used in Examples are listed in Table 4.

**Table 4**

| | structure | measurement result of nuclear magnetic resonance spectrometry |
|---|---|---|
| Compound (A) | (TEA⁺ is triethylammonium) | ¹H NMR (500 MHz, D₂O) *δ* 8.09 (s, 1H), 5.89 (d, *J* = 5.0 Hz, 1H), 4.76-4.74 (m, 1H), 4.55 (s, 1H), 4.30 (s, 1H), 4.19-4.15 (m, 2H). |
| Compound (D) | 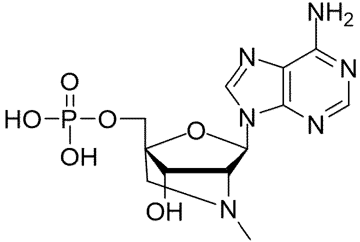 | ¹H NMR (400 MHz, D₂O) *δ* 8.26 (s, 1H), 8.19 (s, 1H), 6.41 (s, 1H), 4.18 (s, 1H), 4.55 (s, 1H), 4.26 (m, 2H), 3.87 (m, 1H), 3.70 (m, 1H), 3.25 (s, 3H). ³¹P NMR (400 MHz, D₂O) *δ* 0.25. |
| Compound (E) | 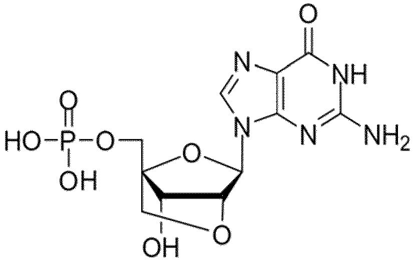 | ¹H NMR (400 MHz, D₂O) δ 7.81 (s, 1H), 5.73 (s, 1H), 4.44 (s, 1H), 4.37 (s, 1H), 3.99 (d, *J =* 6.1 Hz, 2H), 3.94 (d, *J* = 8.6 Hz, 1H), 3.83 (d, *J =* 8.6 Hz, 1H). |
| Compound (F) | 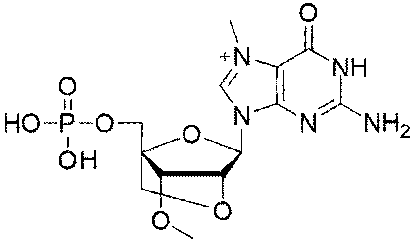 | ¹H NMR (500 MHz, D₂O) *δ* 6.03 (s, 1H), 4.92 (s, 1H), 4.26-4.25 (m, 1H), 4.23-4.22 (m, 1H), 4.17 (s, 3H), 4.15-4.01 (m, 2H), 3.43 (s, 3H). |
| Compound (H) | 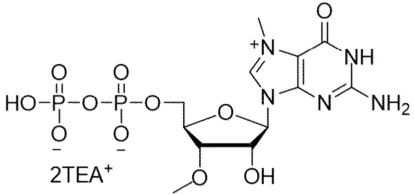 (TEA⁺ is triethylammonium) | ¹H NMR (400 MHz, D₂O) δ 5.90 (d, *J* = 4.4 Hz, 1H), 4.38 (s, 1H), 4.26-4.20 (m, 1H), 4.06-4.01 (m, 2H), 3.97 (s, 3H), 3.38 (s, 1H), 3.35 (s, 3H). |
| Compound (I) | 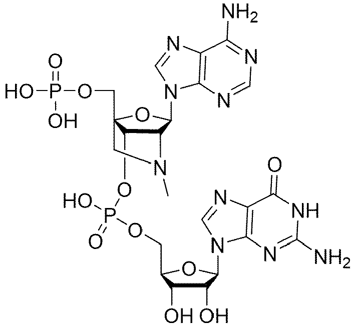 | ¹H NMR (400 MHz, DMSO-*d*₆) *δ* 8.05 (s, 1H), 7.93 (s, 1H), 7.62 (s, 1H), 6.09 (s, 1H), 5.48 (d, *J =* 4.0 Hz, 1H), 4.73 (d, *J =* 7.2 Hz, 1H), 4.32 (s, 1H), 4.28-4.11 (m, 5H), 3.99-3.96 (m, 1H), 3.42 (d, *J =* 10.8 Hz, 1H), 3.21 (t, *J =* 6.8 Hz, 2H), 2.88 (s, 3H). |
| Compound (J) | 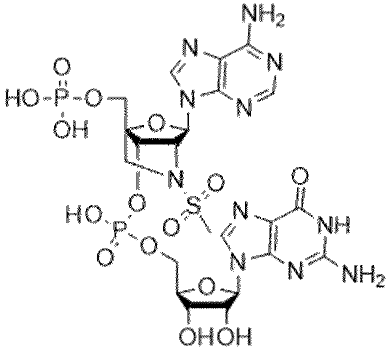 | ¹H NMR (400 MHz, D₂O) *δ* 8.06 (s, 1H), 7.96 (s, 1H), 7.62 (s, 1H), 6.01 (s, 1H), 5.50 (d, *J* = 4.4 Hz, 1H), 4.91 (s, 1H), 4.35 (s, 1H), 4.29-3.97 (m, 7H), 3.62-3.57 (m, 2H), 3.11 (s, 3H). |
| Compound (K) | 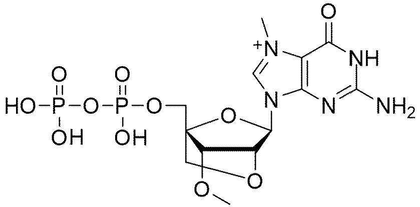 | ¹H NMR (500 MHz, D₂O) *δ* 5.97 (s, 1H), 4.87 (s, 2H), 4.37-4.29 (m, 2H), 4.08-4.00 (m, 6H), 3.67 (s, 3H), 3.38 (s, 3H). |
| Compound (L) | 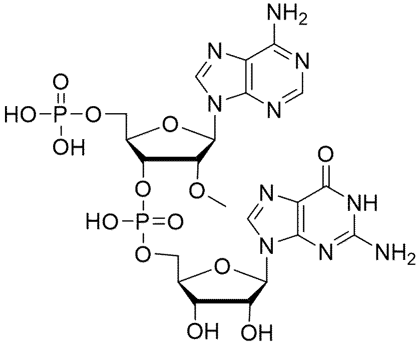 | ¹H NMR (400 MHz, D2O) δ 8.49 (s, 1H), 8.07 (s, 1H), 7.87 (s, 1H), 6.03 (d, *J =* 5.6 Hz, 1H), 5.75 (d, *J* = 5.6 Hz, 1H), 4.88 - 4.84 (m, 1H), 4.47 - 4.42 (m, 2H), 4.39-4.38 (m, 1H), 4.26 - 4.25 (m, 1H), 4.12 (t, *J* = 3.6 Hz, 2H), 3.96 - 3.88 (m, 2H), 3.38 (s, 3H), 3.09 (q, *J* = 7.2 Hz, 14H), 1.21 - 1.15 (m, 26H). ³¹P NMR (162 MHz, D₂O) δ 3.20 (s, 1H), -0.90 (s, 1H). |
| Compound (M) | 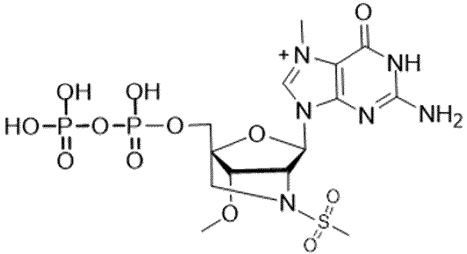 | ¹H NMR (500 MHz, D₂O) *δ* 9.03 (s, 1H), 6.10 (s, 1H), 4.99 (s, 1H), 4.42-4.38 (m, 2H), 4.10-4.08 (m, 5H), 3.63 (ABX, *J* = 10.0 Hz, 9.5 Hz, 2H), 3.41 (s, 3H). |
| Compound (N) | 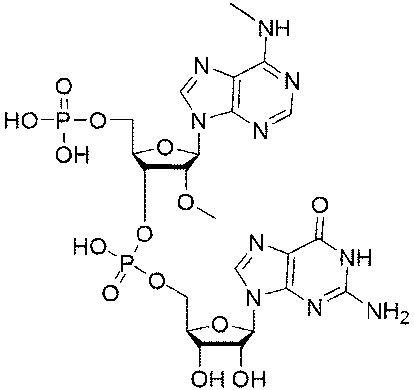 | ¹H NMR (400 MHz, D₂O) *δ* 8.40 (s, 1H), 7.98 (s, 1H), 7.80 (s, 1H), 5.96 (d, *J =* 5.6 Hz, 1H), 5.66 (d, *J* = 5.6 Hz, 1H), 4.78-4.58 (m, 2H), 4.43-3.85 (m, 8H), 3.34 (s, 3H), 2.88 (s, 3H), 3.06 (q, *J =* 7.2 Hz, 18H), 1.14 (t, *J* = 7.2 Hz, 27H). ³¹P NMR (162 MHz, D₂O) *δ* 3.50 (s, 1P), -0.89 (s, 1P). |
| Compound (O) | 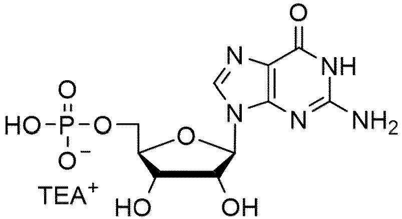 (TEA⁺ is triethylammonium) | ¹H NMR (500 MHz, D₂O) *δ* 8.13 (s, 1H), 5.89 (d, *J =* 5.5 Hz, 1H), 4.74 (t, *J* = 5.5 Hz, 6.5 Hz, 2H), 4.46-4.44 (m, 1H), 4.29 (s, 1H), 4.00 (s, 2H). |

### Example 1

Compound (A) (2.30 mmol) was added to a stirred solution of 5 mL water, and slowly add acetic acid dropwise to adjust the solution's pH to 4.0. Next, dimethyl sulfate (23.0 mmol) was slowly added. Next, the result was stirred at room temperature for 3 hours. As methylation proceeds, the pH of the mixture decreased to approximately 2.0. Next, a sodium hydroxide aqueous solution (with a concentration of 1M) was added to adjust the pH value of the mixture to 4.0. After stirring for 5 hours at room temperature, the result was extracted by water (10ml) and washed by dichloromethane (30ml), and then the water phase was collected. Next, the result was purified by an ion-exchange resin (DEAE) with the extraction solvent being a 0.1M triethylammonium bicarbonate (TEAB) aqueous solution, obtaining Compound (B) (white solid).

The synthesis pathway of the above reaction was as follows:

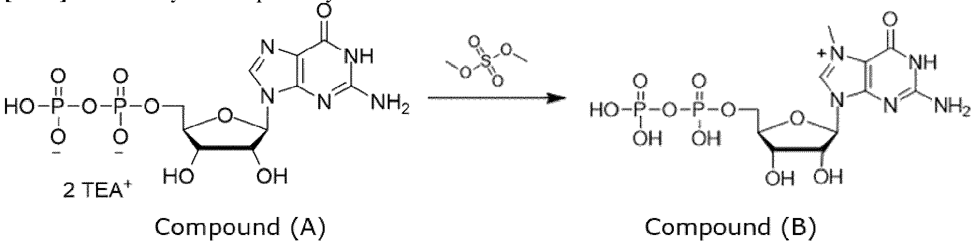

The measurement results of nuclear magnetic resonance spectrometry of Compound (B) are shown below: ¹H NMR (400 MHz, D₂O) *δ* 5.98 (d, *J* = 7.2 Hz, 1H), 4.55 (t, *J* = 4.0 Hz, 1H), 4.49 (t, *J* = 5.4 Hz, 1H), 4.27 (m, 1H), 4.21 (m, 1H), 4.02 (s, 3H), 3.11 (q, *J =* 7.2 Hz, 19H), 1.19 (t, *J* = 7.2 Hz, 29H)

Compound (B) (0.44 mmol), triphenylphosphine (PPh₃) (2.18 mmol), imidazole (4.36 mmol), and 2-(pyridin-2-yldisulfanyl)pyridine (2.18 mmol) were dissolved in dimethylformamide (DMF) (2ml). Under a nitrogen atmosphere, the mixture was stirred at room temperature for 6 hours. Next, the result was dropwisely added into acetone (250ml) at -20°C to form a white solid. The precipitate was collected by centrifugation, washed five times with 30 mL of acetone, and dried under vacuum at room temperature, obtaining Compound (C) (white solid).

The synthesis pathway of the above reaction was as follows:

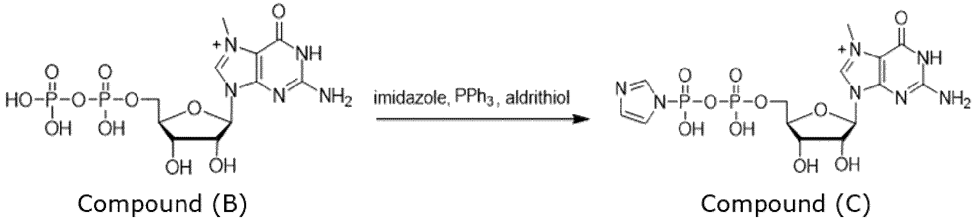

The measurement results of nuclear magnetic resonance spectrometry of Compound (C) are shown below: 1H NMR (400 MHz, MeOD) δ 8.02 (s, 1H), 7.44 (d, J = 1.6 Hz, 1H), 6.99 (d, J = 1.6 Hz, 1H), 6.02 (d, J = 2.8 Hz, 1H), 5.51 (s, 1H), 4.56 (m, 1H), 4.46 (m, 1H), 4.30-4.16 (m, 7H).

At room temperature, Compound (C) (0.06 mmol), Compound (D) (0.05 mmol), and ZnCl₂ (0.07 mmol) were added into dimethylformamide (DMF) (1ml). After stirring under nitrogen atmosphere for 48 hours, ethylenediaminetetraacetic acid (EDTA) aqueous solution (5 ml, with a concentration of 1M) was added into the result. Next, the result was purified by an ion-exchange resin (DEAE) with the extraction solvent being a 0.1M triethylammonium bicarbonate (TEAB) aqueous solution, obtaining Compound (1) (white solid).

The synthesis pathway of the above reaction was as follows:

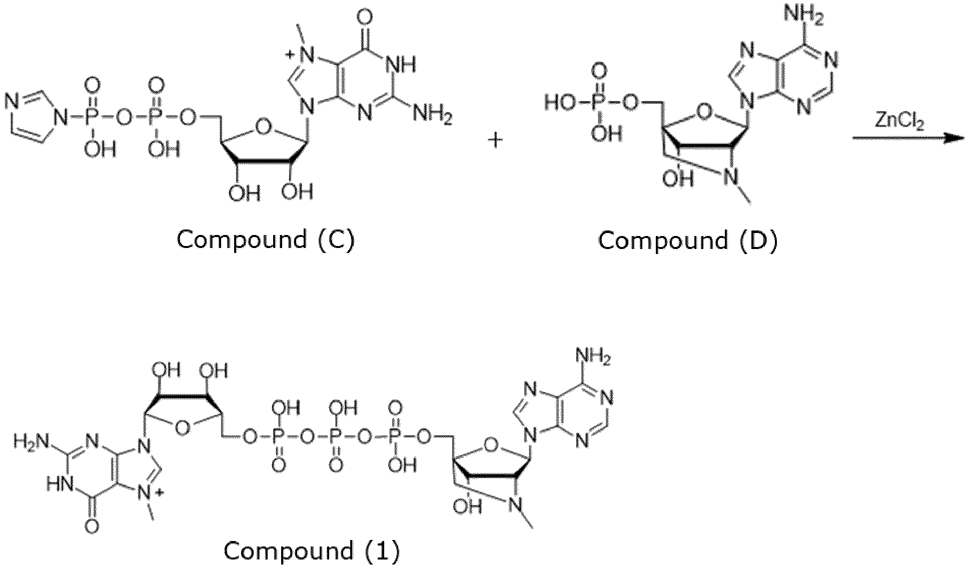

Next, the measurement results of nuclear magnetic resonance spectrometry of Compound (1) are shown below: ¹H NMR (400 MHz, D₂O) δ 7.87(s, 1H), 5.86 (s, 1H), 5.73 (s, 1H), 4.55 (s, 1H), 4.45 (d, *J =* 9.0Hz, 1H), 4.37-4.43(m, 2H), 4.05(s, 3H), 4.04 (d, *J =* 8.5 Hz, 1H), 3.21 (*J* = 7.3Hz, 18H), 1.28 (t, *J =* 7.5Hz, 30H). Next, Compound (1) was analyzed using liquid chromatography-mass spectrometry (LC-MS), and M/Z results show: [M]⁺ = 812(C₂₃H₃₃N₁₁O₁₆P₃⁺).

### Example 2

At room temperature, Compound (C) (0.06 mmol), Compound (E) (0.05 mmol), and ZnCl₂(0.07 mmol) were added into dimethylformamide (DMF) (1ml). After stirring under nitrogen atmosphere for 48 hours, ethylenediaminetetraacetic acid (EDTA) aqueous solution (5 ml, with a concentration of 1M) was added into the result. Next, the result was purified by an ion-exchange resin (DEAE) with the extraction solvent being a 0.1M triethylammonium bicarbonate (TEAB) aqueous solution, obtaining Compound (2) (white solid).

The synthesis pathway of the above reaction was as follows:

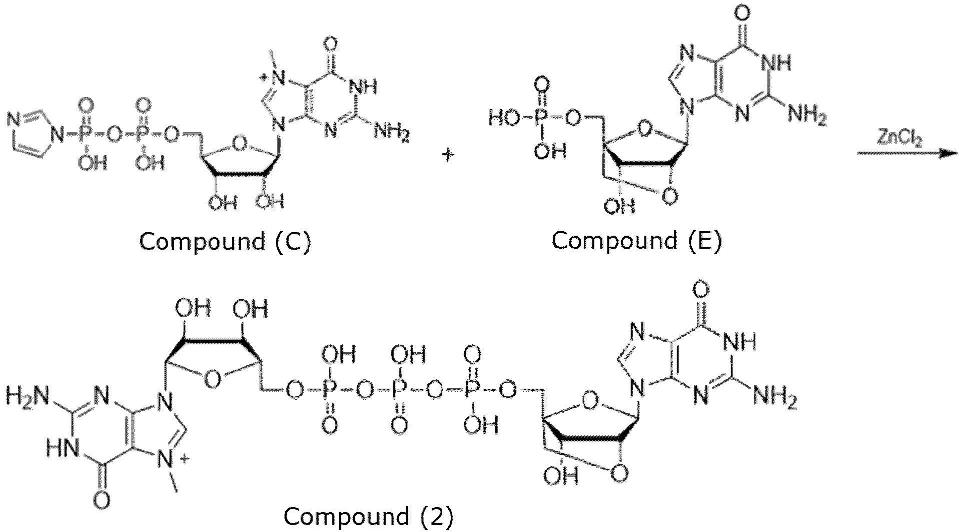

Next, the measurement results of nuclear magnetic resonance spectrometry of Compound (2) are shown below: ¹H NMR (400 MHz, D₂O) δ 7.87(s, 1H), 5.86 (s, 1H), 5.73 (s, 1H), 4.55 (s, 1H), 4.45 (d, *J* = 9.0Hz, 1H), 4.37-4.43(m, 2H), 4.05(s, 3H), 4.04 (d, *J =* 8.5 Hz, 1H), 3.21 (*J* = 7.3Hz, 18H), 1.28 (t, *J* = 7.5Hz, 30H). Compound (2) was analyzed using liquid chromatography-mass spectrometry (LC-MS), and M/Z results show: [M]⁻ = 815 (C₂₂H₃₀N₁₀O1₈P₃⁺).

### Example 3

At room temperature, Compound (F) (0.06 mmol), Compound (G) (0.05 mmol), and ZnCl₂(0.07 mmol) were added into dimethylformamide (DMF) (1ml). After stirring under nitrogen atmosphere for 48 hours, ethylenediaminetetraacetic acid (EDTA) aqueous solution (5 ml, with a concentration of 1M) was added into the result. Next, the result was purified by an ion-exchange resin (DEAE) with the extraction solvent being a 0.1M triethylammonium bicarbonate (TEAB) aqueous solution, obtaining Compound (3) (white solid).

The synthesis pathway of the above reaction was as follows:

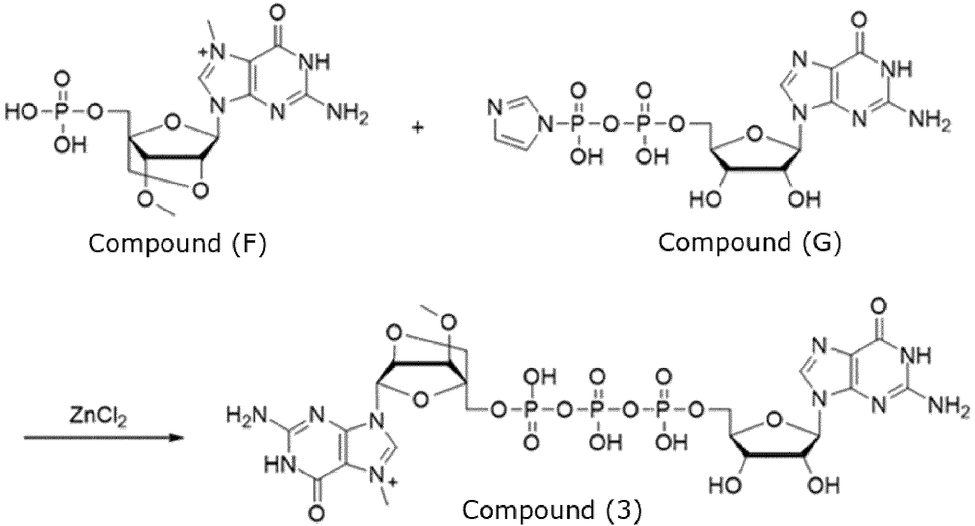

Next, the measurement results of nuclear magnetic resonance spectrometry of Compound (3) are shown below: ¹H NMR (500 MHz, D₂O) *δ* 7.97 (s, 1H), 5.71-5.65 (m, 1H), 5.64 (s, 1H), 4.79 (s, 2H), 4.59 (s, 1H), 4.41-4.38 (m, 3H), 4.30 (s, 1H), 4.24 (s, 2H), 3.38 (s, 3H), 3.11 (q, *J =* 7.2 Hz, 17H), 1.19 (t, *J* = 7.2 Hz, 27H). Compound (3) was analyzed using liquid chromatography-mass spectrometry (LC-MS), and M/Z results show: [M+H]⁺ = 829(C₂₃H₃₂N₁₀O₁₈P₃⁺).

### Example 4

At room temperature, Compound (M) (0.06 mmol) was dissolved in a mixed solution of water (0.03 mL) and dimethyl sulfoxide (DMSO) (0.28 mL). Then, 1-(3-dimethylaminopropyl)-3-ethylcarbodiimide hydrochloride (EDC·HCl) (0.10 mmol) was added, followed by the addition of imidazole (0.19 mmol). The result was stirred at room temperature for 4 hours, after which an aqueous solution of magnesium chloride (MgCl₂, concentration 3.15 M, 0.03 mL) was added, followed by the addition of Compound (O) (0.04 mmol). The mixture was stirred at room temperature for 16 hours, and then the result was mixed with an aqueous solution of ethylenediaminetetraacetic acid (EDTA, 5 mL, concentration 1 M), wherein the amount of EDTA was three times that of magnesium chloride. Next, the mixture was purified using ion-exchange resin (DEAE) with triethylammonium bicarbonate (TEAB) aqueous solution (concentration 0.1 M) as the eluent. After concentration and drying, the product was redissolved in water, and acetone and sodium perchlorate were added, leading to the formation of a precipitate. The precipitate was centrifuged, washed with acetone, dissolved in water, and freeze-dried to yield Compound (4).

The synthesis pathway of the above reaction was as follows:

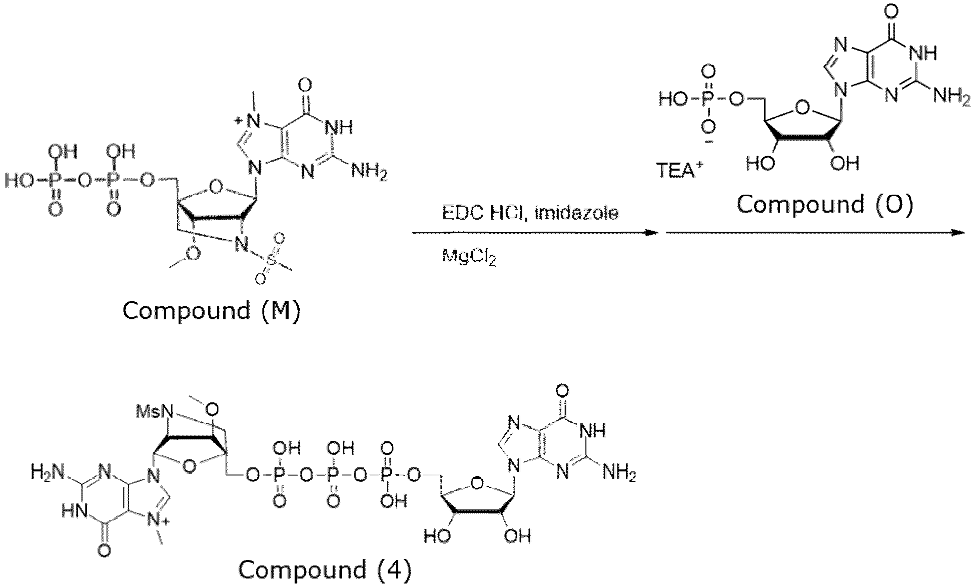

Next, the measurement results of nuclear magnetic resonance spectrometry of Compound (4) are shown below: ¹H NMR (400 MHz, D₂O) δ 8.03 (s, 1H), 5.80-5.77 (2H), 4.89 (s, 1H), 4.68 (t, J = 6.0 Hz, 5.0 Hz, 1H), 4.48-4.45 (m, 2H), 4.40-4.35 (m, 2H), 4.32-4.26 (m, 2H), 4.09-4.07 (m, 4H), 3.62 (dd, J = 9.0 Hz, 21.0 Hz, 2H), 3.43 (s, 3H). Next, Compound (42) was analyzed using liquid chromatography-mass spectrometry (LC-MS), and M/Z results show: [M+H]⁺ = 906.58 (C₂₄H₃₅N₁₁O₁₉P₃S⁺).

### Example 25

At room temperature, Compound (H) (0.06 mmol) was dissolved in a mixed solution of water (0.03 mL) and dimethyl sulfoxide (DMSO) (0.28 mL). Then, 1-(3-dimethylaminopropyl)-3-ethylcarbodiimide hydrochloride (EDC·HCl) (0.10 mmol) was added, followed by the addition of imidazole (0.19 mmol). The result was stirred at room temperature for 4 hours, after which an aqueous solution of magnesium chloride (MgCl₂, concentration 3.15 M, 0.03 mL) was added, followed by the addition of Compound (I) (0.04 mmol). The mixture was stirred at room temperature for 16 hours, and then the result was mixed with an aqueous solution of ethylenediaminetetraacetic acid (EDTA, 5 mL, concentration 1 M), wherein the amount of EDTA was three times that of magnesium chloride. Next, the mixture was purified using ion-exchange resin (DEAE) with triethylammonium bicarbonate (TEAB) aqueous solution (concentration 0.1 M) as the eluent. After concentration and drying, the product was redissolved in water, and acetone and sodium perchlorate were added, leading to the formation of a precipitate. The precipitate was centrifuged, washed with acetone, dissolved in water, and freeze-dried to yield Compound (25).

The synthesis pathway of the above reaction was as follows:

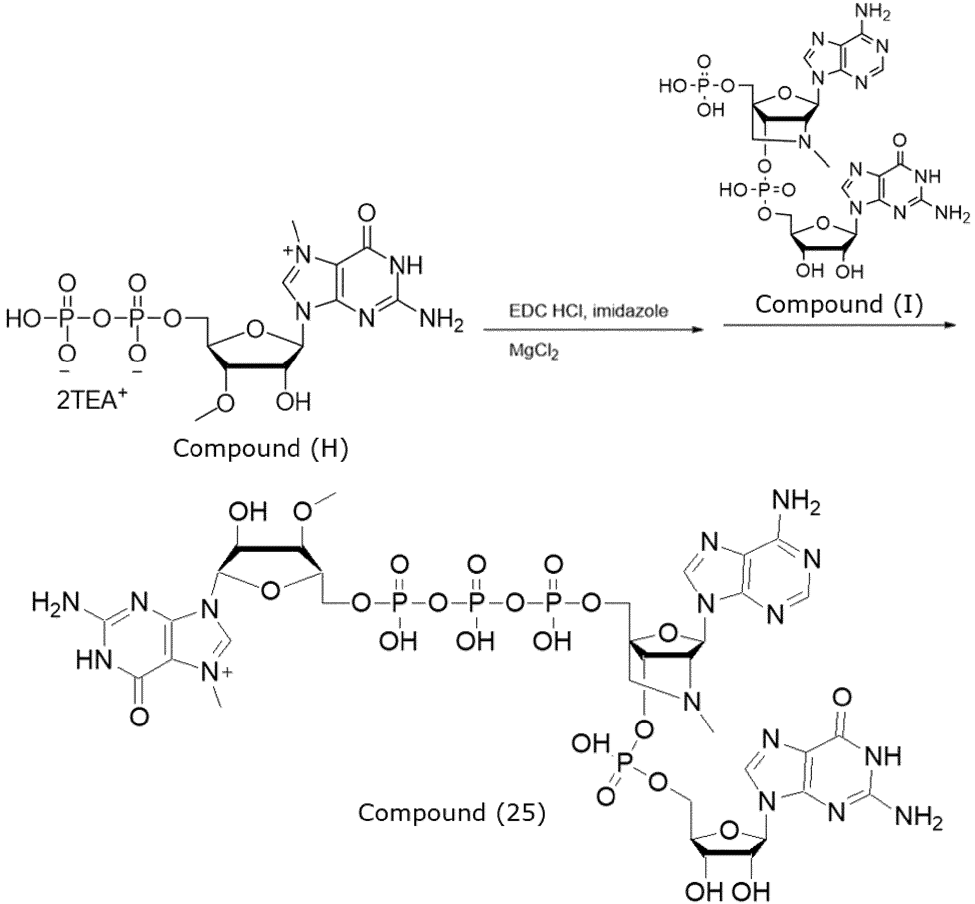

Next, the measurement results of nuclear magnetic resonance spectrometry of Compound (25) are shown below: ¹H NMR (500 MHz, D₂O) δ 9.00 (s, 1H), 8.29 (s, 1H), 8.19 (s, 1H), 8.00 (s, 1H), 6.43 (s, 1H), 5.71 (s, 1H), 5.54 (s, 1H), 4.88 (s, 2H), 4.58 (s, 1H), 4.44 (br, 1H), 4.34-4.20 (m, 5H), 4.14-3.99 (m, 5H), 3.85 (s, 5H), 3.30 (s, 3H), 3.20 (s, 3H). Next, Compound (28) was analyzed using liquid chromatography-mass spectrometry (LC-MS), and M/Z results show: [M+H]⁺ = 1171.7 (C₃₄H₄₇N₁₆O₂₄P₄⁺).

### Example 26

At room temperature, Compound (H) (0.06 mmol) was dissolved in a mixed solution of water (0.03 mL) and dimethyl sulfoxide (DMSO) (0.28 mL). Then, 1-(3-dimethylaminopropyl)-3-ethylcarbodiimide hydrochloride (EDC·HCl) (0.10 mmol) was added, followed by the addition of imidazole (0.19 mmol). The result was stirred at room temperature for 4 hours, after which an aqueous solution of magnesium chloride (MgCl₂, concentration 3.15 M, 0.03 mL) was added, followed by the addition of Compound (J) (0.04 mmol). The mixture was stirred at room temperature for 16 hours, and then the result was mixed with an aqueous solution of ethylenediaminetetraacetic acid (EDTA, 5 mL, concentration 1 M), wherein the amount of EDTA was three times that of magnesium chloride. Next, the mixture was purified using ion-exchange resin (DEAE) with triethylammonium bicarbonate (TEAB) aqueous solution (concentration 0.1 M) as the eluent. After concentration and drying, the product was redissolved in water, and acetone and sodium perchlorate were added, leading to the formation of a precipitate. The precipitate was centrifuged, washed with acetone, dissolved in water, and freeze-dried to yield Compound (26).

The synthesis pathway of the above reaction was as follows:

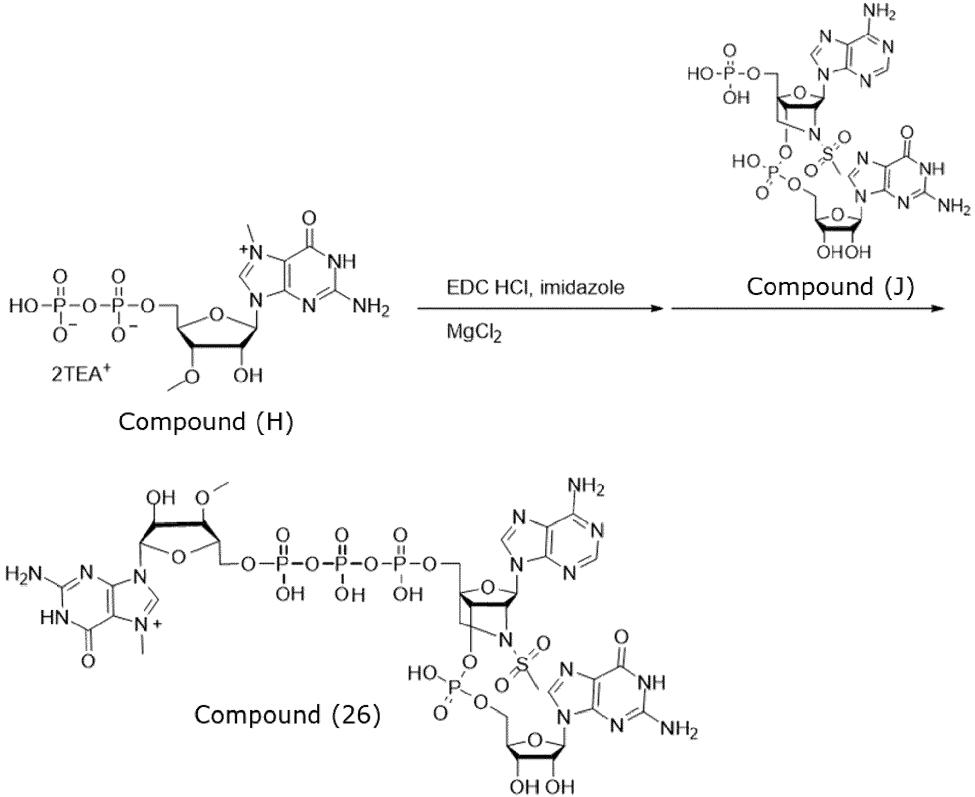

Next, the measurement results of nuclear magnetic resonance spectrometry of Compound (26) are shown below: ¹H NMR (500 MHz, D₂O) δ 9.13 (s, 1H), 8.33 (d, *J* = 12.5 Hz, 2H), 8.08 (s, 1H), 6.17 (s, 1H), 5.80 (s, 1H), 5.66 (s, 1H), 4.94 (s, 1H), 4.71-4.68 (m, 3H), 4.53-4.47 (m, 3H), 4.40-4.33 (m, 5H), 4.26 (s, 1H), 4.19-4.17 (m, 3H), 3.99 (s, 3H), 3.59-3.54 (m, 5H), 3.43 (s, 3H). Next, Compound (26) was analyzed using liquid chromatography-mass spectrometry (LC-MS), and M/Z results show: [M+H]⁺ = 1235.8 (C₃₄H₄₇N₁₆O₂₅P₄S⁺).

### Example 33

At room temperature, Compound (K) (0.06 mmol) was dissolved in a mixed solution of water (0.03 mL) and dimethyl sulfoxide (DMSO) (0.28 mL). Then, 1-(3-dimethylaminopropyl)-3-ethylcarbodiimide hydrochloride (EDC·HCl) (0.10 mmol) was added, followed by the addition of imidazole (0.19 mmol). The result was stirred at room temperature for 4 hours, after which an aqueous solution of magnesium chloride (MgCl₂, concentration 3.15 M, 0.03 mL) was added, followed by the addition of Compound (L) (0.04 mmol). The mixture was stirred at room temperature for 16 hours, then mixed with an aqueous solution of ethylenediaminetetraacetic acid (EDTA, 5 mL, concentration 1 M), wherein the amount of EDTA was three times that of magnesium chloride. Next, the mixture was purified using ion-exchange resin (DEAE) with triethylammonium bicarbonate (TEAB) aqueous solution (concentration 0.1 M) as the eluent. After concentration and drying, the product was redissolved in water, and acetone and sodium perchlorate were added, leading to the formation of a precipitate. The precipitate was centrifuged, washed with acetone, dissolved in water, and freeze-dried to yield Compound (33).

The synthesis pathway of the above reaction was as follows:

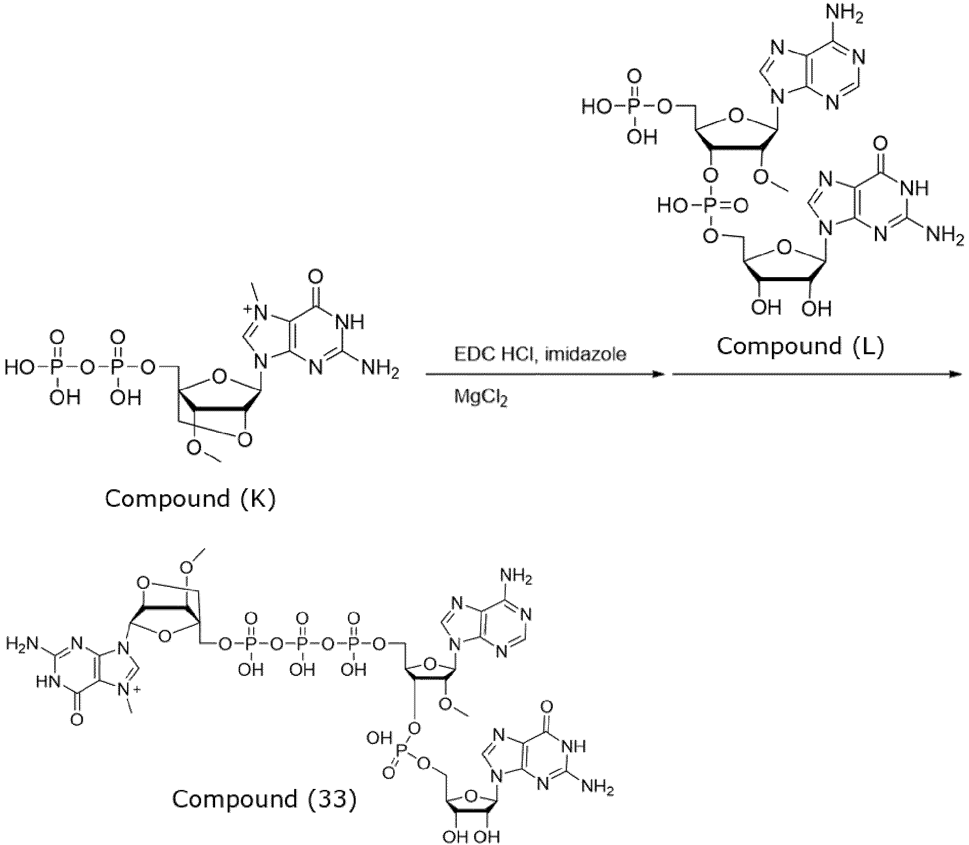

Next, the measurement results of nuclear magnetic resonance spectrometry of Compound (33) are shown below: ¹H NMR (500 MHz, D₂O) δ 8.20 (s, 1H), 7.91 (s, 1H), 7.77 (s, 1H), 5.80 (d, *J* = 4.0 Hz, 1H), 5.65 (d, *J* = 4.0 Hz, 1H), 5.54 (s, 1H), 4.75 (s, 1H), 4.66-4.58 (m, 3H), 4.35-4.31 (m, 3H), 4.26 (s, 3H), 4.22 (br, 3H), 4.16 (br, 1H), 4.09 (s, 3H), 3.84 (d, *J* = 14.0 Hz, 6H), 3.23 (s, 6H). Next, Compound (33) was analyzed using liquid chromatography-mass spectrometry (LC-MS), and M/Z results show: [M+H]⁺ =1172.7 (C₃₄H₄₆N₁₅O₂₄P₄⁺).

### Example 34

At room temperature, Compound (M) (0.06 mmol) was dissolved in a mixed solution of water (0.03 mL) and dimethyl sulfoxide (DMSO) (0.28 mL). Then, 1-(3-dimethylaminopropyl)-3-ethylcarbodiimide hydrochloride (EDC·HCl) (0.10 mmol) was added, followed by the addition of imidazole (0.19 mmol). The result was stirred at room temperature for 4 hours, after which an aqueous solution of magnesium chloride (MgCl₂, concentration 3.15 M, 0.03 mL) was added, followed by the addition of Compound (L) (0.04 mmol). The mixture was stirred at room temperature for 16 hours, then mixed with an aqueous solution of ethylenediaminetetraacetic acid (EDTA, 5 mL, concentration 1 M), wherein the amount of EDTA was three times that of magnesium chloride. Next, the mixture was purified using ion-exchange resin (DEAE) with triethylammonium bicarbonate (TEAB) aqueous solution (concentration 0.1 M) as the eluent. After concentration and drying, the product was redissolved in water, and acetone and sodium perchlorate were added, leading to the formation of a precipitate. The precipitate was centrifuged, washed with acetone, dissolved in water, and freeze-dried to yield Compound (34).

The synthesis pathway of the above reaction was as follows:

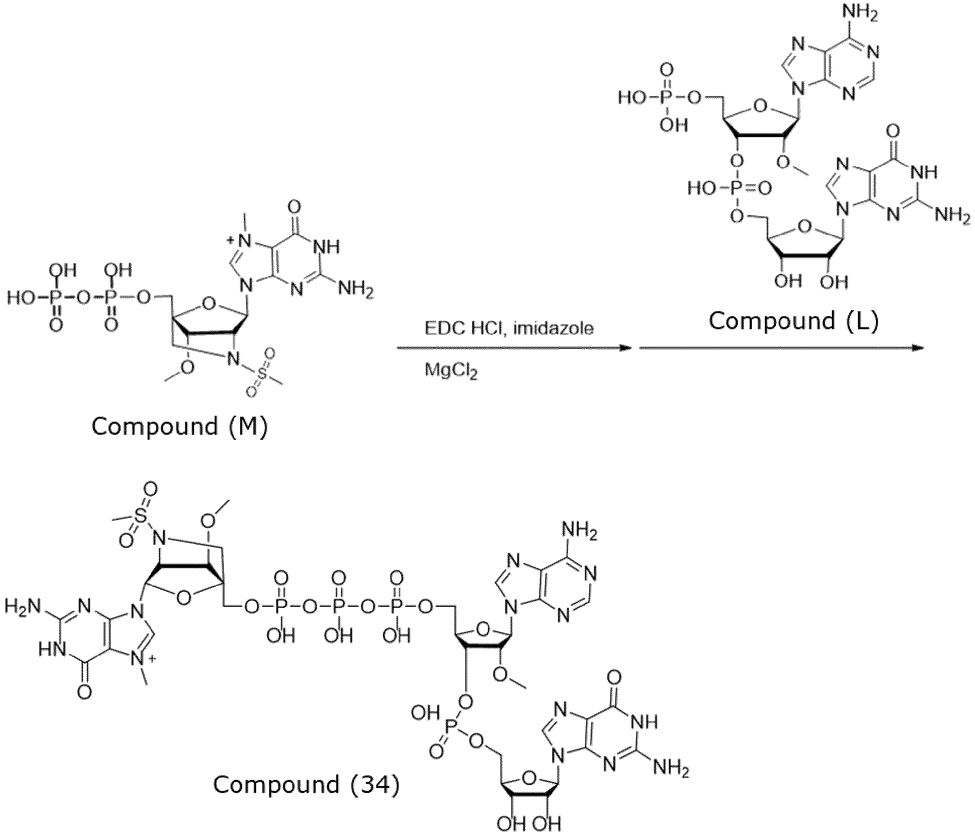

Next, the measurement results of nuclear magnetic resonance spectrometry of Compound (34) are shown below: ¹H NMR (400 MHz, D₂O) δ 8.24 (s, 1H), 7.95 (s, 1H), 7.80 (s, 1H), 5.84 (d, *J* = 6.0 Hz, 1H), 5.67 (d, *J* = 5.6 Hz, 1H), 5.61 (s, 1H), 4.81-4.72 (m, 1H), 4.69 (s, 1H), 4.35-4.31 (m, 3H), 4.27-4.23 (m, 2H), 4.17-4.11 (m, 4H), 4.08-4.03 (m, 2H), 3.89-3.85(m, 4H), 3.42 (d, *J* = 9.6 Hz, 1H), 3.37 (d, *J* = 9.6 Hz, 1H), 3.26 (s, 3H), 3.24 (s, 3H), 3.04 (q, *J* = 7.2 Hz, 12H), 2.99 (s, 3H), 1.12 (t, *J* = 7.2 Hz, 19H). Next, Compound (34) was analyzed using liquid chromatography-mass spectrometry (LC-MS), and M/Z results show: [M+H]⁺ =1249.17 (C₃₅H₄₉N₁₆O₂₅P₄S⁺).

### Example 35

At room temperature, Compound (M) (0.06 mmol) was dissolved in a mixed solution of water (0.03 mL) and dimethyl sulfoxide (DMSO) (0.28 mL). Then, 1-(3-dimethylaminopropyl)-3-ethylcarbodiimide hydrochloride (EDC·HCl) (0.10 mmol) was added, followed by the addition of imidazole (0.19 mmol). The result was stirred at room temperature for 4 hours, after which an aqueous solution of magnesium chloride (MgCl₂, concentration 3.15 M, 0.03 mL) was added, followed by the addition of Compound (N) (0.04 mmol). The mixture was stirred at room temperature for 16 hours, then mixed with an aqueous solution of ethylenediaminetetraacetic acid (EDTA, 5 mL, concentration 1 M), wherein the amount of EDTA was three times that of magnesium chloride. Next, the mixture was purified using ion-exchange resin (DEAE) with triethylammonium bicarbonate (TEAB) aqueous solution (concentration 0.1 M) as the eluent. After concentration and drying, the product was redissolved in water, and acetone and sodium perchlorate were added, leading to the formation of a precipitate. The precipitate was centrifuged, washed with acetone, dissolved in water, and freeze-dried to yield Compound (35).

The synthesis pathway of the above reaction was as follows:

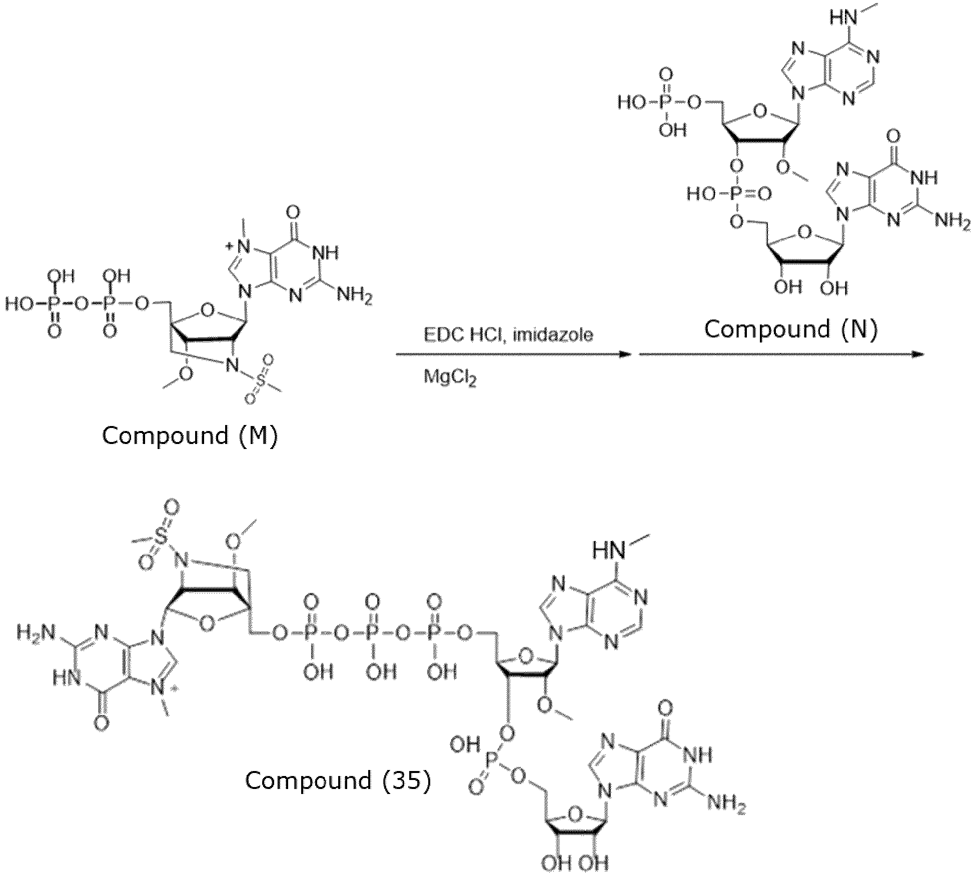

Next, the measurement results of nuclear magnetic resonance spectrometry of Compound (35) are shown below: ¹H NMR (400 MHz, D₂O) δ 8.28 (s, 1H), 8.01 (s, 1H), 7.86 (s, 1H), 5.91 (d, J = 5.5 Hz, 1H), 5.75 (d, J = 5.5 Hz, 1H), 5.63 (s, 1H), 4.86-4.84 (m, 3H), 4.75 (s, 2H), 4.66 (t, J = 5.5 Hz, 5.5 Hz, 1H), 4.45 (s, 1H), 4.41 (t, J = 3.5 Hz, 5.0 Hz, 1H), 4.37-4.33 (m, 2H), 4.27-4.25 (m, 4H), 4.21-4.13 (m, 2H), 3.93 (s, 4H), 3.50 (dd, J = 9.0 Hz, 19.5 Hz, 3H), 3.38 (s, 3H), 3.32 (s, 3H), 3.08 (s, 3H), 3.00 (s, 3H). Next, Compound (35) was analyzed using liquid chromatography-mass spectrometry (LC-MS), and M/Z results show: [M+H]⁺ =1263.8 (C₃₆H₅₁N₁₆O₂₅P₄S⁺).

### Evaluation of mRNA Yield:

mRNA was produced using *in vitro* transcription (IVT) technology with cap analogs of Compound (25), (26) and (33) to (35) of the disclosure, as well as the TriLink CleanCap cap analog (product number #N-7113). First, the DNA template plasmid required for IVT was prepared, followed by an enzymatic reaction using T7 enzyme to synthesize mRNA. The resulting mRNA yield was evaluated, and the results are shown in Table 5.

**Table 5**

| | Input DNA (µg) | mRNA(µg) |
|---|---|---|
| Compound (25) | 5 | 167 |
| Compound (26) | 5 | 95 |
| Compound (33) | 5 | 134 |
| Compound (34) | 5 | 133 |
| Compound (35) | 5 | 138 |
| Trilink CleanCap BTfLuc mRNA | 5 | 169 |

### Capping efficiency evaluation (1): Using Anti-Cap antibody

Nuclease-free water was added into the lyophilized carrier RNA to obtain an RNA stock solution (with a concentration of 2 µg/µL). Next, the RNA stock solution with various cap analogs (Compound (3) and Jena-ARCA cap analog, with trade number #NU-855L, commercially available from Jena Bioscience) was heated individually at 80°C for 2 minutes, and then cooled at 0°C for 5 minutes. Next, the result was placed on a nitrocellulose membrane and exposed to UV illumination to obtain a crosslinked RNA sample.

Then, 2.5% skim milk solution was added to block the nitrocellulose membrane with crosslinked RNA sampleat room temperature for 30 minutes. Thereafter, the primary antibody (Anti-7-methylguanosine (m7G)-Cap mAb) was diluted with the 2.5% skim milk solution and incubated at room temperature for 1 hour. Next, the sample was washed three times with TBST buffer (10 minutes each time). The result was reacted with chemiluminescent reagent (Femto) for 1-3 minutes. Finally, an image analysis of the result was performed via a chemiluminescent imaging system (Fujifilm LAS 4000). The results of the image analysis demonstrate that Compound (3) of the disclosure possesses capping capability comparable to that of commercially available ARCA.

### Capping efficiency evaluation (2): Using IP-RP-UPLC(1)

Nuclease-free water was added into the lyophilized carrier RNA to obtain an RNA stock solution (with a concentration of 2 µg/µL). Next, deionized water (0.1 mL) and RNA stock solution (10 µL) were mixed with cap analogs (compound (3) and Jena-ARCA cap analog (product number #NU-855L, commercially available from Jena Bioscience)) individually to obtain the samples.

Next, the sample (10 µL) was injected into a column to perform ion-pair reverse-phase ultra-performance liquid chromatography (IP-RP UPLC) using the solution gradient outlined in Table 6 (45 minutes). The absorbance at the UV260 wavelength was monitored. The column used was an oligonucleotide column (Acquity Premier Oligonucleotide C18, manufactured and sold by Waters Corp). A water-based buffer solution (triethylamine acetate buffer, pH 7.0, concentration 100 mM) was used as Solution A, and an acetonitrile (ACN)-based buffer solution (composed of Solution A and ACN in a 3:1 volume ratio) was used as Solution B.

**Table 6**

| time (minute) | Solution A (vol%) | Solution B (vol%) | flow rate (mL/ minute) |
|---|---|---|---|
| 0 | 90 | 10 | 0.5 |
| 36 | 85.5 | 14.5 | 0.5 |
| 36.1 | 90 | 10 | 0.5 |
| 45 | 90 | 10 | 0.5 |

The analysis results indicate that the capping efficiency of Compound (3) of the disclosure is approximately 60.8%, comparable to the capping efficiency of the commercially available Jena-ARCA (approximately 63%).

### Capping efficiency evaluation (3): Using IP-RP-UPLC

mRNA was produced using *in vitro* transcription (IVT) technology with cap analogs of Compound (25), (33) to (35) of the disclosure herein, as well as the TriLink CleanCap cap analog (product number #N-7113). The DNA template used in this process consisted of 40-nucleotide (nt) fragments, and mRNA was synthesized through an enzymatic reaction using T7 enzyme to produce 40-nt mRNAs.

Nuclease-free water was added to the lyophilized carrier RNA to obtain an RNA stock solution (with concentration of 2 µg/µL). Deionized water (0.1 mL) and RNA stock solution (10 µL) were then mixed with the 40-nt mRNAs obtained by aforementioned cap analogs to obtain the samples.

Next, 10 µL of each sample was injected into a column and analyzed using ion-pair reversed-phase ultra-performance liquid chromatography (IP-RP UPLC) (45 minutes)according to the solution gradient listed in Table 7. The absorbance value of UV 260 nm was monitored, and the capping efficiency (%) was evaluated based on the obtained results, as shown in Table 8. The column used for the analysis was an oligonucleotide column (Acquity Premier Oligonucleotide C18, manufactured and sold by Waters Corp). A water-based buffer solution (triethylammonium acetate buffer, pH 7.0, concentration 100 mM) was used as Solution A, while an acetonitrile (ACN)-based buffer solution (a mixture of Solution A and acetonitrile in a 3:1 volume ratio) was used as Solution B.

**Table 7**

| time (minute) | Solution A (vol%) | Solution B (vol%) | flow rate (mL/ minute) |
|---|---|---|---|
| 0 | 95 | 5 | 0.3 |
| 30 | 90 | 10 | 0.3 |
| 50 | 80 | 20 | 0.3 |
| 50.1 | 95 | 5 | 0.3 |
| 60 | 95 | 5 | 0.3 |

**Table 8**

| | Capping efficiency (%) |
|---|---|
| Compound (25) | 71.3 |
| Compound (33) | 100 |
| Compound (34) | 100 |
| Compound (35) | 81.4 |
| Trilink CleanCap BTfLuc mRNA | 79 |

According to the analysis results, it was determined that the capping efficiency of Compounds (25) and (33) to (35) of the disclosure ranges from approximately 71.3% to 100%, which is comparable to the commercially available TriLink CleanCap BTfLuc capping efficiency.

### Transfection efficiency evaluation

Nuclease-free water was added to the lyophilized carrier RNA to obtain an RNA stock solution (with concentration of 2 µg/µL). Next, deionized water (0.1 mL) and RNA stock solution (10 µL) were mixed with cap analogs (compound (3), compounds (25), (33) to (35), and Jena-ARCA capping analog (number #NU-855L, commercially available from Jena Bioscience)) individually to obtain the samples.

HEK293 cells were seeded at 4×10⁴ cells per well in a 96-well culture plate and cultured for 24 hours for attachment. Next, each well was treated with transfection reagent (catalog #Lipofectamine MessengerMax, commercially available from Thermo Fisher, 0.3 µL per well) and samples (50 ng per well). The cells were incubated in a 37°C incubator with 5% carbon dioxide for 5 hours. Subsequently, 100 µL of signal-activating fluorescence detection reagent (One-Glo Luciferase) was added to each well and incubated at room temperature for 5 minutes to measure luciferase activity. Then, 100 µL of the reaction mixture was pipetted and the fluorescence values were recorded using a microplate reader (GloMax Microplate Reader). According to the results, it has been shown that luciferase mRNA capped with Compound (3), (25) and (33) to (35) of the disclosure exhibited superior nucleic acid translation and protein expression in HEK293 cells compared to mRNA capped with the commercially available Jena-ARCA.

### Cell immunogenicity evaluation

HEK293 (RIG-I⁺)-IFNβ-fLuc cells were seeded at 1×10⁴ cells per well in a 96-well culture plate and cultured for 24 hours for attachment. Each well was treated with transfection reagent (catalog #Lipofectamine MessengerMax, commercially available from Thermo Fisher, 0.15 µL per well) and samples (200 ng per well). The cells were incubated in a 37°C incubator with 5% carbon dioxide for 5 hours. Next, 100 µL of signal-activating fluorescence detection reagent (One-Glo Luciferase) was added to each well and incubated at room temperature for 5 minutes to measure luciferase activity. Next, 180 µL of the reaction mixture was pipetted, and the fluorescence values were recorded using a microplate reader (GloMax Microplate Reader). According to the results, it has been indicated that luciferase mRNA capped with Compound (3) of the disclosure induced a lower immune response in HEK293 (RIG-I⁺)-IFNβ-fLuc cells compared to mRNA capped with the commercially available Jena-ARCA.

It will be clear that various modifications and variations can be made to the disclosed methods and materials. It is intended that the specification and examples be considered as exemplary only, with the true scope of the disclosure being indicated by the following claims and their equivalents.

## Claims

1. A compound, which has a structure represented by Formula (I), Formula (II) or Formula (III) 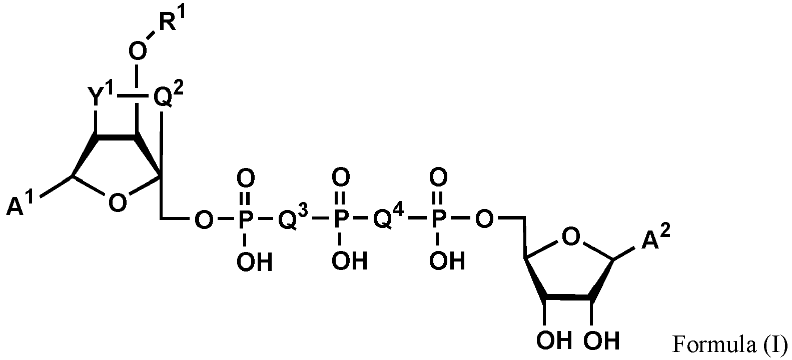 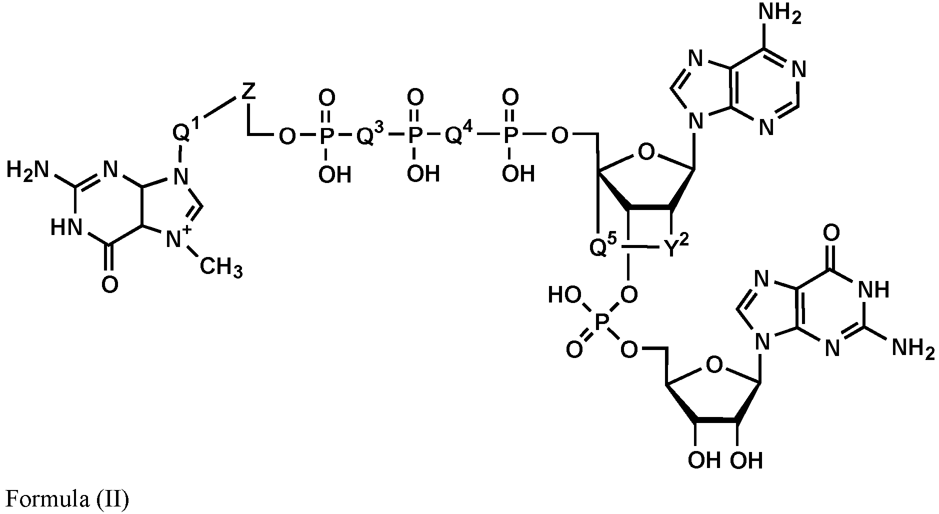 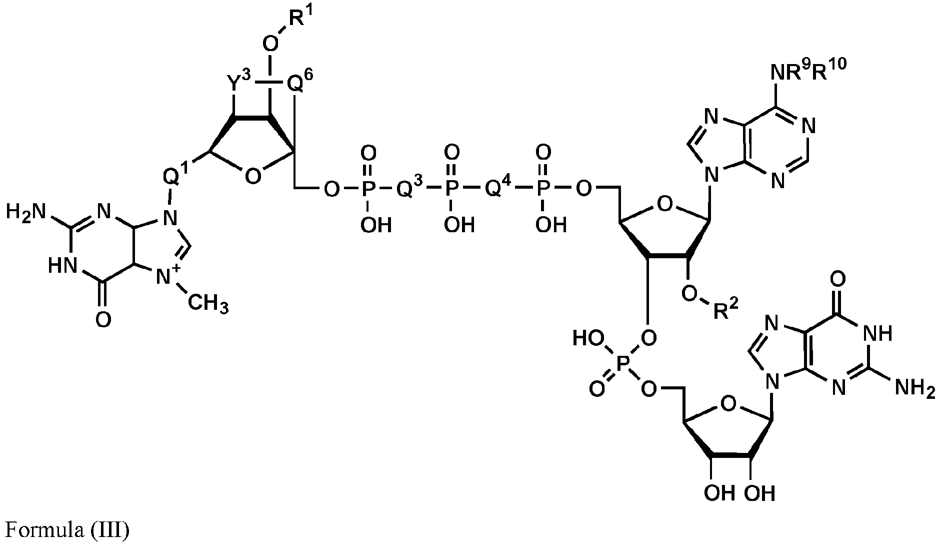 , wherein A¹ and A² are independently 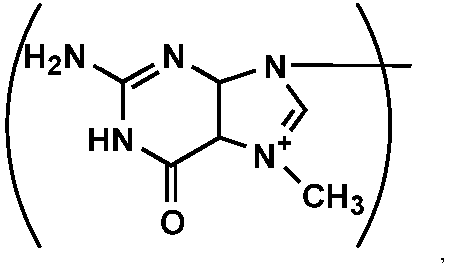 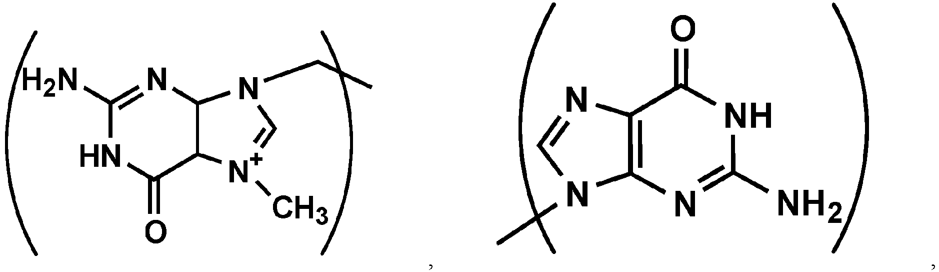 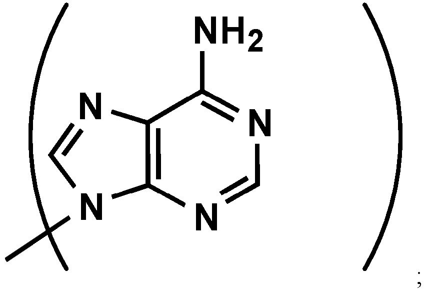 R¹ and R² are independently hydrogen, methyl or phenyl group; Q¹ is single bond or -CH₂-; Y¹ and Y³ are independently -O-, 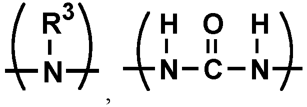 or 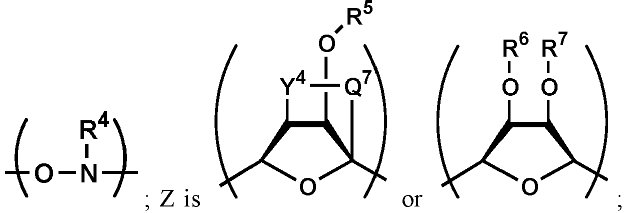 Y² and Y⁴ are independently -O-, 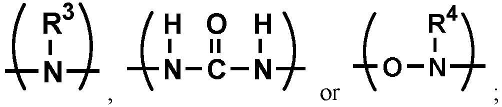 Q², Q⁵, Q⁶ and Q⁷ are independently -CH₂- or 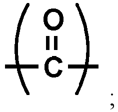 Q³ and Q⁴ are independently -O-, -CH₂- or -CCl₂-; R³ is C1-C6 alkyl group or 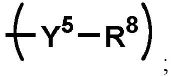 R⁴ is hydrogen or methyl; R⁵, R⁶ and R⁷ are independently hydrogen, methyl or phenyl group; Y⁵ is 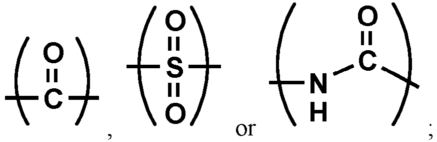 R⁸ is C1-C6 alkyl group, C4-C8 cycloalkyl group, phenyl group, benzyl group or C3-C5 heterocyclic group; and R⁹ and R¹⁰ are independently hydrogen, C1-C6 alkyl group or benzyl group.

2. The compound as claimed in Claim 1, wherein Y¹ is 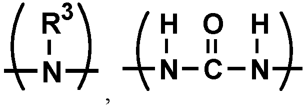 or 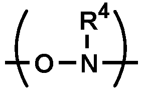 when Q² is -CH₂-; Y¹ is 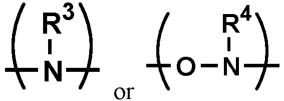 when Q⁶ is 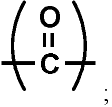 Y³ is 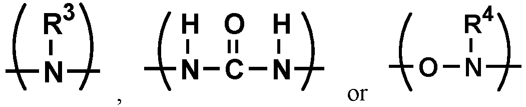 when Q² is -CH₂-; Y³ is 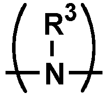 or 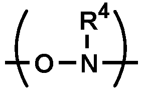 when Q⁶ is

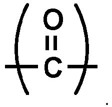

3. The compound as claimed in Claim 1, wherein Y² is -O-, 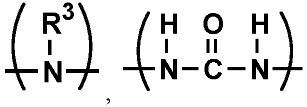 or 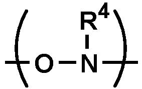 when Q⁵ is -CH₂-; Y² is 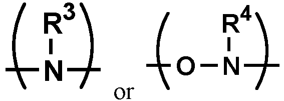 when Q⁵ is 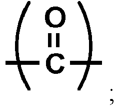 Y⁴ is -O-, 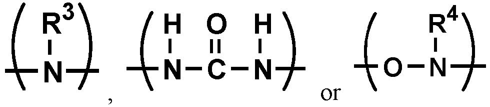 when Q⁷ is -CH₂-; and Y⁴ is 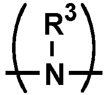 or 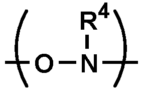 when Q⁷ is

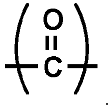

4. The compound as claimed in Claim 1, when the compound has a structure represented by Formula (II) and Y² is -O-, wherein Z is 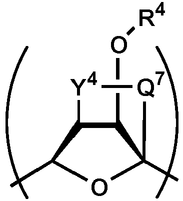 and Y⁴ is 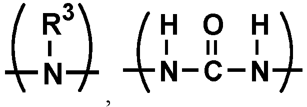 or

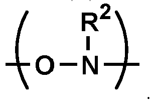

5. The compound as claimed in Claim 1, wherein the compound is 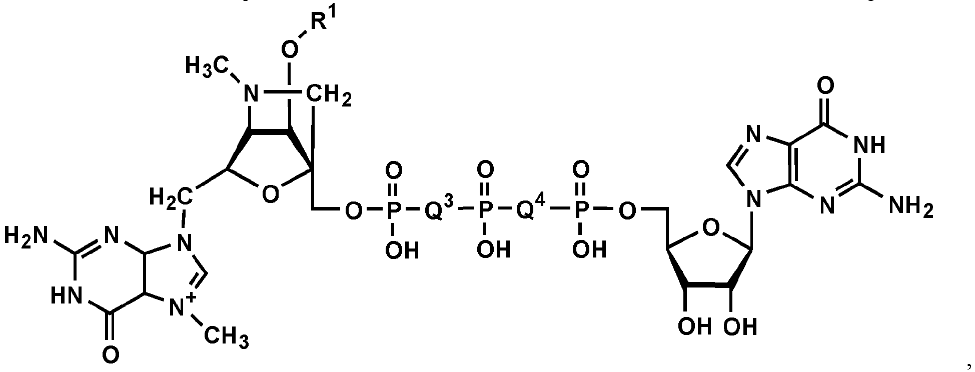 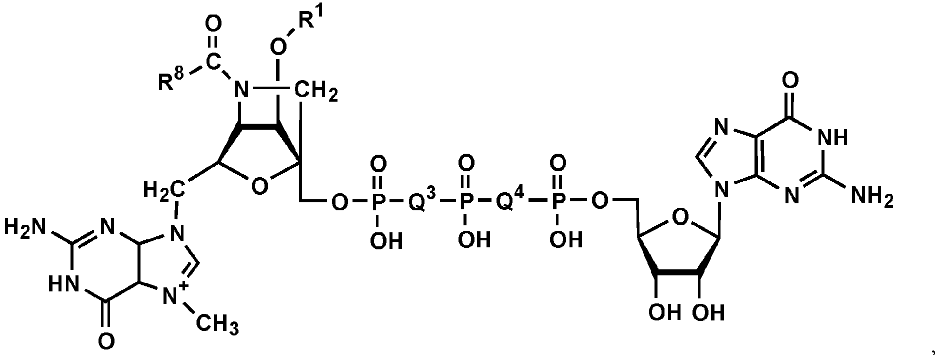 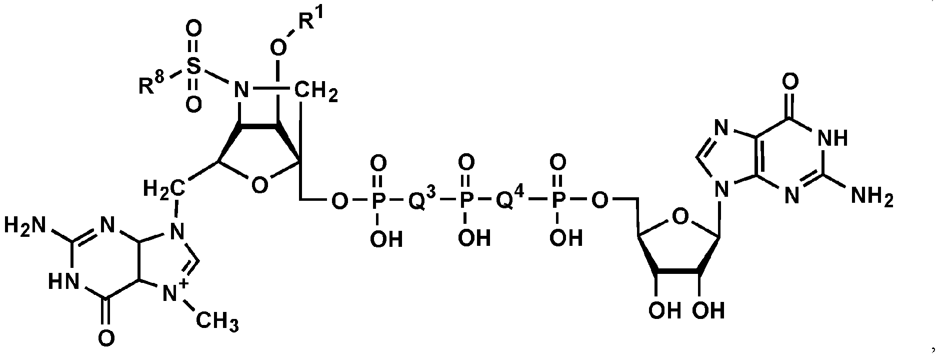 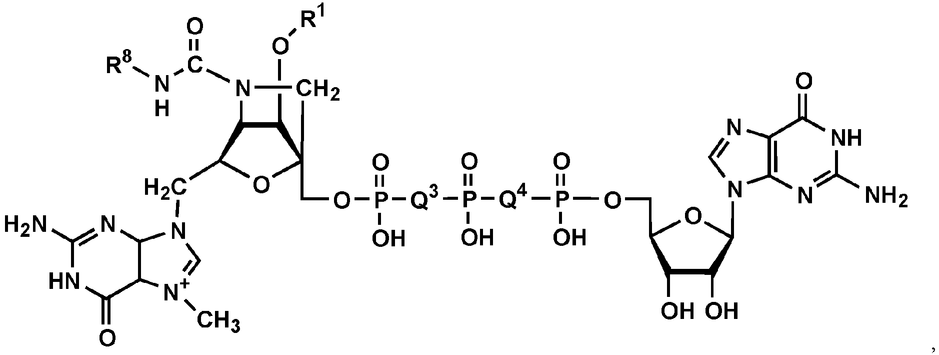 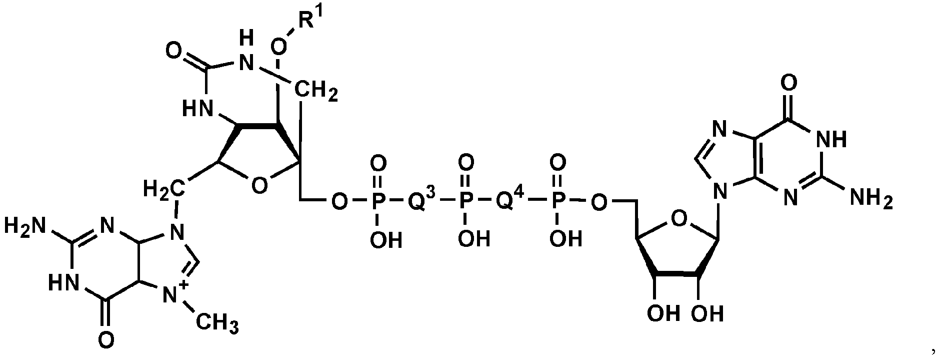 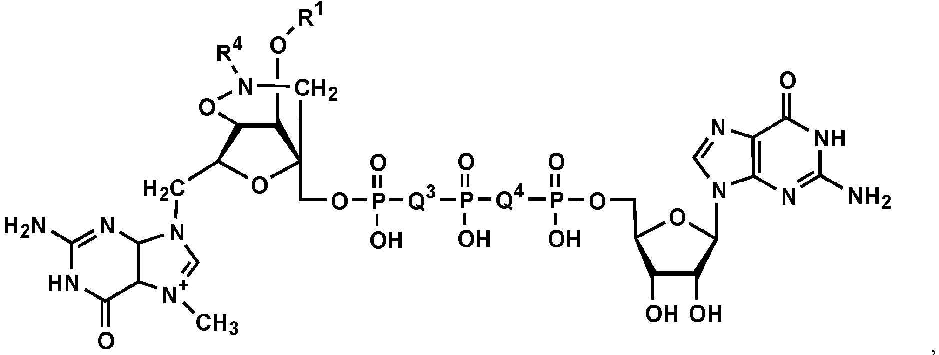 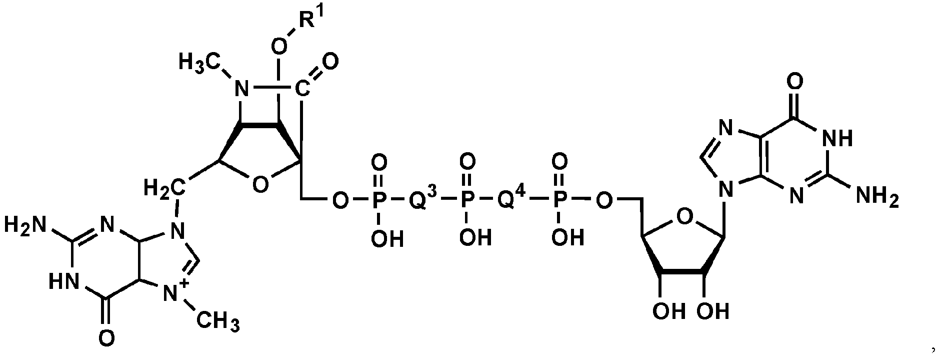 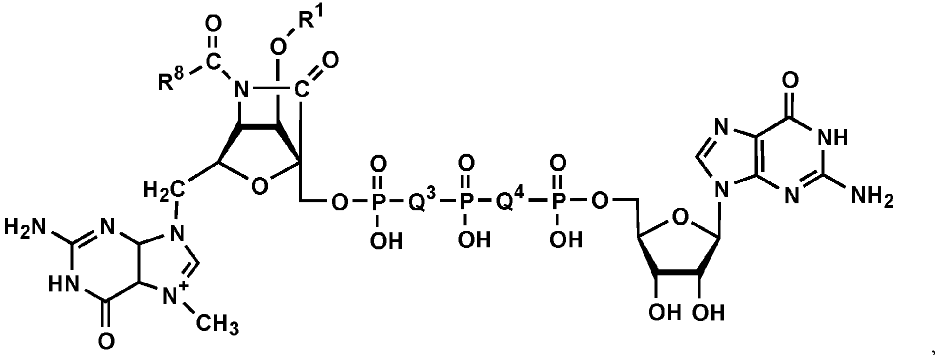 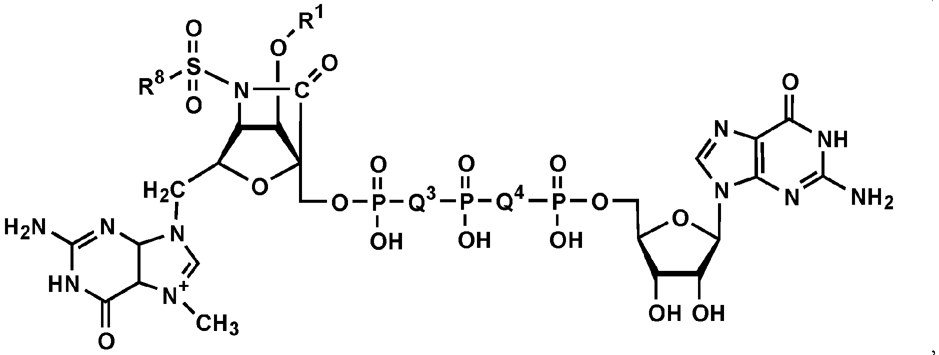 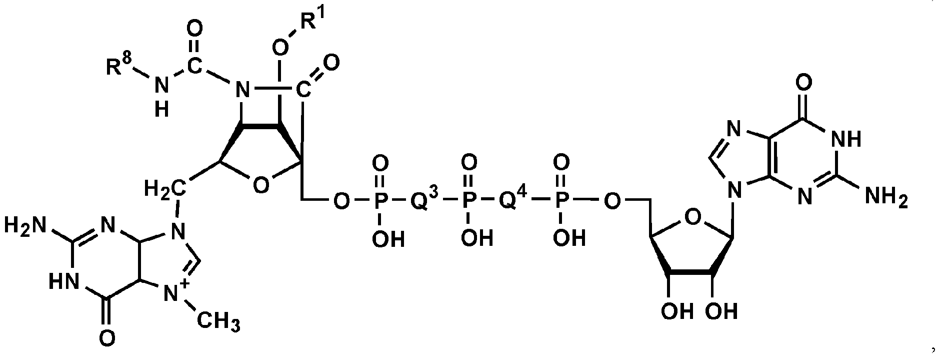 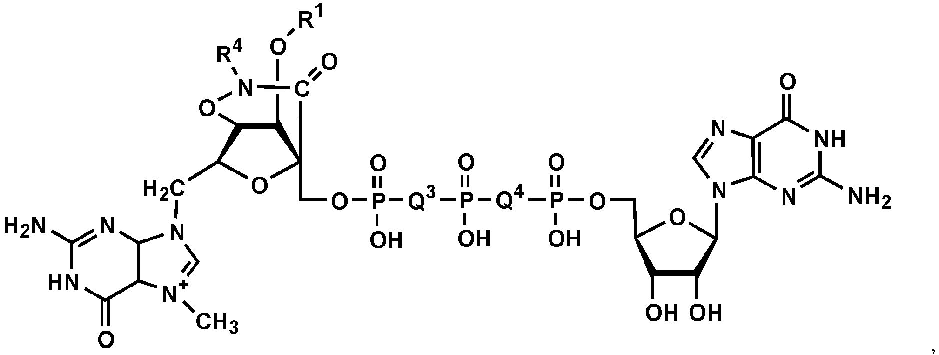 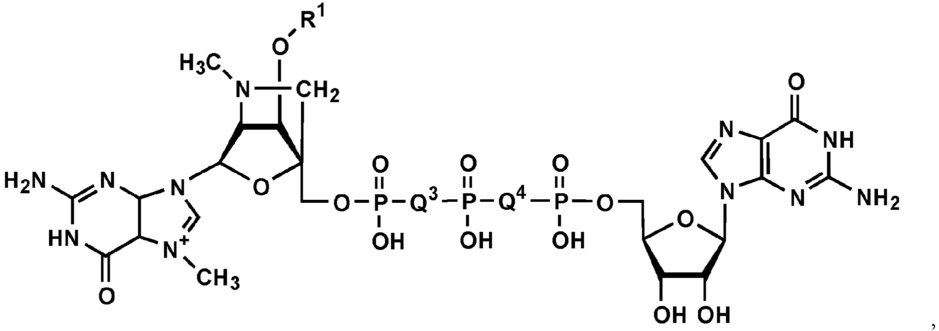 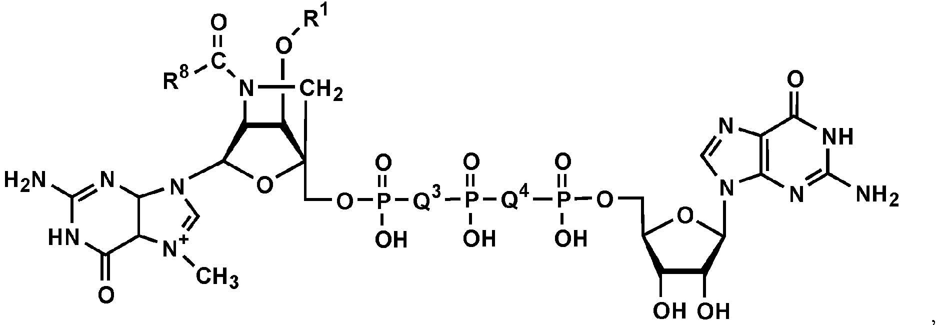 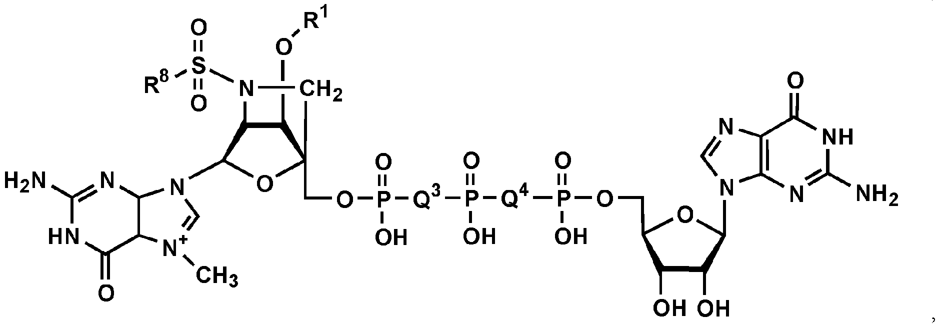 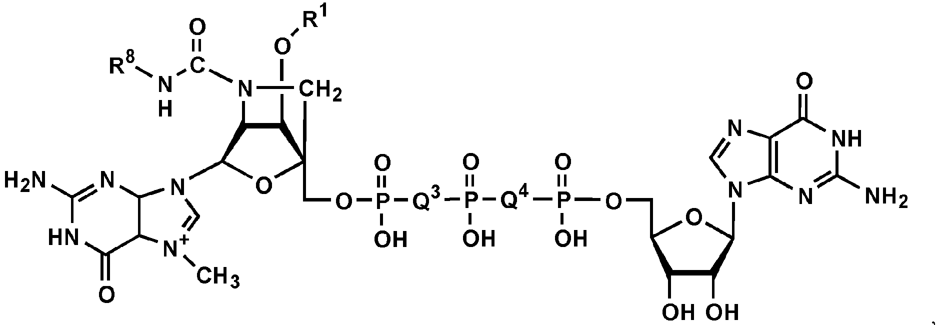 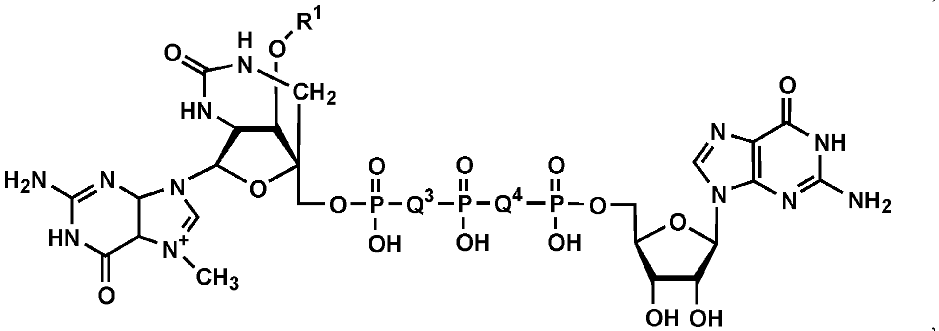 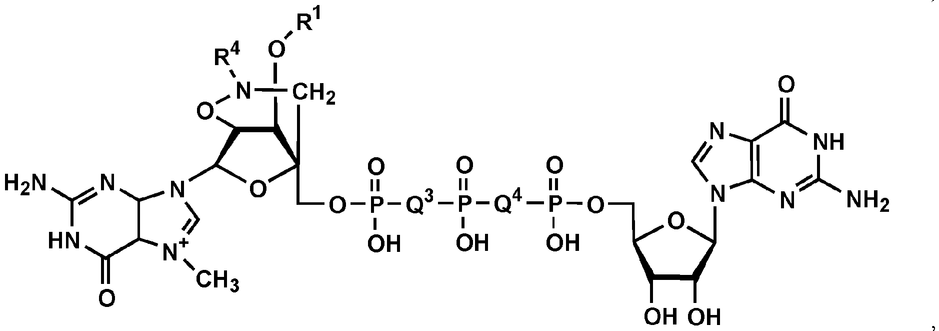 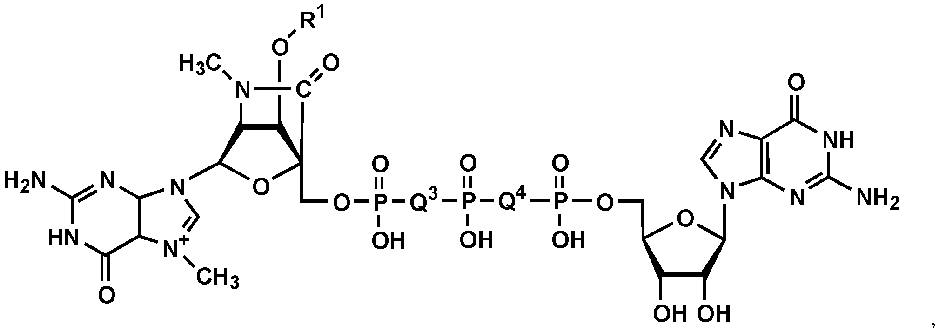 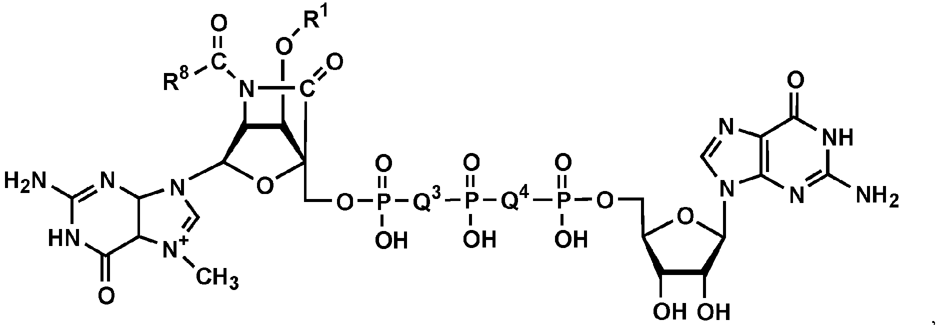 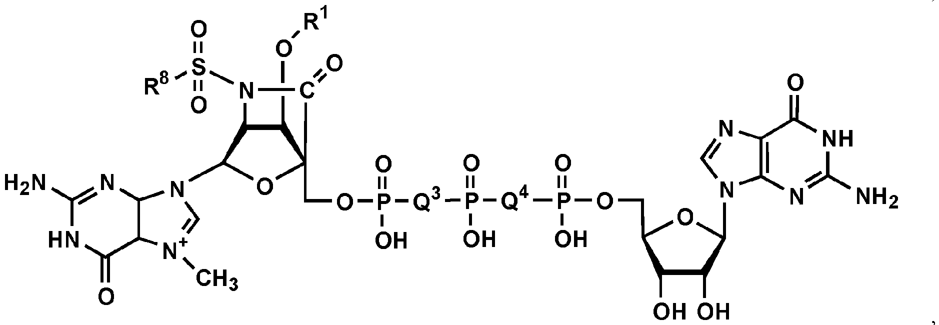 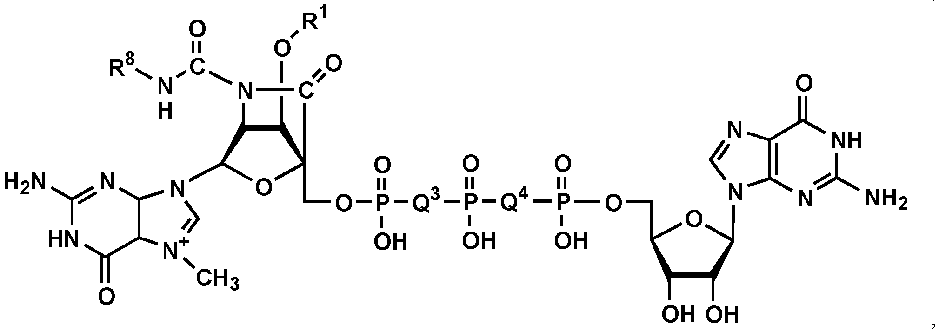 or 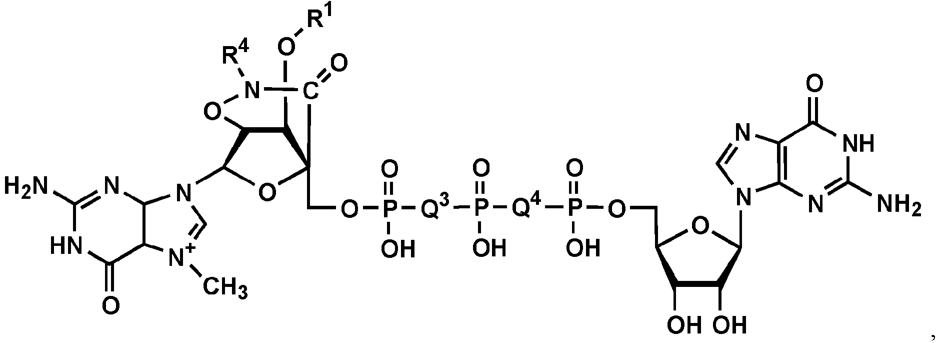 wherein R¹ is hydrogen, methyl or phenyl group; Q³ and Q⁴ are independently -O-, -CH₂- or -CCl₂-; R⁴ is hydrogen or methyl; and R⁸ is C1-C6 alkyl group, C4-C8 cycloalkyl group, phenyl group, benzyl group or C3-C5 heterocyclic group.

6. The compound as claimed in Claim 1, wherein the compound is 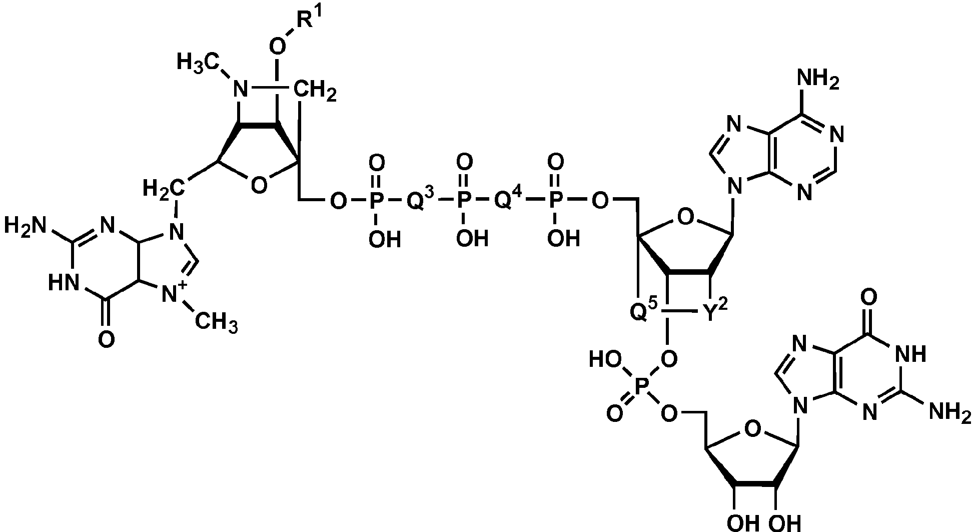 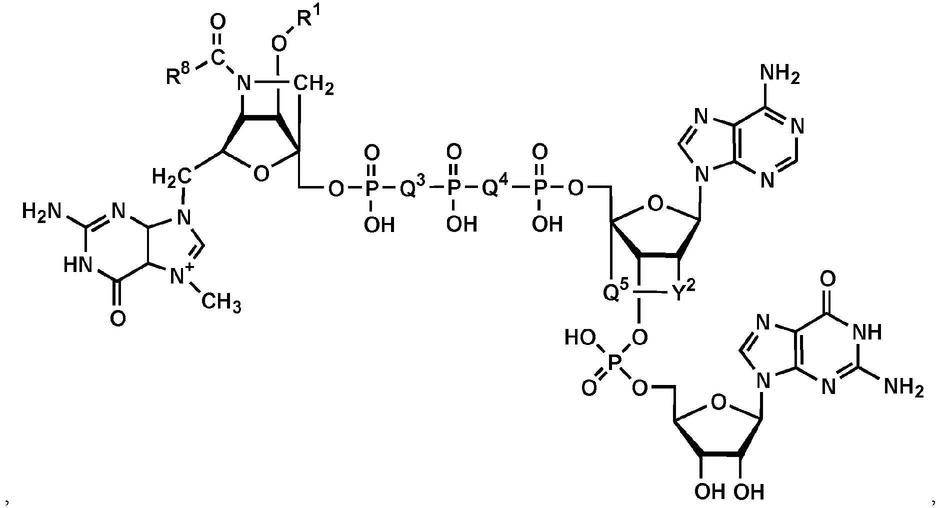 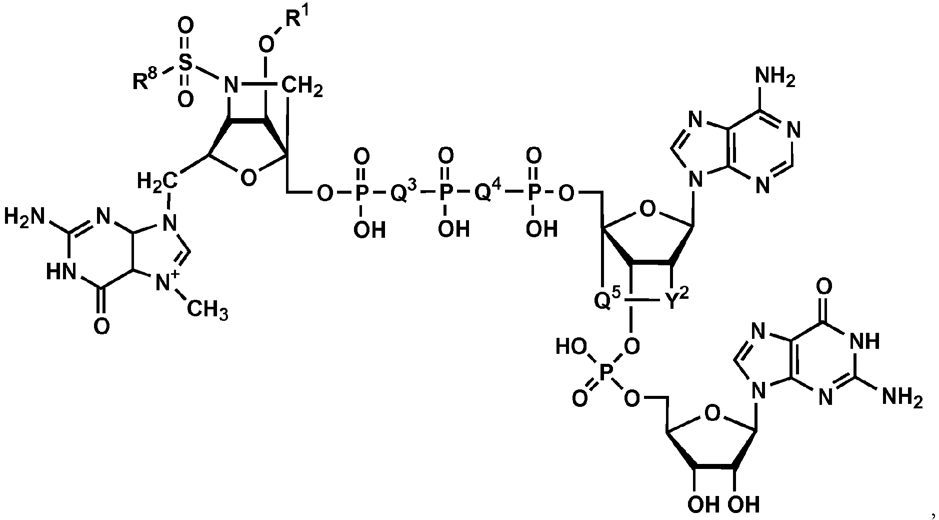 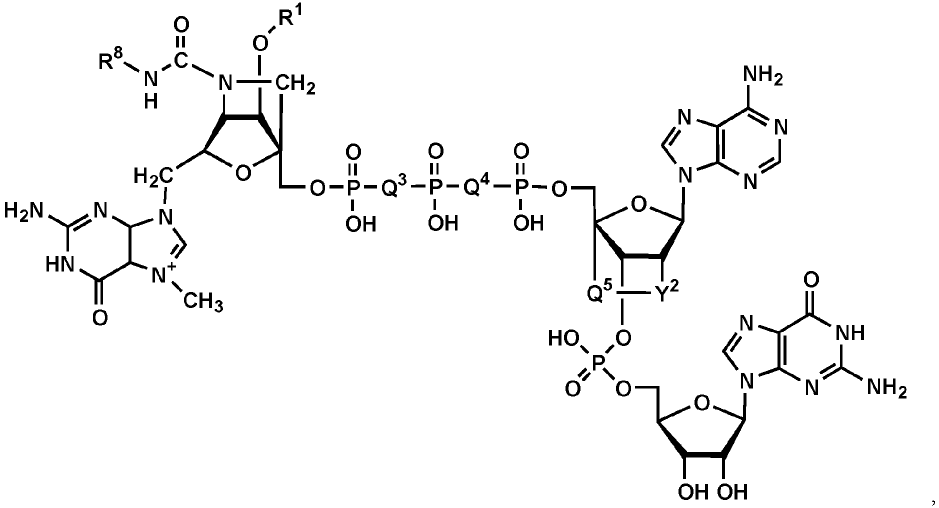 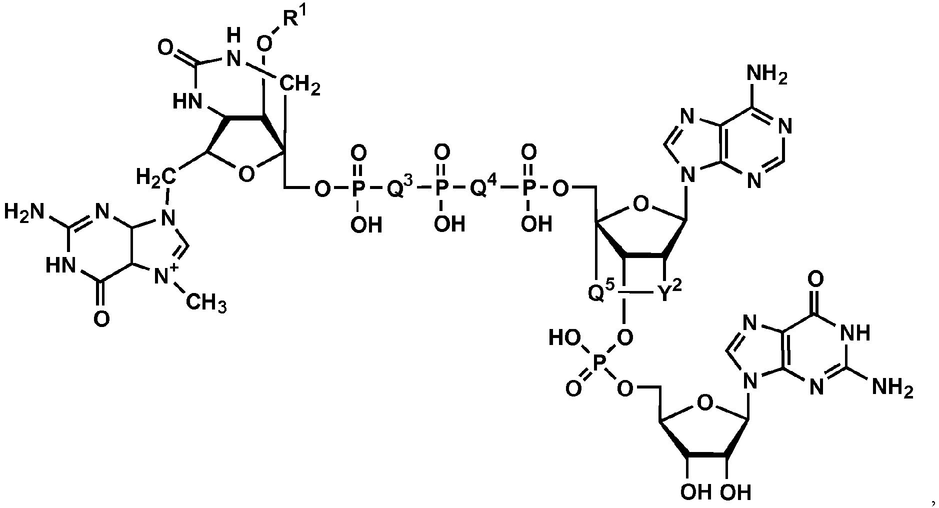 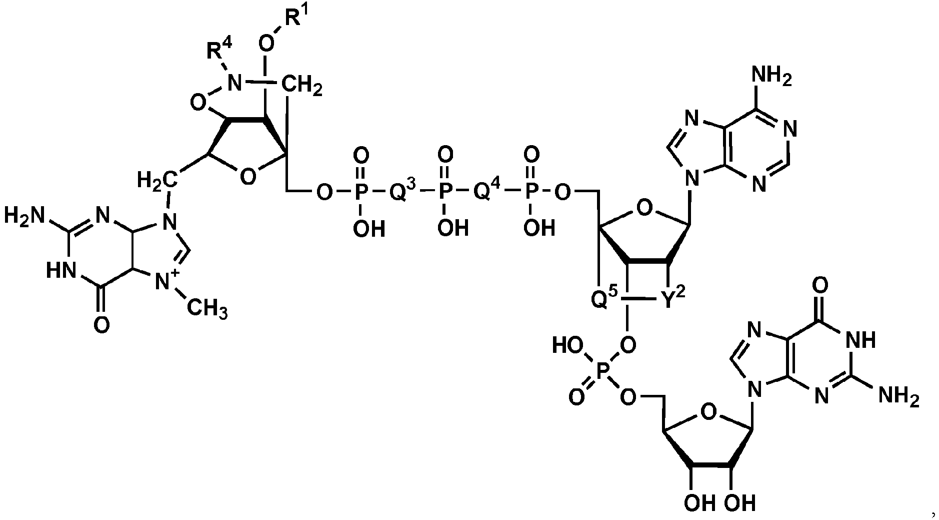 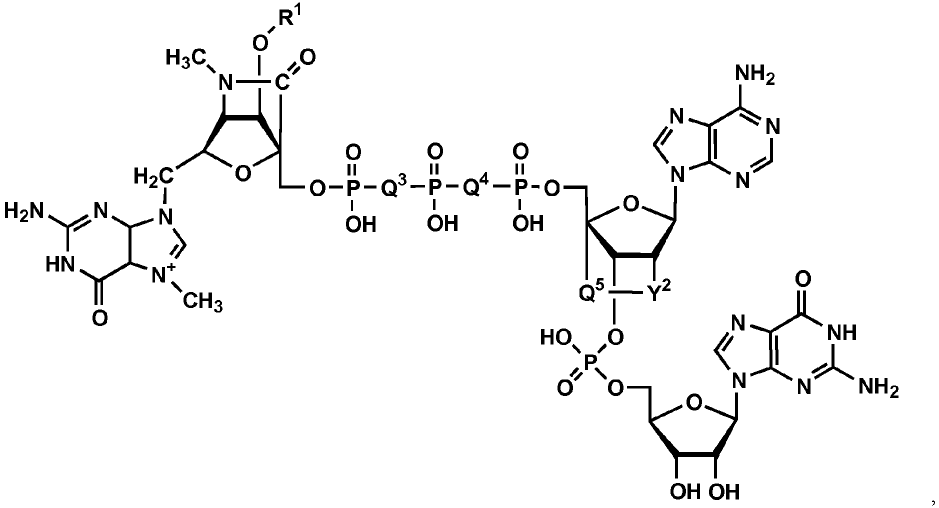 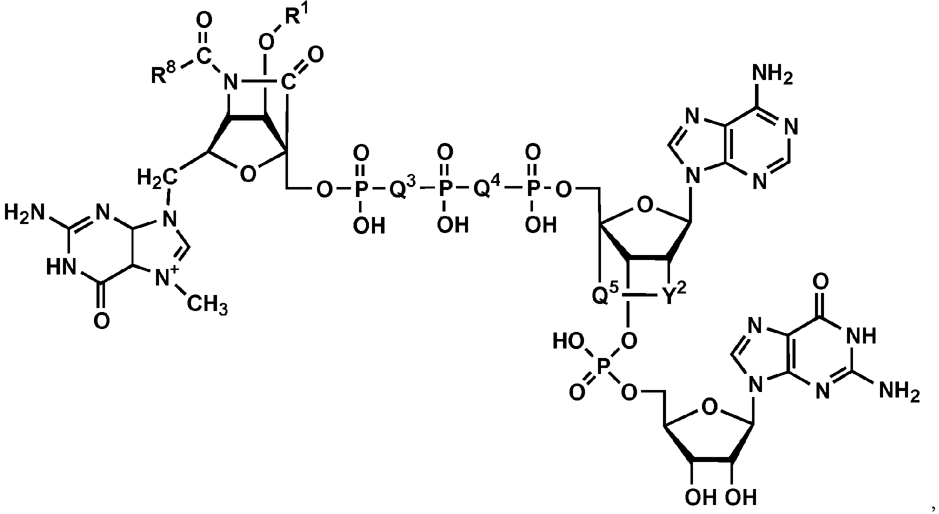 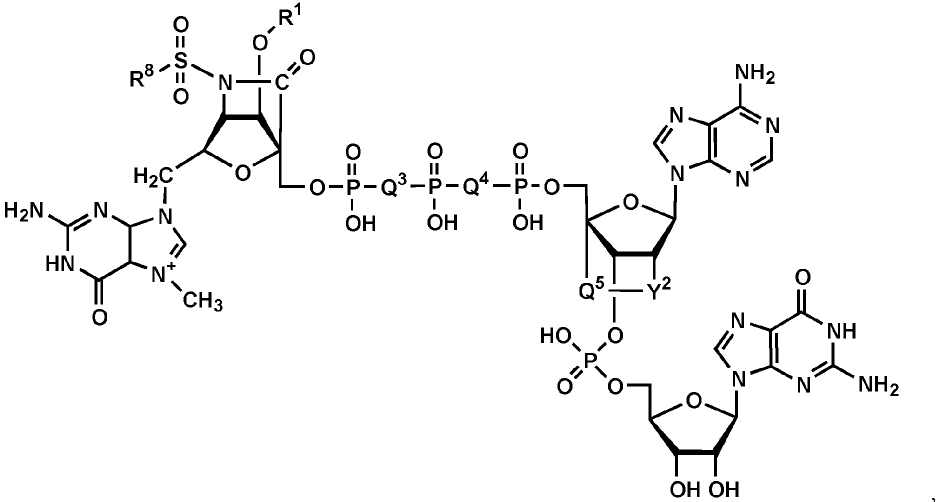 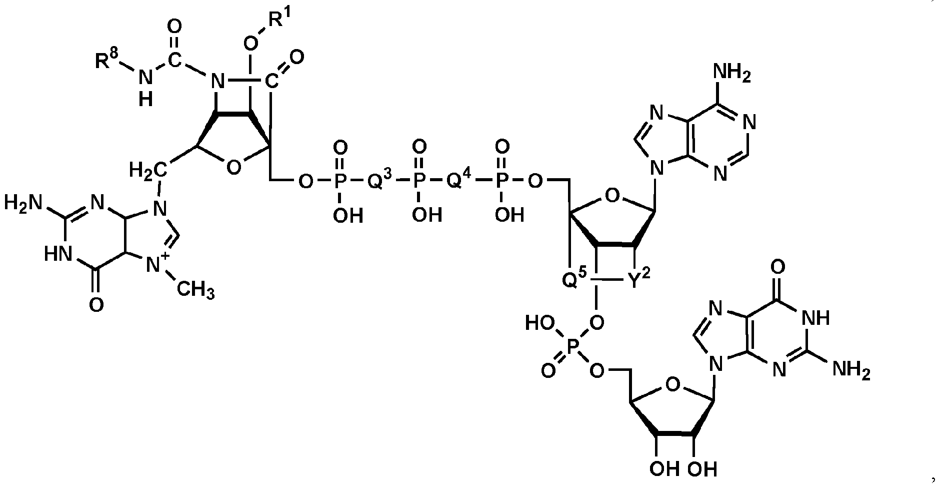 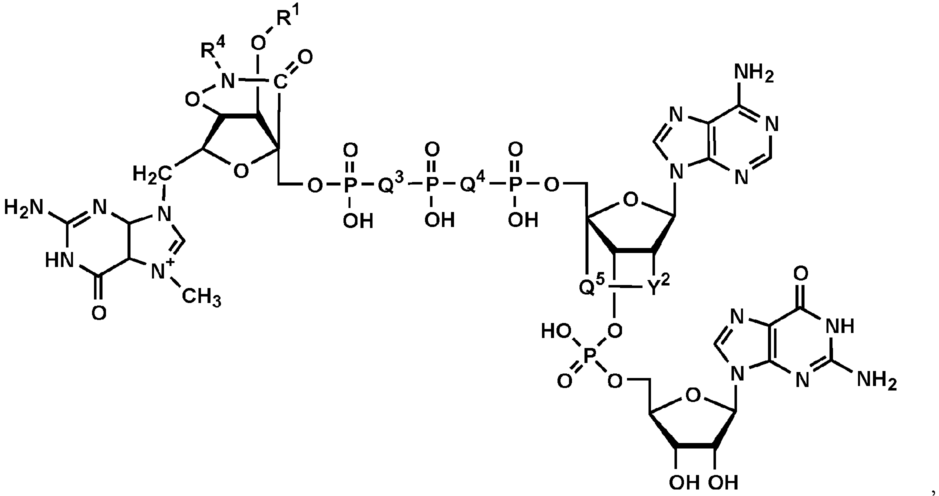 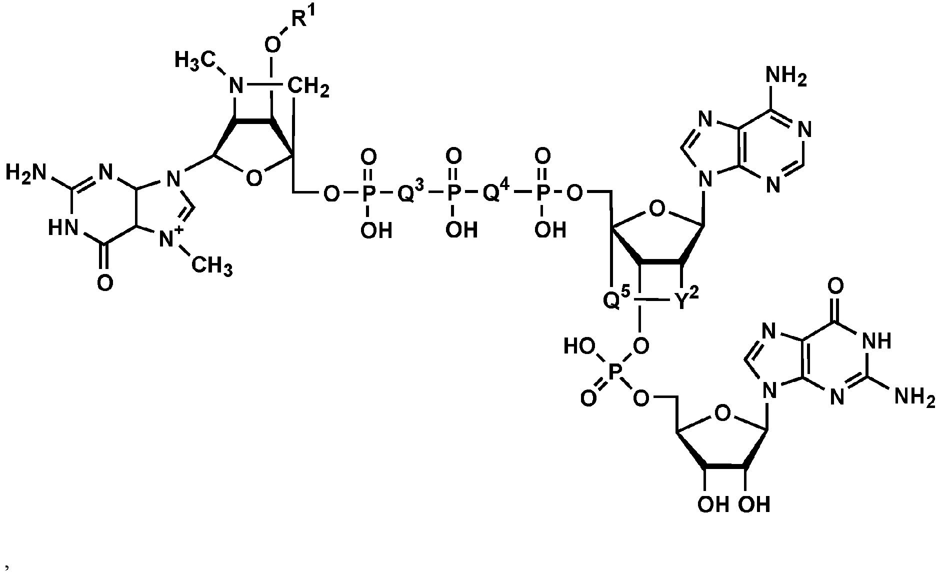 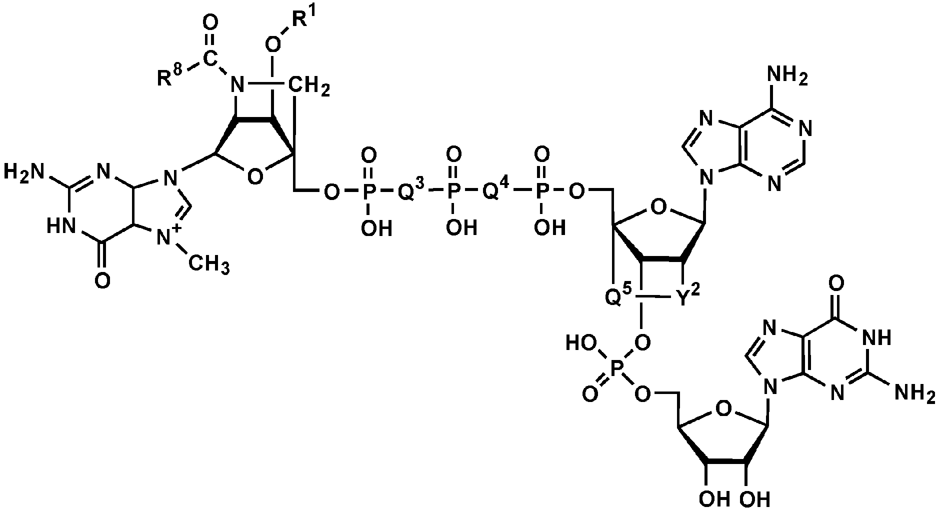 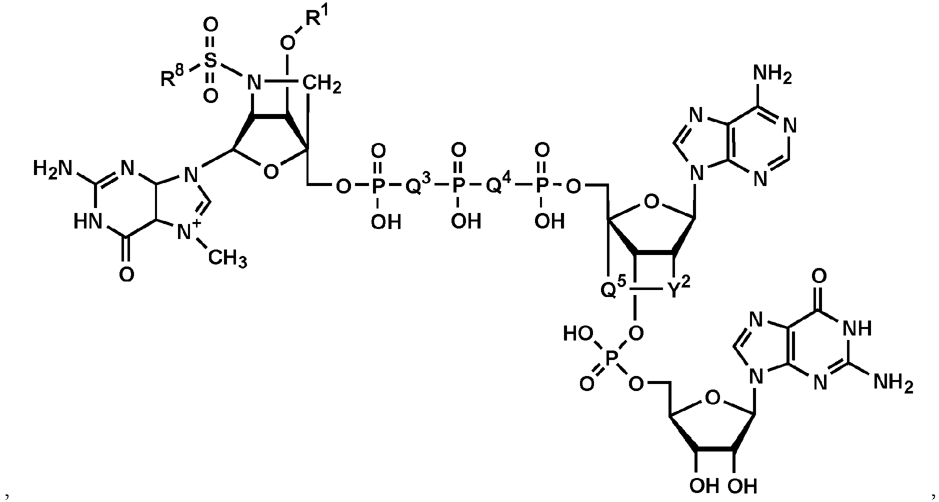 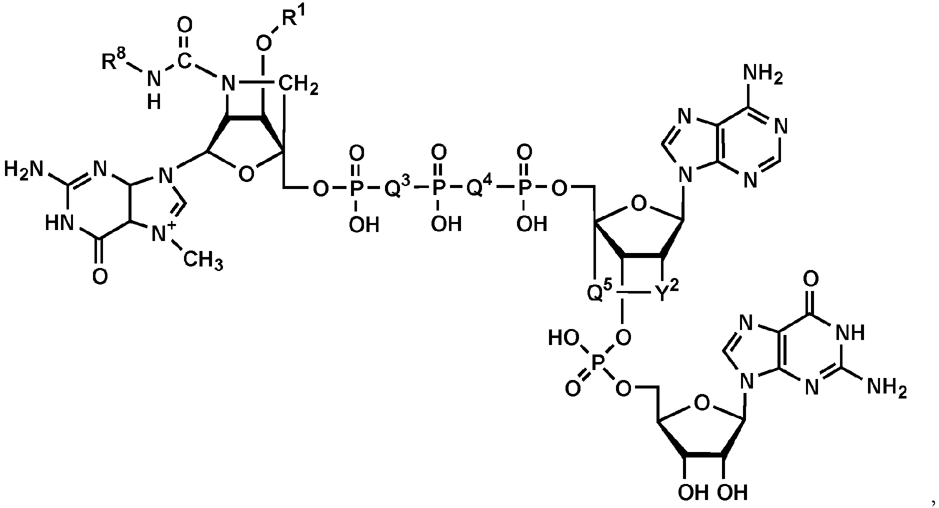 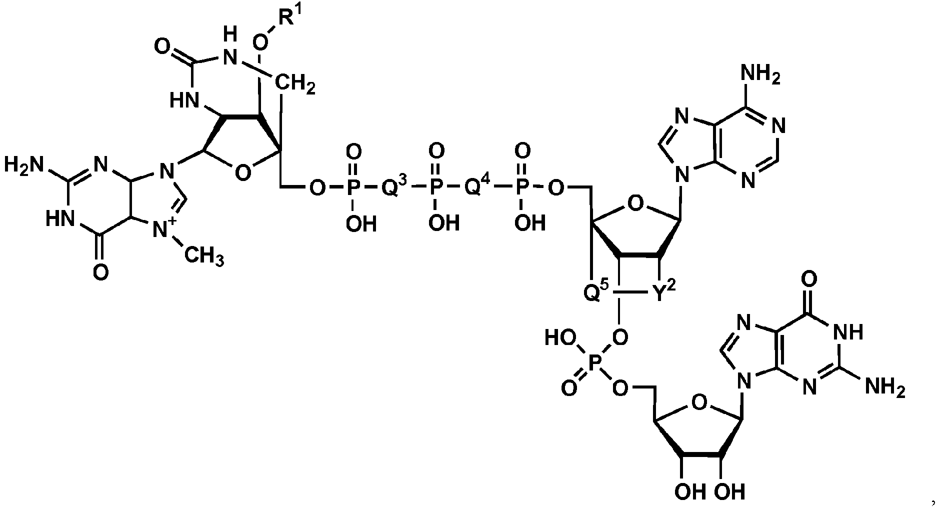 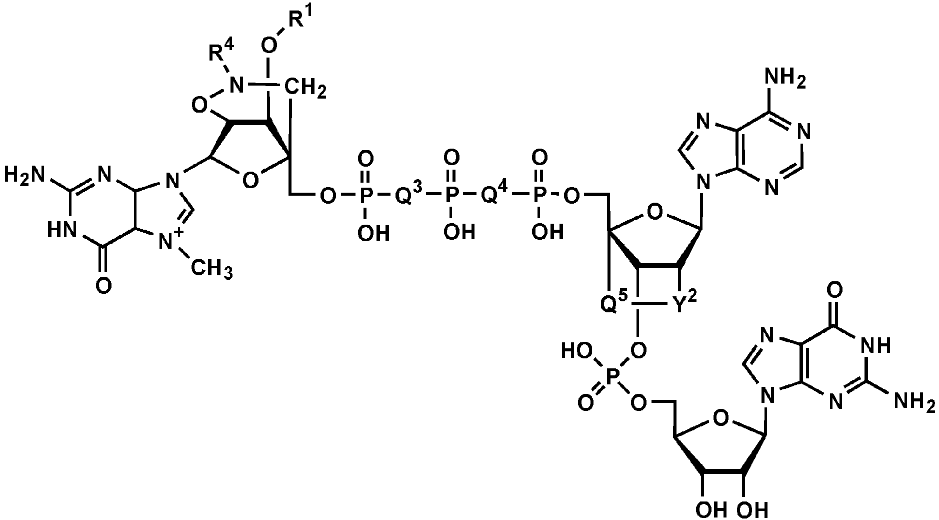 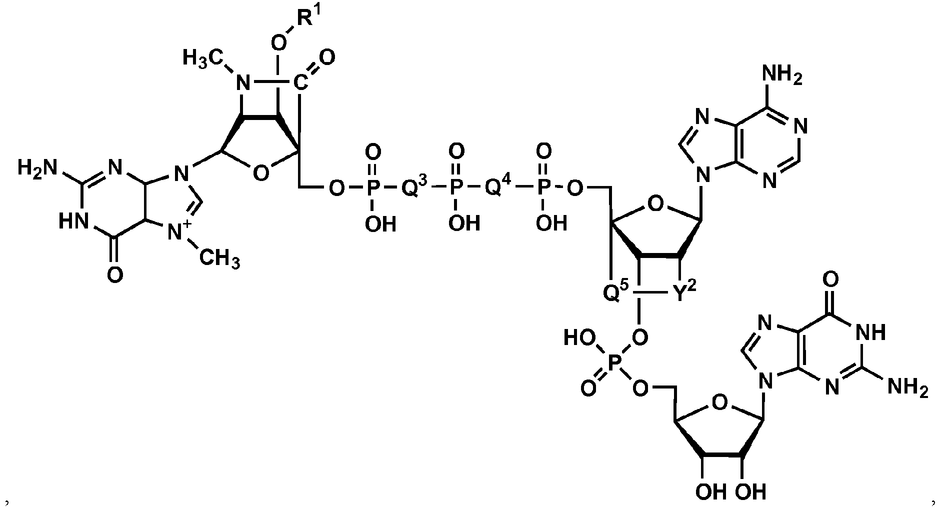 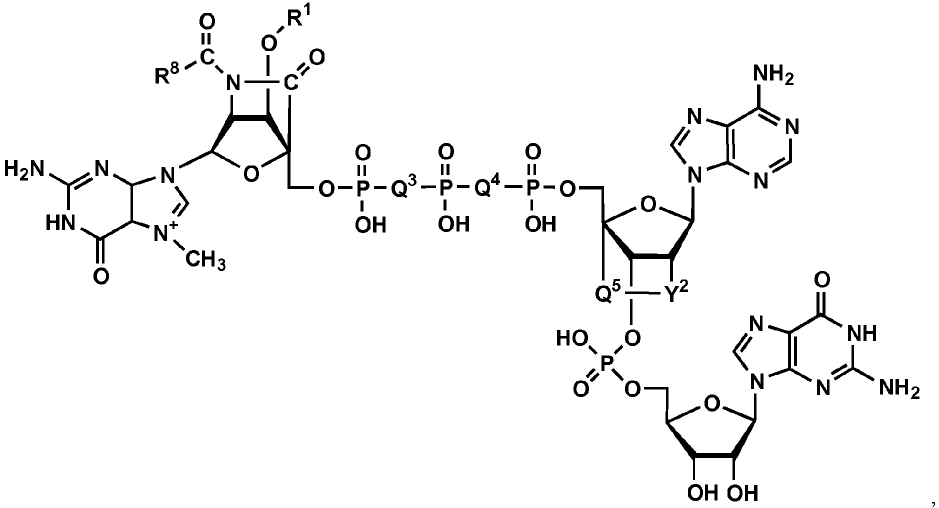 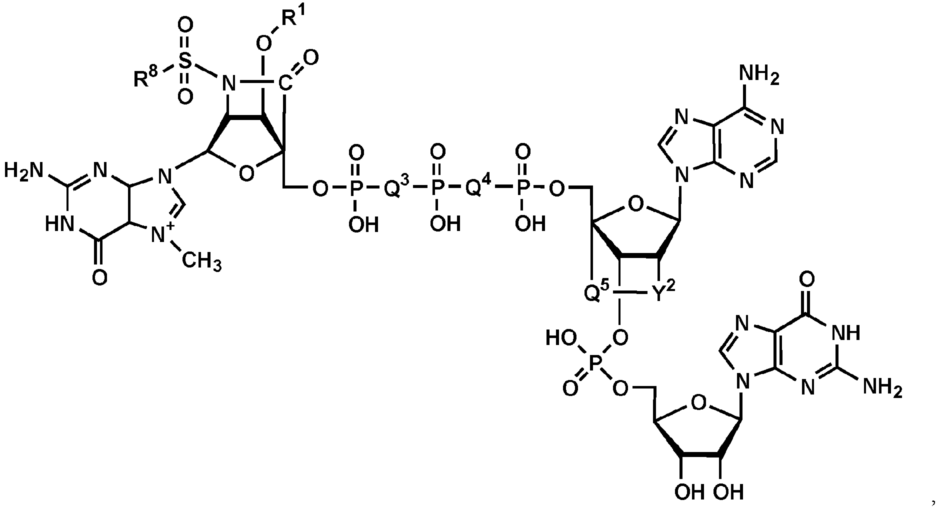 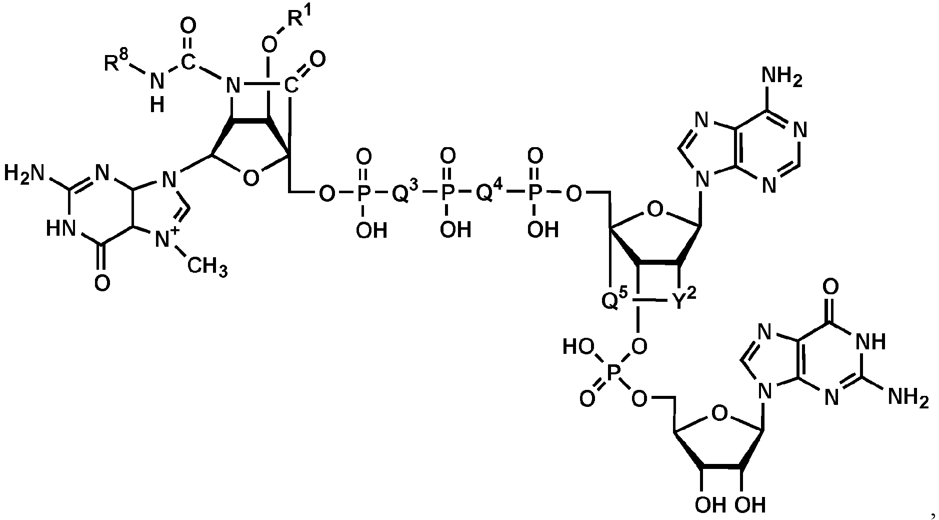 or 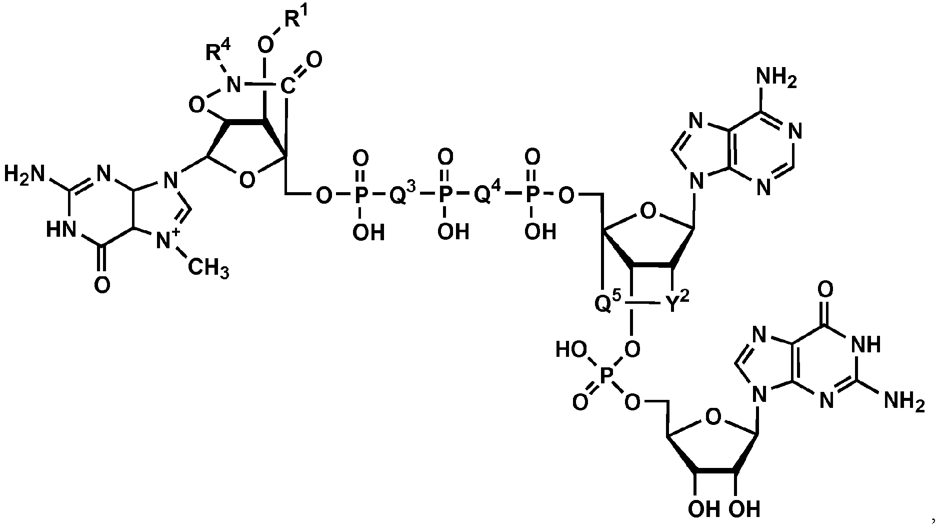 wherein R¹ is hydrogen, methyl or phenyl group; Q³ and Q⁴ are independently -O-, -CH₂- or -CCl₂-; Q⁵ is -CH₂- or 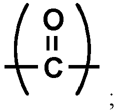 Y² is -O-, 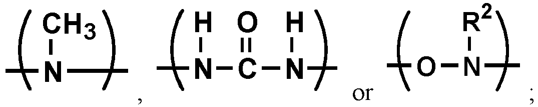 R⁴ is hydrogen or methyl; and R⁸ is C1-C6 alkyl group, C4-C8 cycloalkyl group, phenyl group, benzyl group or C3-C5 heterocyclic group.

7. The compound as claimed in Claim 1, wherein the compound is 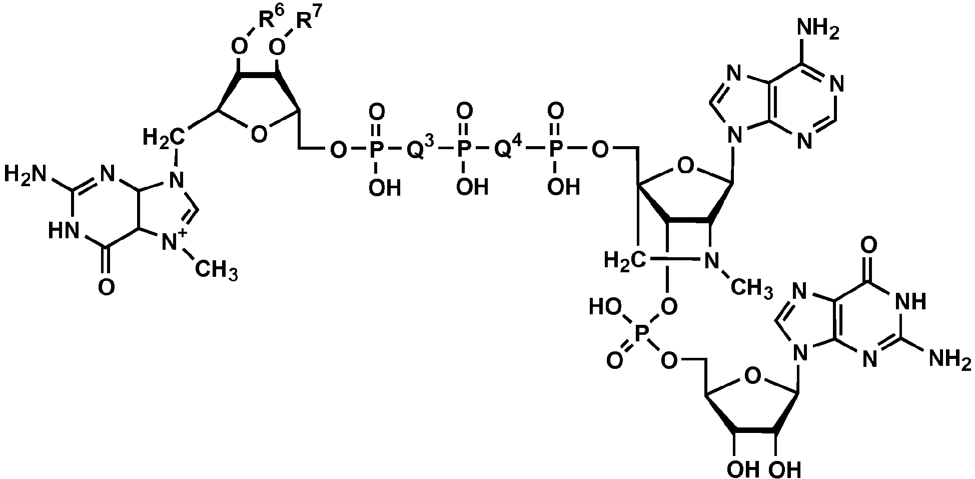 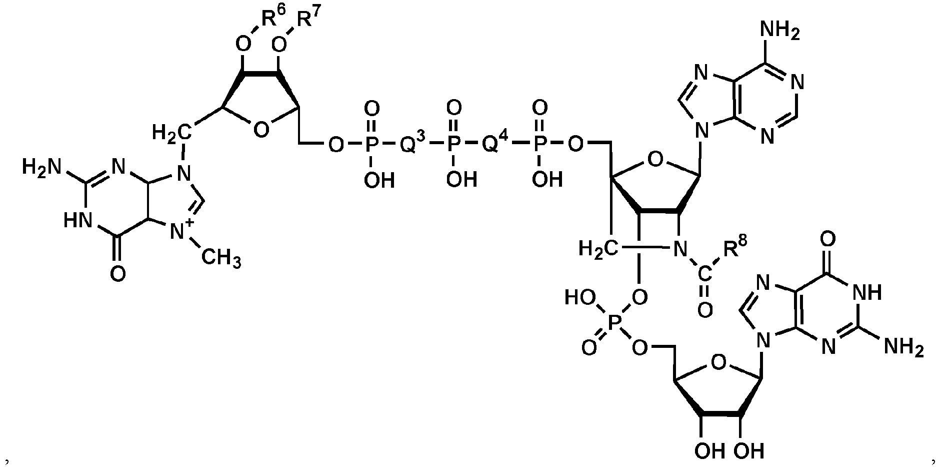 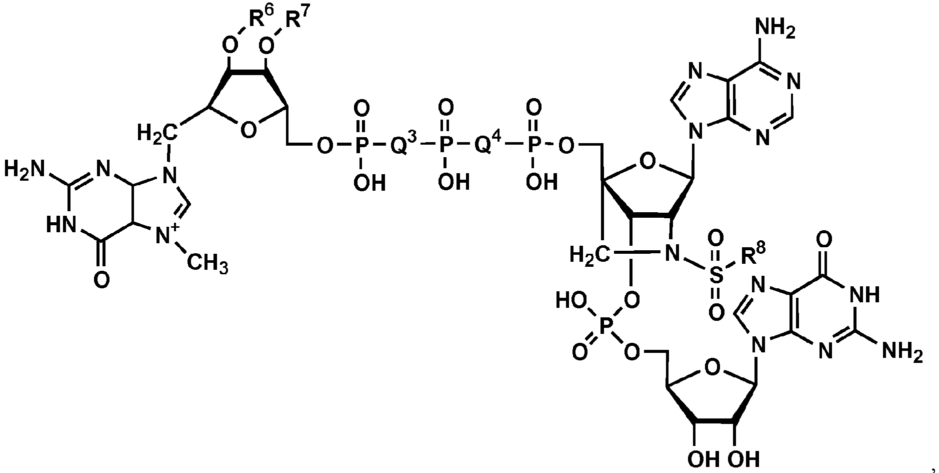 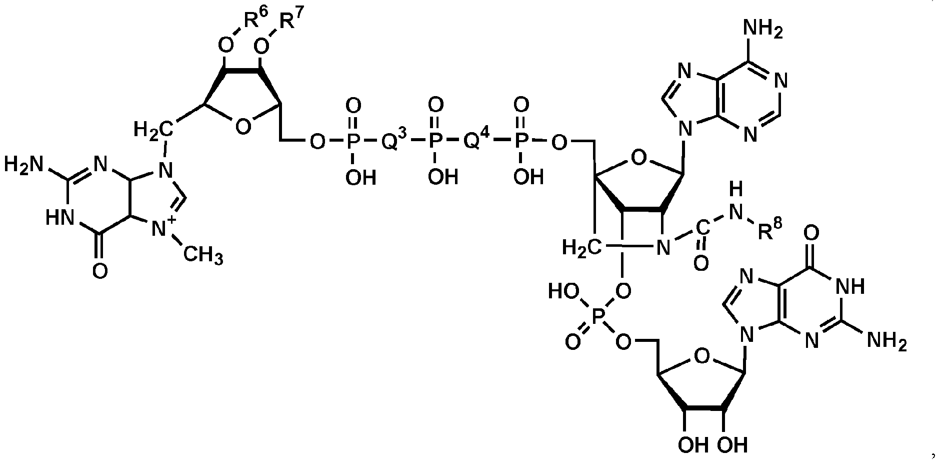 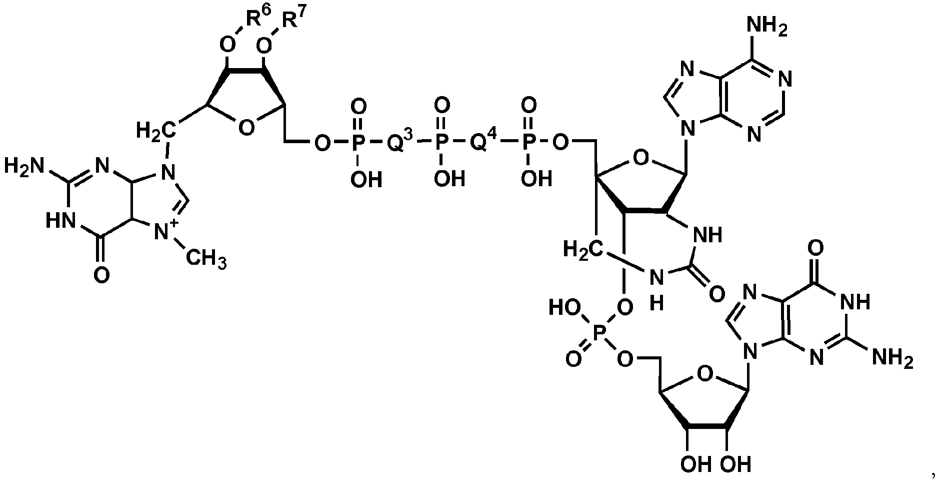 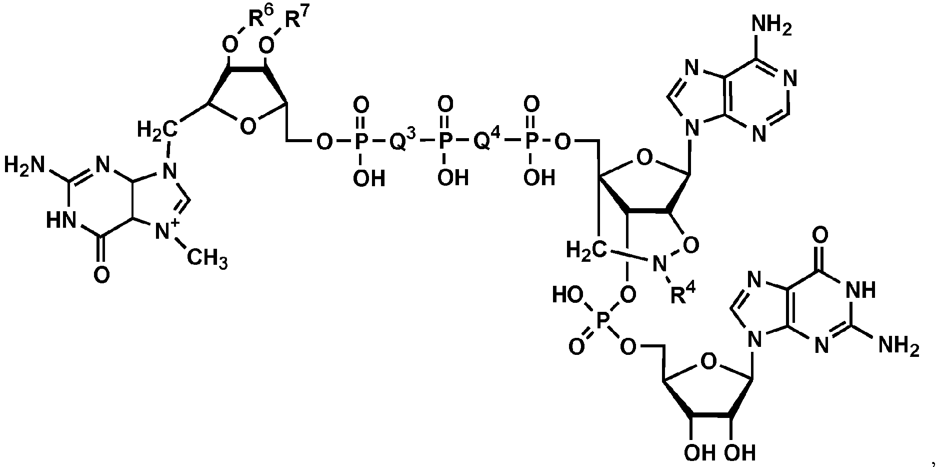 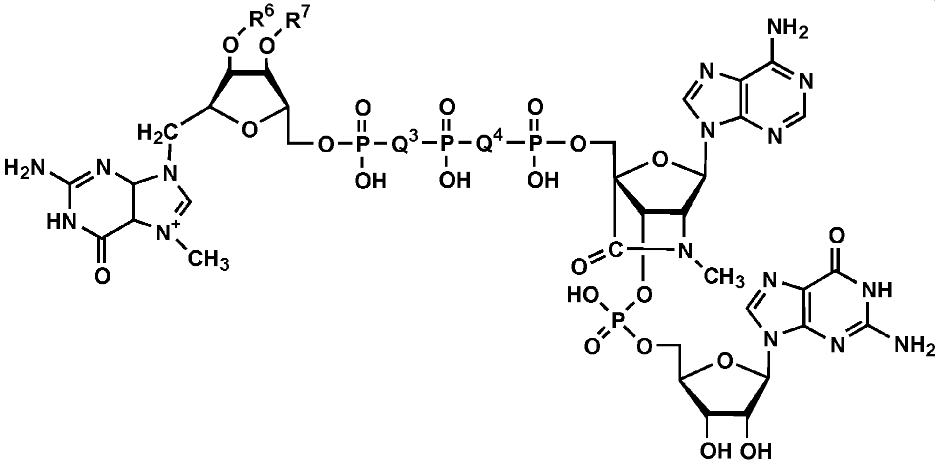 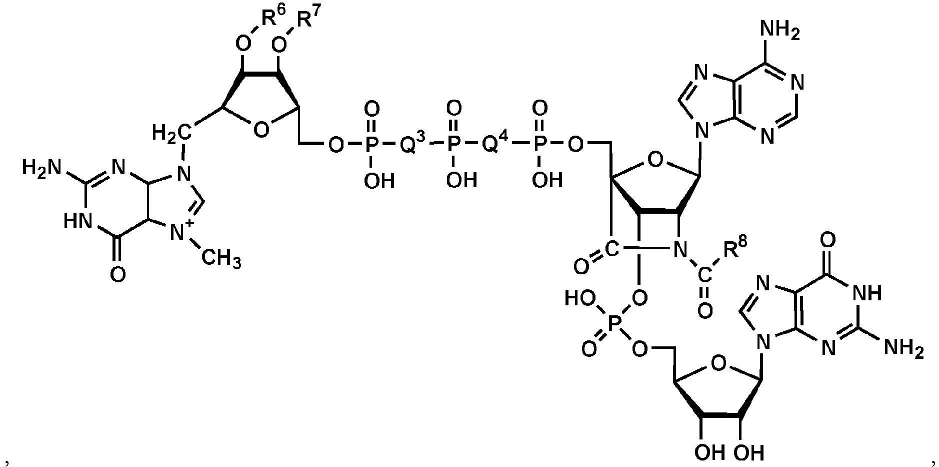 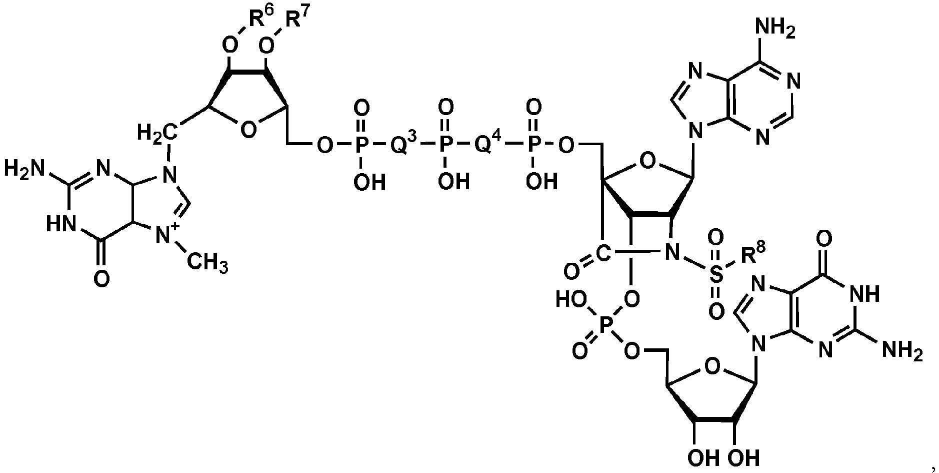 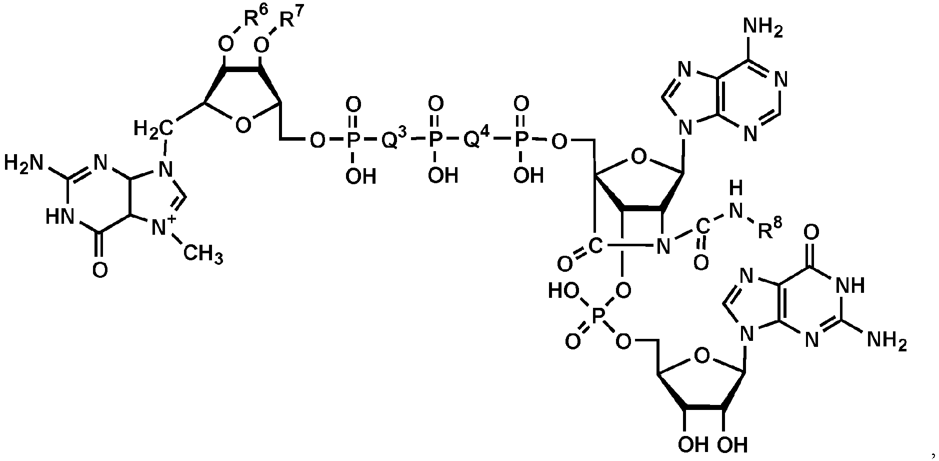 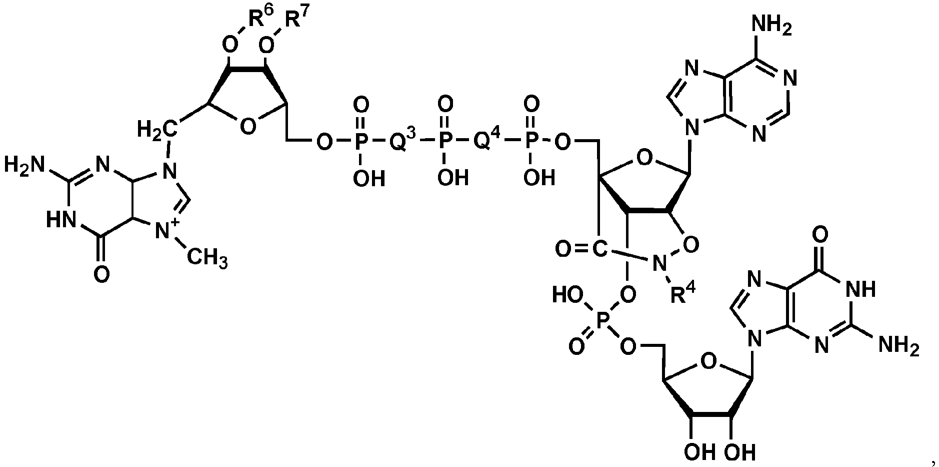 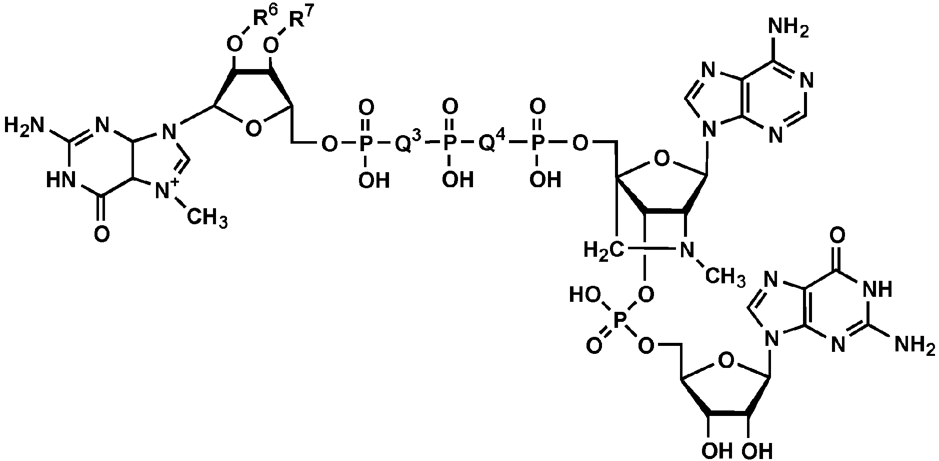 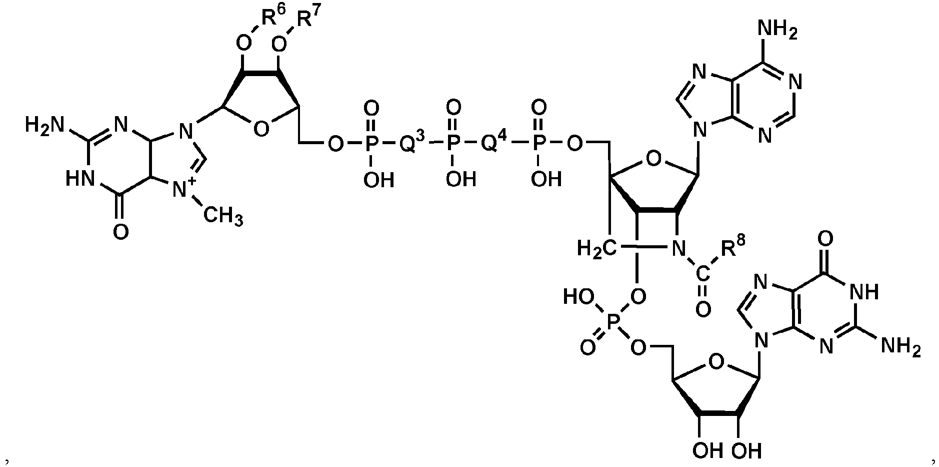 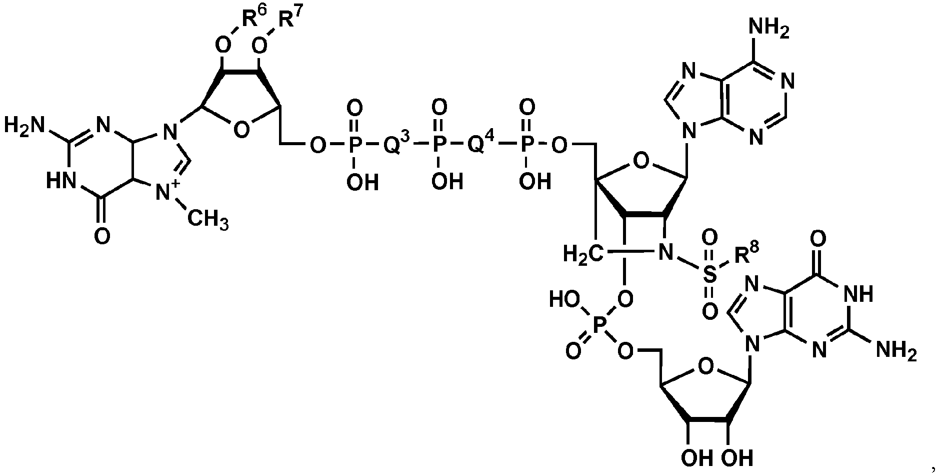 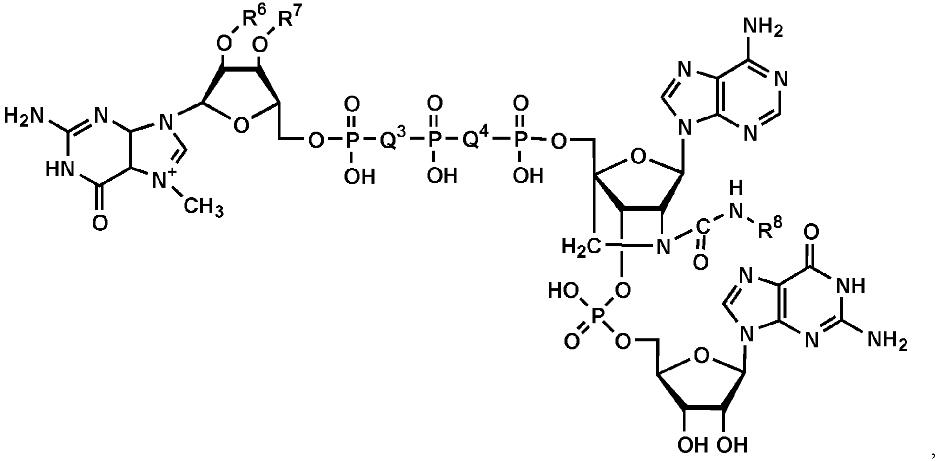 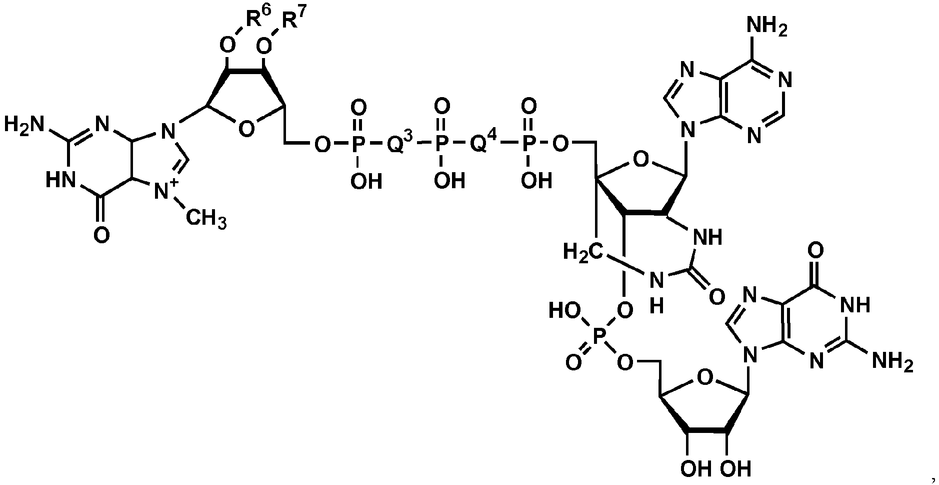 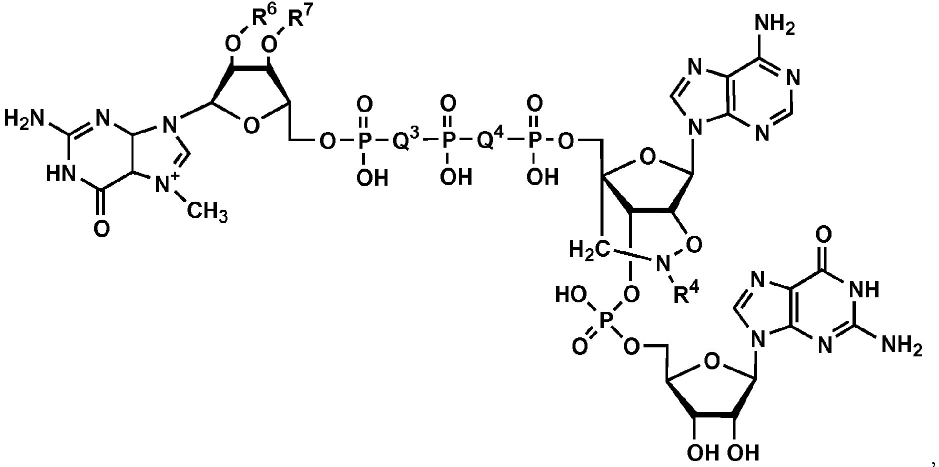 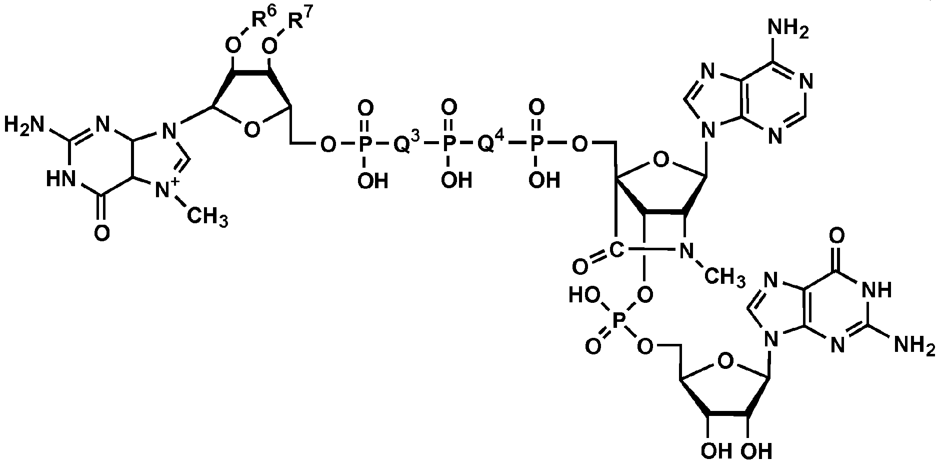 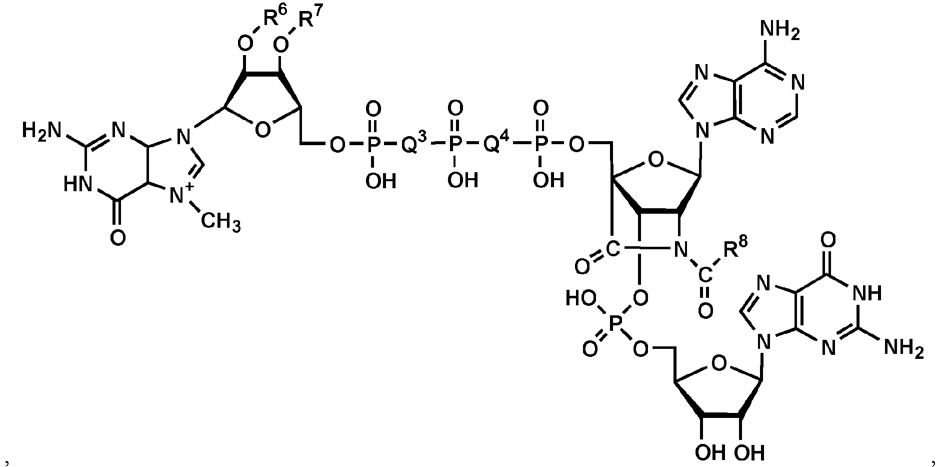 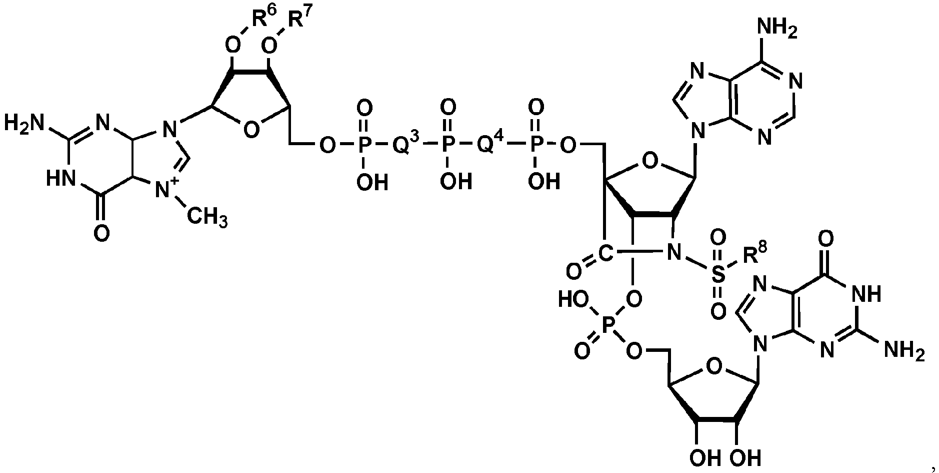 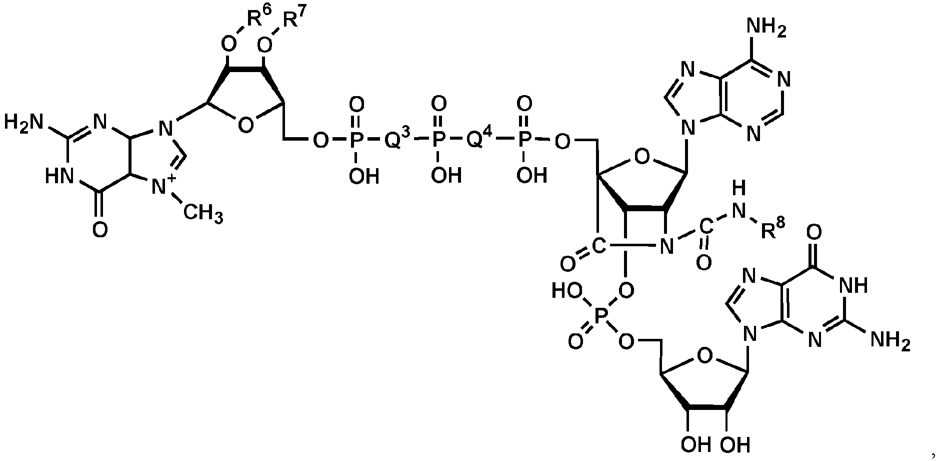 or 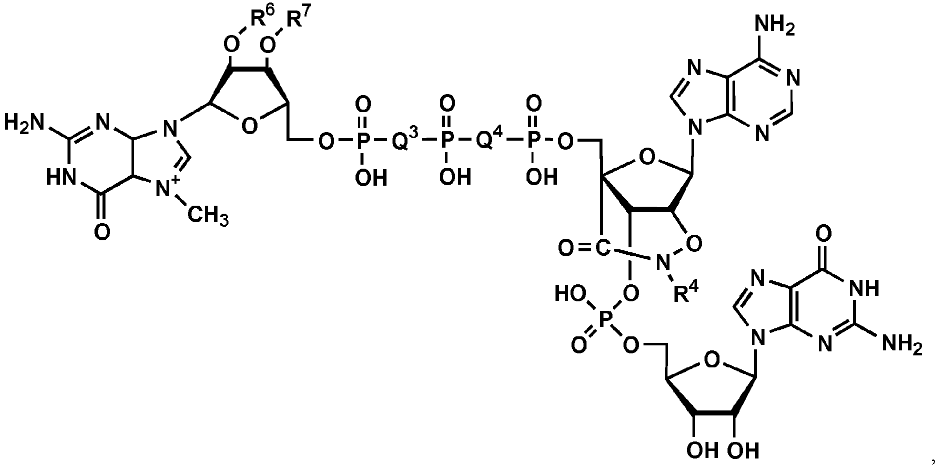 wherein Q³ and Q⁴ are independently -O-, -CH₂- or -CCl₂-; R⁴ is hydrogen or methyl; R⁶ and R⁷ are hydrogen, methyl or phenyl group; and R⁸ is C1-C6 alkyl group, C4-C8 cycloalkyl group, phenyl group, benzyl group or C3-C5 heterocyclic group.

8. The compound as claimed in Claim 1, wherein the compound is 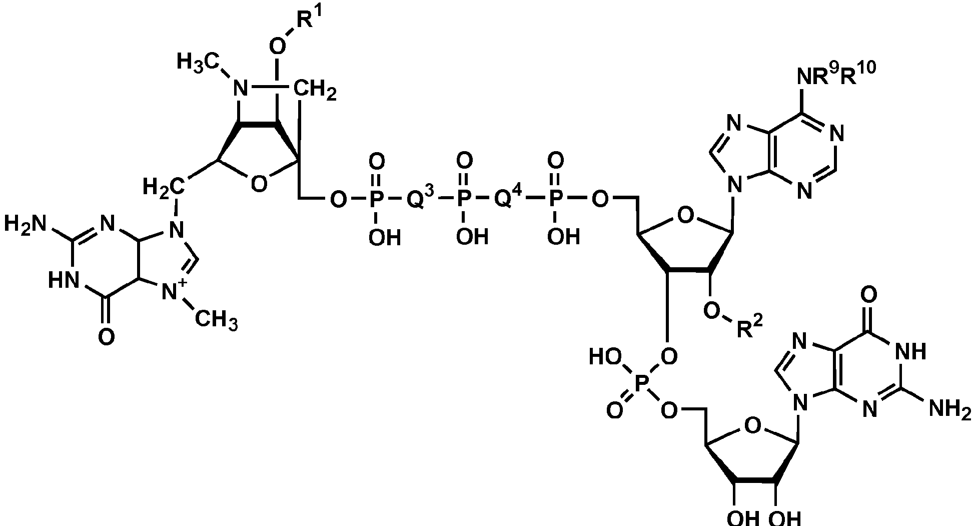 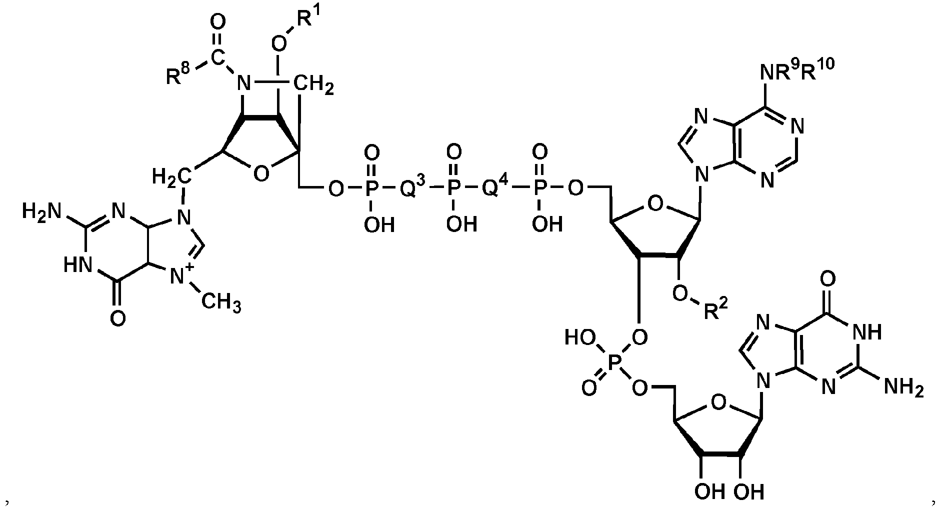 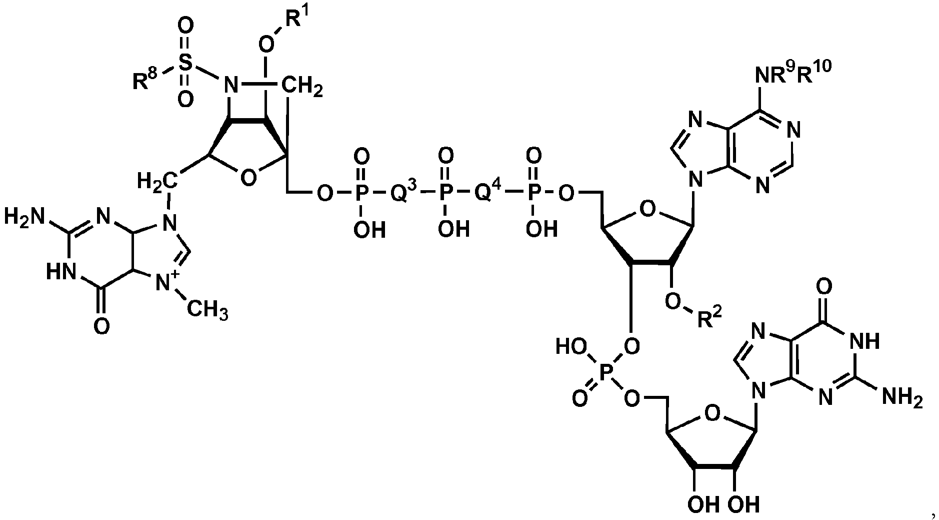 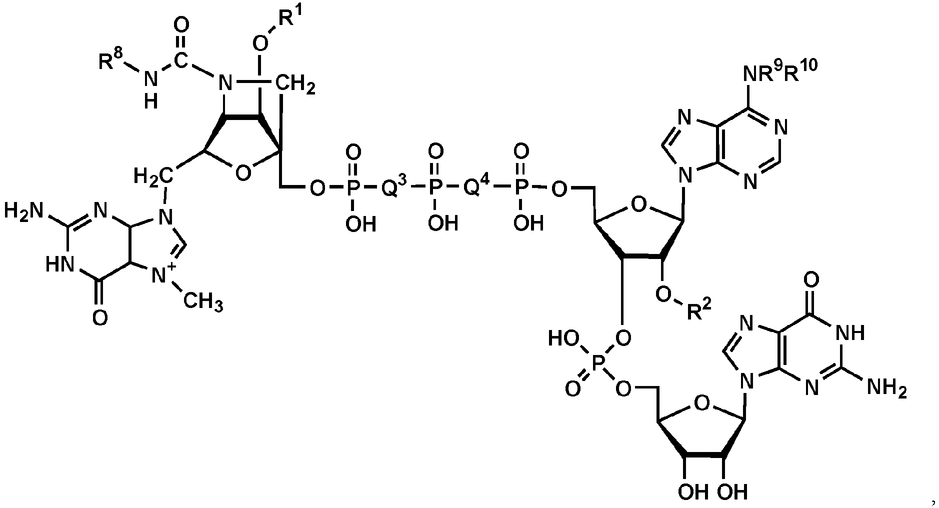 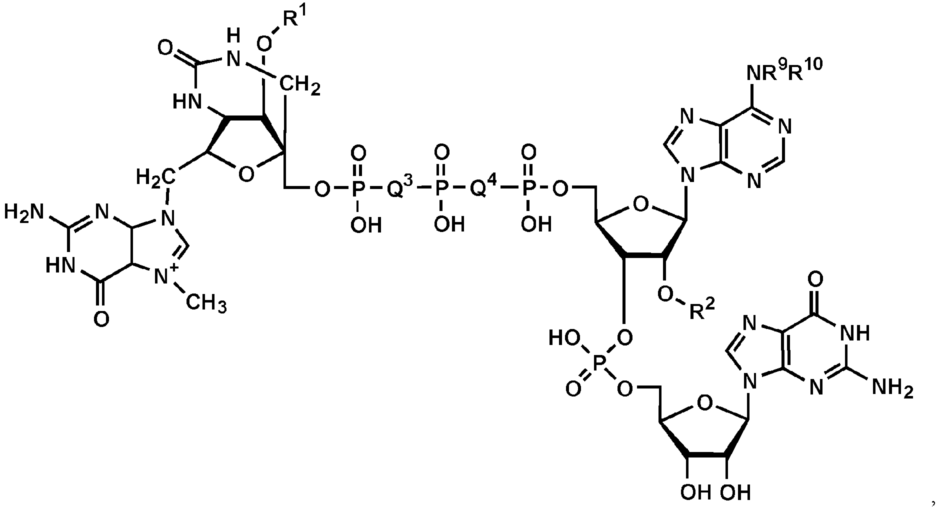 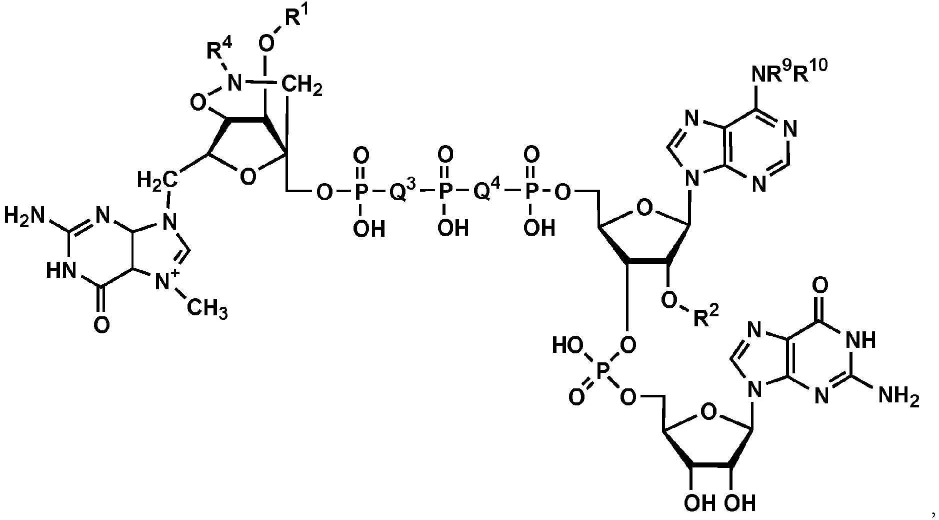 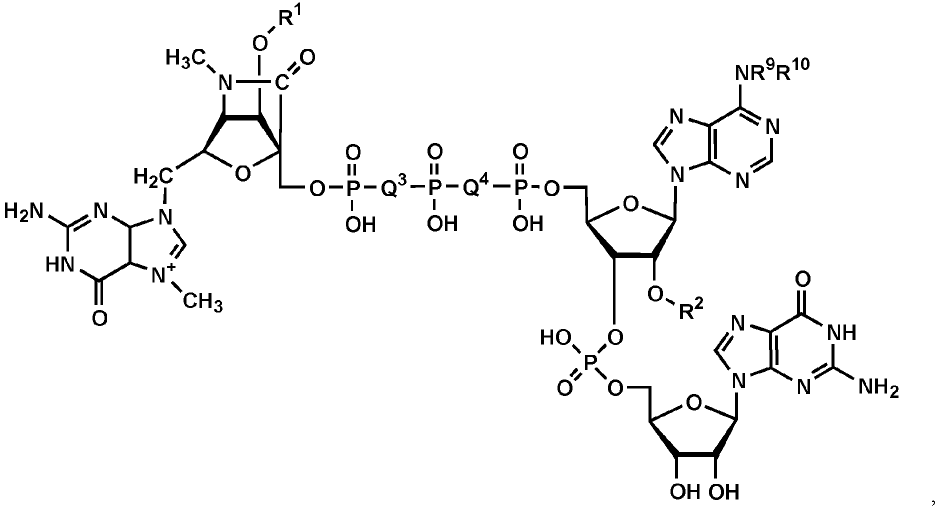 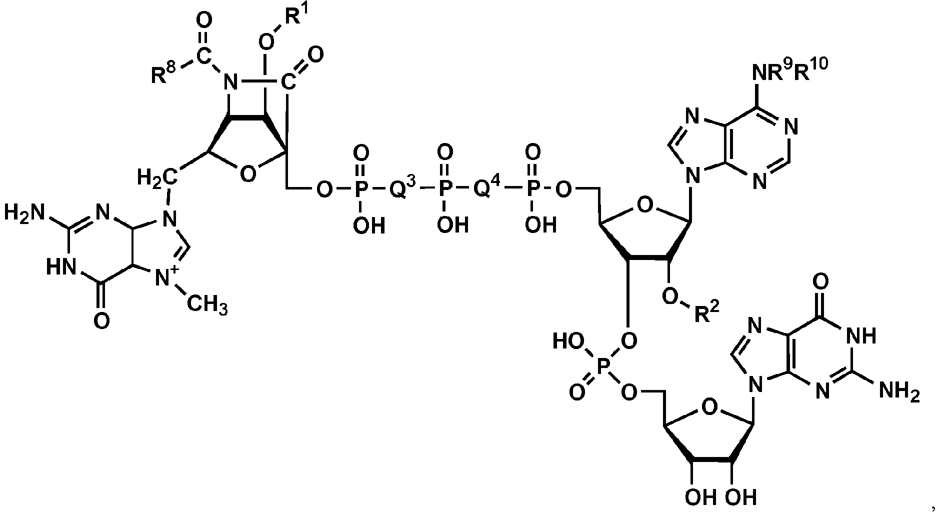 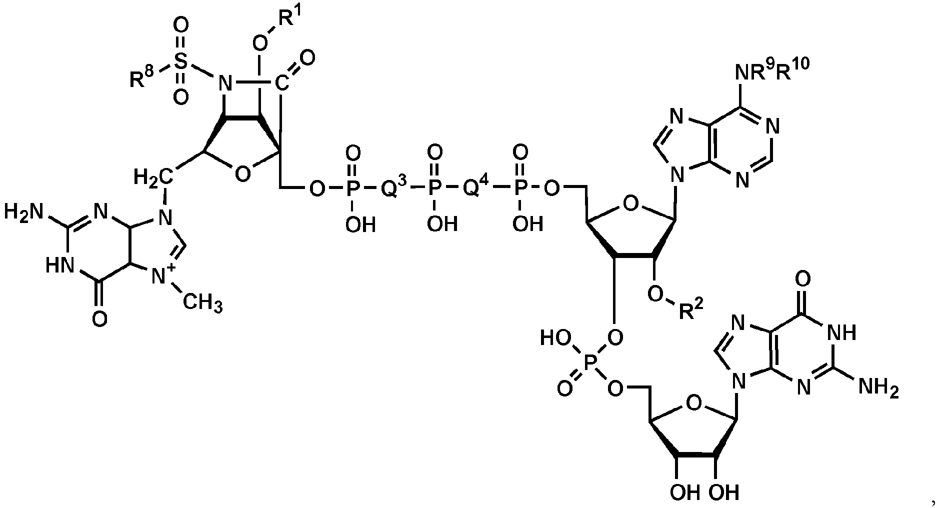 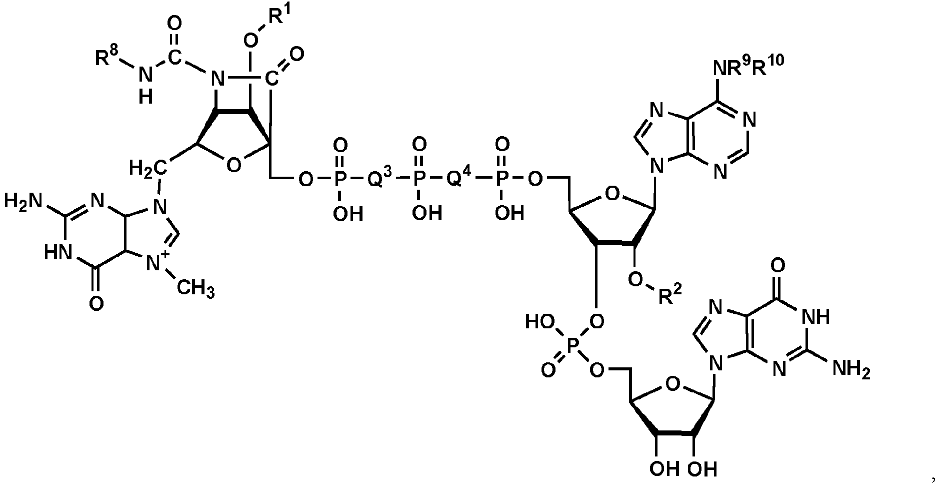 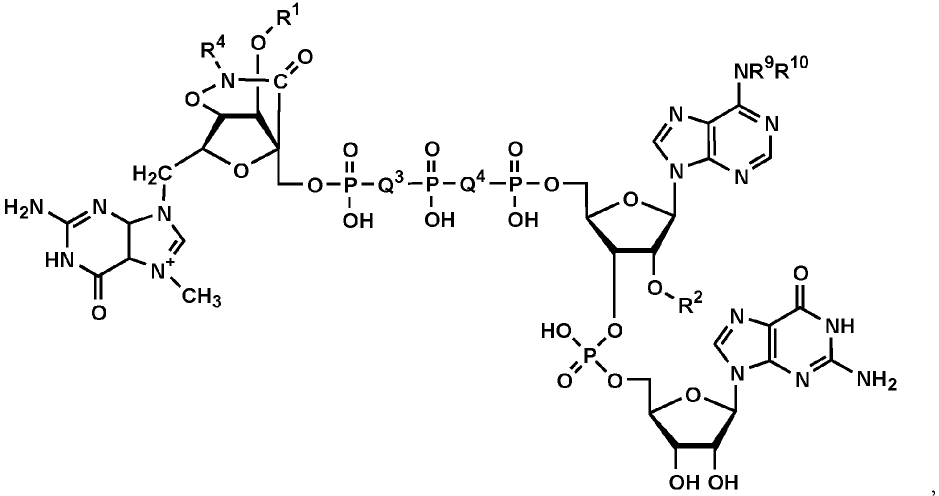 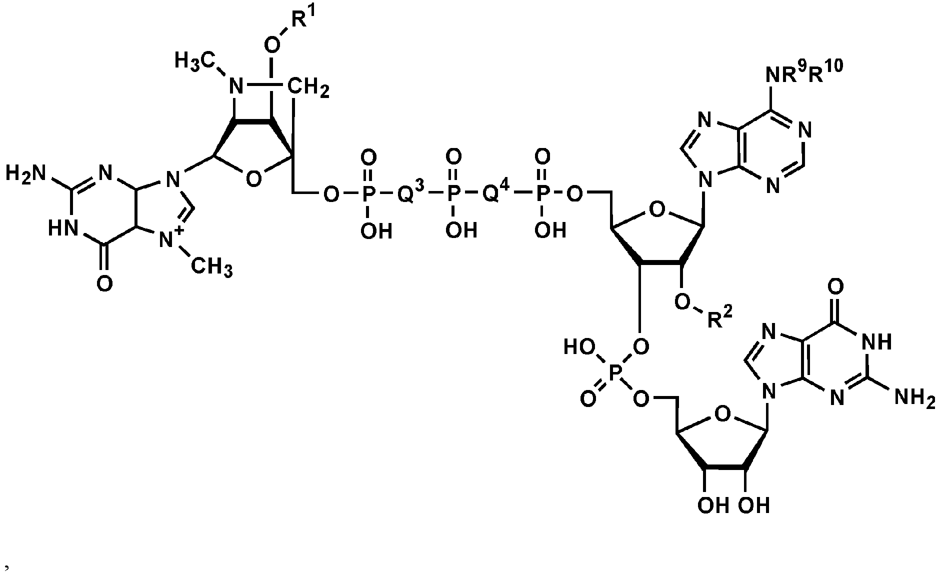 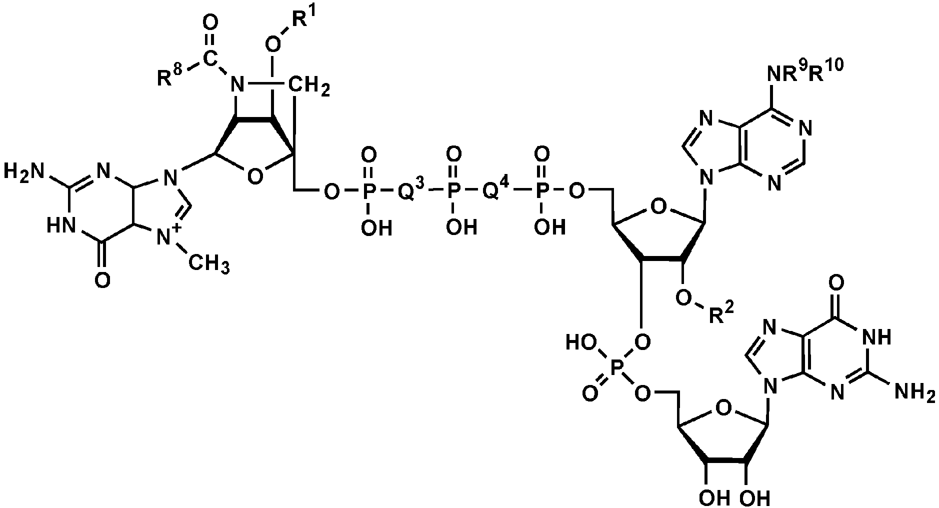 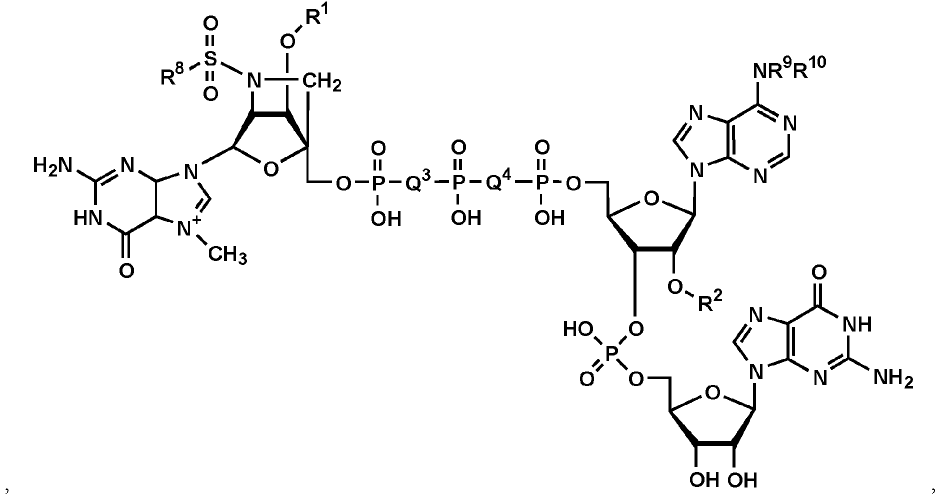 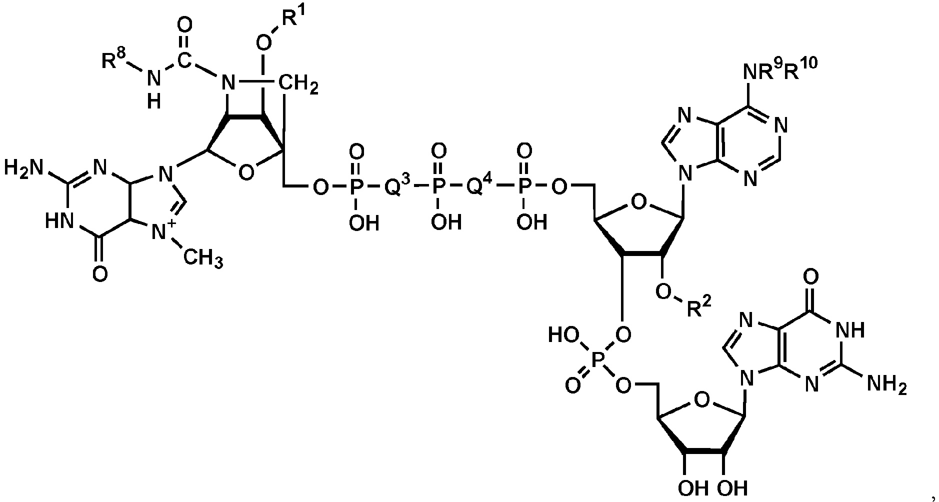 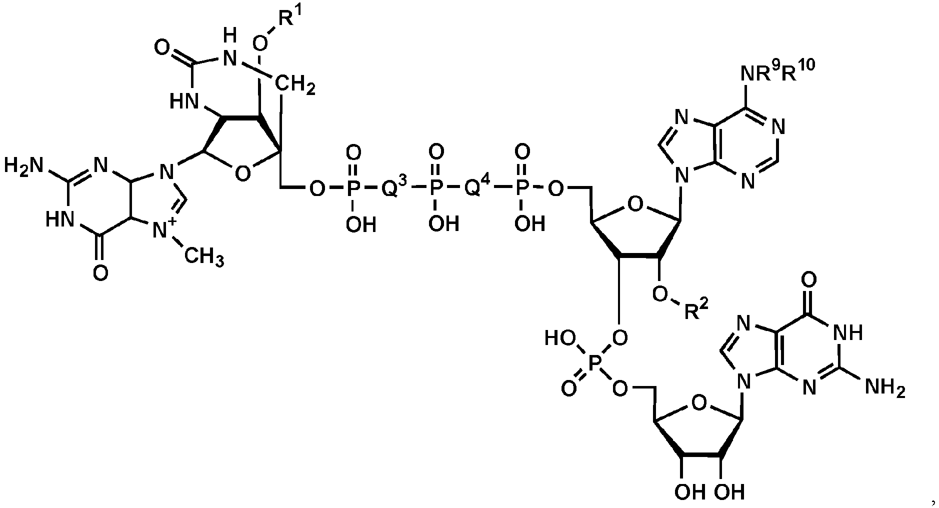 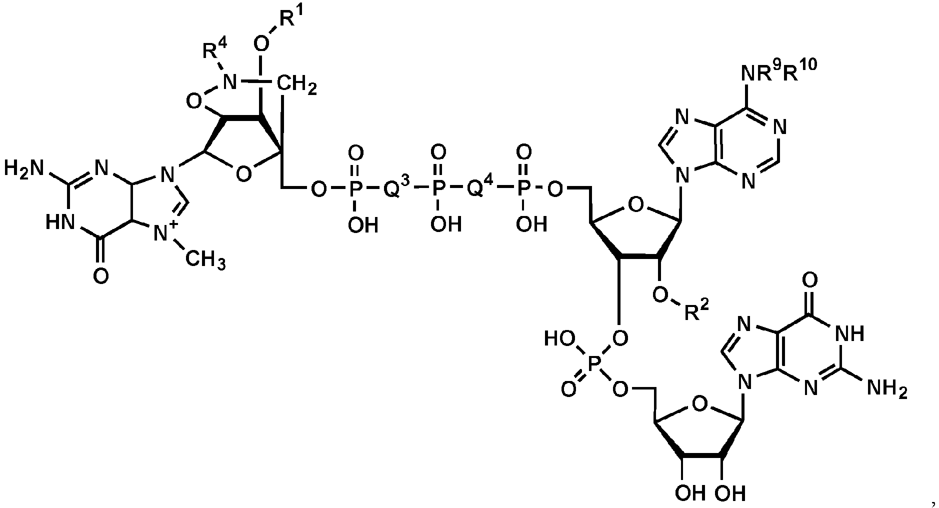 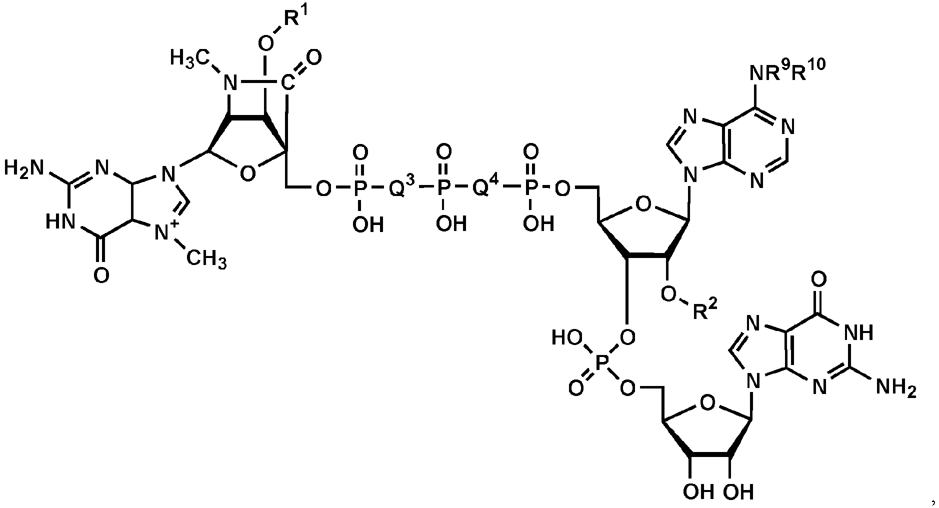 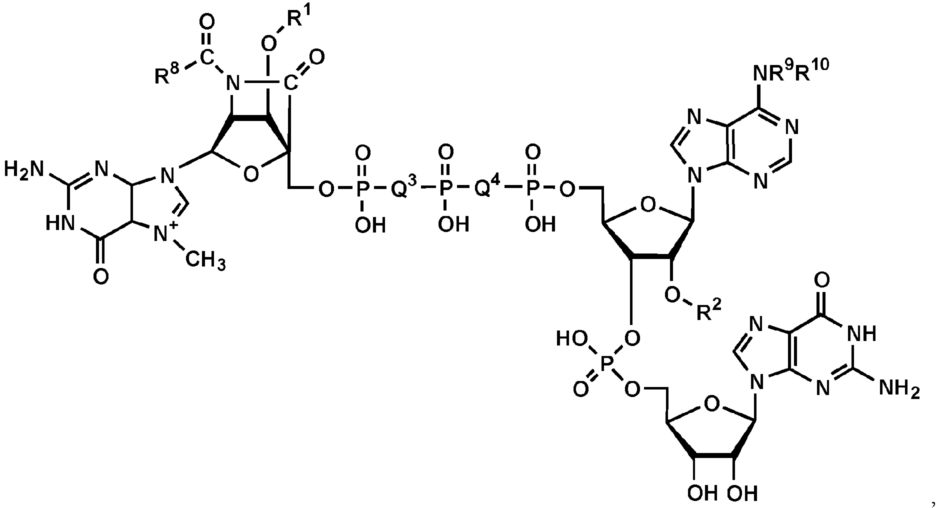 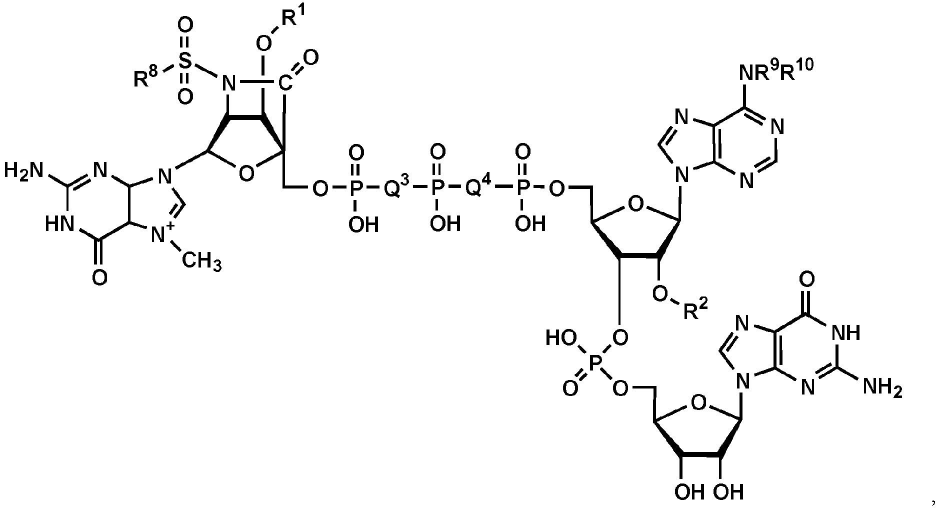 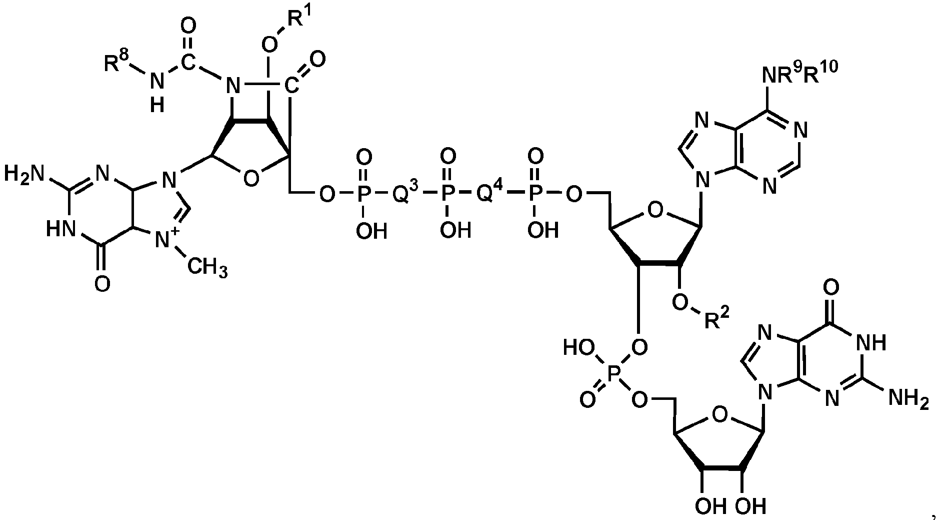 or 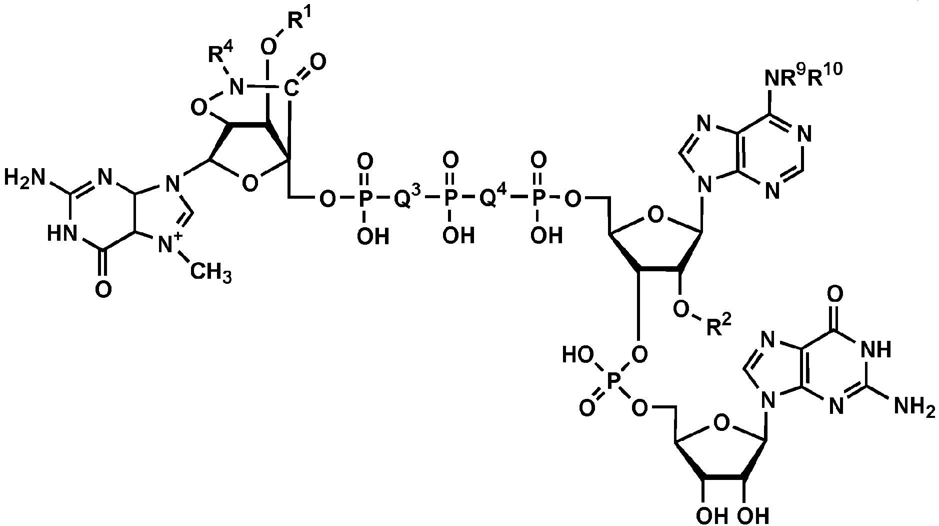 , wherein R¹ is hydrogen, methyl or phenyl group; R² is hydrogen or methyl; Q³ and Q⁴ are independently -O-, -CH₂- or -CCl₂-; Q⁵ is -CH₂- or 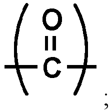 R⁴ is hydrogen or methyl; R⁸ is C1-C6 alkyl group, C4-C8 cycloalkyl group, phenyl group, benzyl group or C3-C5 heterocyclic group; and R⁹ and R¹⁰ are independently hydrogen, C1-C6 alkyl group, or benzyl group.

9. A pharmaceutical composition, comprising:
the compound as claimed in Claim 1; and
an RNA molecule, wherein the compound is enabled to covalently bond with the RNA
molecule.

10. A kit for capped RNA transcript, comprising:
the compound as claimed in Claim 1; and
an RNA polymerase.

11. The kit for capped RNA transcript as Claimed in Claim 10, further comprising:
an RNA molecule, wherein the RNA molecule is an mRNA molecule.

12. A method for in vitro transcription, comprising:
providing a composition, wherein the composition includes an RNA polymerase, a nucleoside triphosphate, and the compound as claimed in Claim 1; and
contacting a DNA template with the composition to transcribe the DNA template into RNA *in vitro.*
